# EUROPEAN PATENT SPECIFICATION

(11) **EP 1 981 342 B1**
(45) Date of publication and mention of the grant of the patent: **30.11.2016**
(21) Application number: 06774300.5
(22) Date of filing: 29.06.2006
(51) Int. Cl.: A01N 43/66, A01N 43/54, A01N 43/42, A01N 43/40, A61K 31/44, A61K 31/47, C07D 221/06, C07D 471/02, C07D 491/02, C07D 498/02, C07D 513/02, C07D 515/02, C07D 215/00, C07D 215/38, C07D 215/46, C07D 215/04, C07D 417/00, C07D 211/72, C07D 211/84, C07D 213/62, C07D 403/00

(54) **SELECTIVE BENZYLAMINE DERIVATIVES AND THEIR UTILITY AS CHOLESTEROL ESTER-TRANSFER PROTEIN INHIBITORS**
AUSGEWÄHLTE BENZYLAMINDERIVATE UND IHRE VERWENDUNG ALS CHOLESTEROLESTERTRANSFERPROTEINHEMMER
DERIVES DE BENZYLAMINE SELECTIFS ET LEUR UTILITE EN TANT QU'INHIBITEURS DE LA PROTEINE DE TRANSFERT DE L'ESTER DE CHOLESTEROL

(30) Priority: 28.12.2005 US 320120; 28.12.2005 WO PCT/US2005/047203
(43) Date of publication of application: 22.10.2008
(73) Proprietor: Dr. Reddy's Laboratories Ltd., Hyderabad 500 016 (IN)
(72) Inventor: BARUAH, Anima, Hyderabad, Andhra Pradesh 500049 (IN); DE, Dibyendu, Suwanee GA 30024 (US); KHANNA, Ish Kumar, Alpharetta GA 30022 (US); PILLARISETTI, Sivaram, Norcross GA 30092 (US); ALEXANDER, Christopher W., Atlanta GA 30345 (US); SREENU, Jennepali, Hyderabad, Andhra Pradesh 500016 (IN); MAITRA, Santanu, Hyderabad 500072, Andhra Pradesh (IN); ALIKINJU, Shanavas, Secunderabad 500 015 (IN)
(74) Representative: Bates, Philip Ian
(86) International application number: PCT/US2006/025427
(87) International publication number: WO 2007/075194

(56) References cited:
- EP-A2- 0 585 913
- WO-A1-2004/020393
- WO-A1-2005/100298
- WO-A1-2005/100298
- WO-A1-2006/056854
- WO-A2-98/04528
- WO-A2-2006/073973
- US-A1- 2003 114 454
- US-A1- 2005 059 810

## Description

### FIELD OF THE INVENTION

The present invention relates to selective benzylamine compounds, and compositions for use in treating or preventing conditions or diseases associated with lipoprotein metabolism.

### BACKGROUND OF THE INVENTION

Cholesteryl ester-transfer protein (CETP) is an important player in metabolism of lipoproteins such as, for example, a high density lipoprotein (HDL). CETP is a 70 kDa plasma glycoprotein that is physically associated with HDL particles. It facilitates the transport of cholesteryl ester from HDL to apolipoprotein B-containing lipoproteins. This transfer is accompanied by transfer of triglycerides in the opposite direction. Thus, a decrease in CETP activity can result in an increase in the level of HDL cholesterol and a decrease in the level of very low density lipoprotein (VLDL) and low density lipoprotein (LDL). CETP can therefore simultaneously affect the concentrations of pro-atherogenic (e.g., LDL) and anti-atherogenic (e.g., HDL) lipoproteins.

Human and clinical studies have shown that inhibitors of CETP can be effective in elevating HDL levels by 30-60%. And, epidemiological studies have shown that decreased high-density lipoprotein cholesterol (HDL-C) is a powerful risk factor for coronary artery disease (CAD). Gordon et al., Circulation, 79, pp. 8-15, 1989; Despres et al., Atherosclerosis 153: 263-272, 2000. Elevating HDL-C has been shown to decrease this risk and it is estimated that each 1 mg/dl (0.02 mmol/l) elevation of HDL-C is associated with a 2-3% reduction in coronary heart disease (CHD) risk, a magnitude comparable to that for low density lipoprotein (LDL) lowering. It has been recommended that serum HDL-C levels of >40 mg/dl be considered as a therapeutic target in primary and secondary prevention. This goal appears to be particularly important in patients with low serum HDL-C levels and ischemic heart disease (IHD) or its equivalents, even if the therapeutic target for serum low-density lipoprotein cholesterol (LDL-C) levels (<100 mg/dl) has been achieved.

It is believed that the anti-atherogenic role of HDL is in part due its ability to promote the efflux of free cholesterol from cells and to transport it to the liver, a process termed reverse cholesterol transport. HDL could protect against atherosclerosis by several other mechanisms. For example, several studies showed HDL to have antioxidant and anti-inflammatory effects. Oxidative products of lipid metabolism induce inflammatory cell recruitment in vascular cells. HDL particles carry enzymes that retard LDL oxidation, including paraoxonase, platelet-activating factor acetylhydrolase, and lecithin-cholesterol acyltransferase. These enzymes degrade pro-inflammatory, oxidized phospholipids, limiting their accumulation in LDL. In addition, apoA-I can bind oxidized lipids and remove them from LDL. Further, HDL also can act as a carrier vehicle for small molecules, including bacterial lipopolysaccharide (LPS) thus regulating the inflammatory effects of LPS. In animal models of endotoxic shock, HDL attenuates organ injury and adhesion molecule expression. Thus elevating HDL is not only anti-atherogenic but it could also potentially be anti-inflammatory.

Existing therapies such as, for example, HDL-elevating therapies and anti-atherosclerosis therapies have limitations including serious toleration issues. There is a present need to find alternative therapies including methods of preventing or treating conditions or diseases associated with lipoprotein metabolism such as, for example, atherosclerosis.

WO 2006/05685 os directed to dibenzyl amine compounds and derivatives.

US2003/114454 relates to substituted polycyclic aryl and heteroaryl tertiary heteroalkylamine compounds that inhibit cholesteryl ester transfer protein (CETP).

EP0585913 is directed to condensed heterocyclic compounds which inhibits the enzyme acyl-CoA: cholesterol acyl transferase (ACAT) and has a high tachykinin receptor antagonising activity.

WO98/04528 is directed to substituted biaryl compounds which inhibit cholesterol ester transfer proteins (CETPs), stimulate reverse cholesterol transport and inhibit the action of glucagon.

US2005/059810 relates to a novel CETP activity inhibitor, particularly a therapeutic agent or a prophylactic agent of arteriosclerosis or hyperlipidemia.

WO2005/100298 is directed to a class of chemical compounds that inhibit cholesterol ester transfer protein (CETP) and may have utility in the treatment of and prevention of atherosclerosis.

WO2006/073973 relates to benzylamine compounds, methods and compositions for making and using the benzylamine compounds and compositions and methods for treating or preventing conditions or diseases associated with lipoprotein metabolism.

### SUMMARY OF THE INVENTION

The present invention relates to a compound selected from 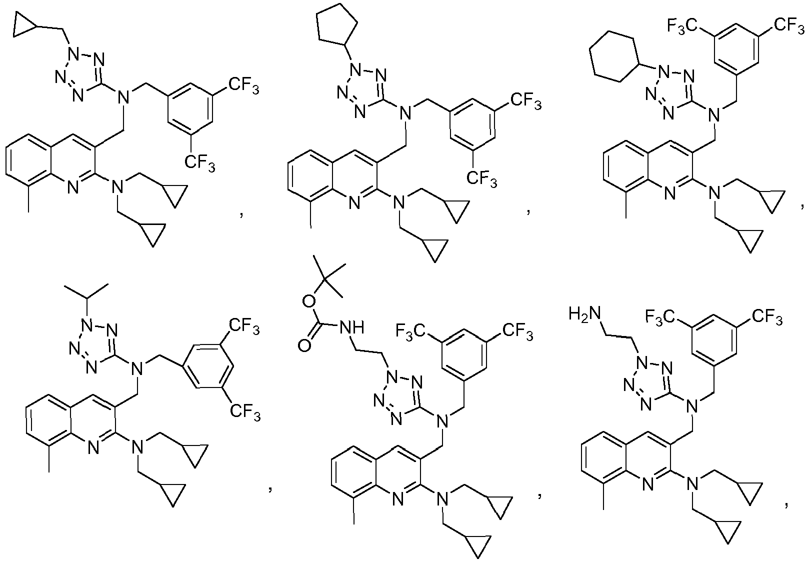 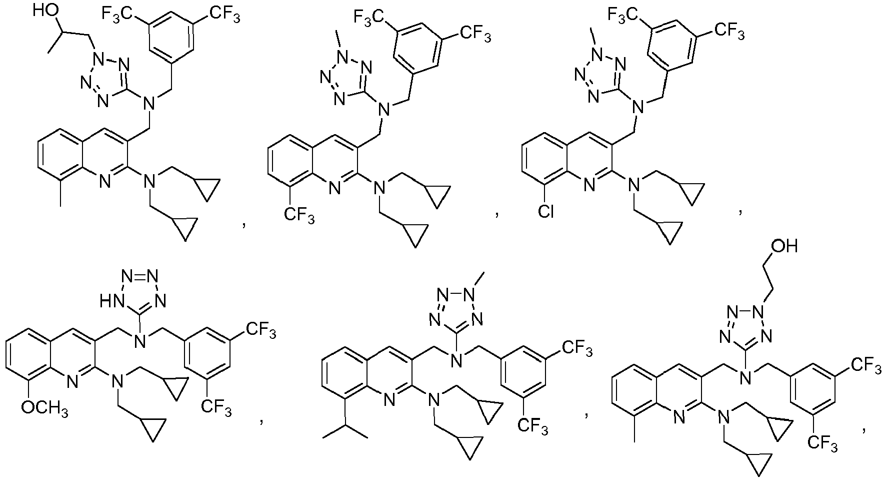 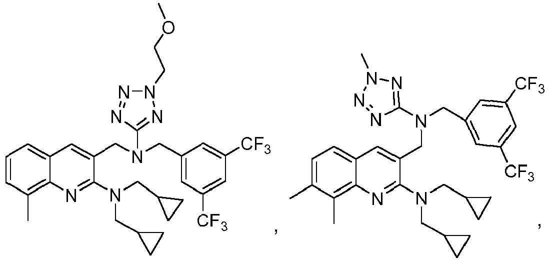 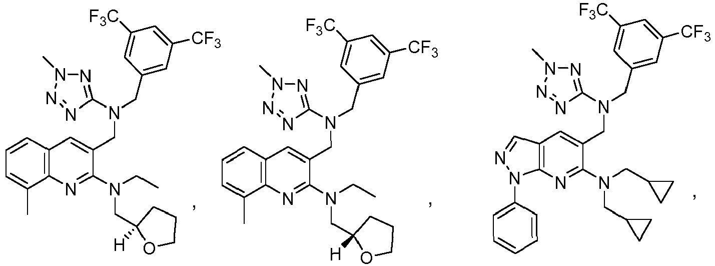 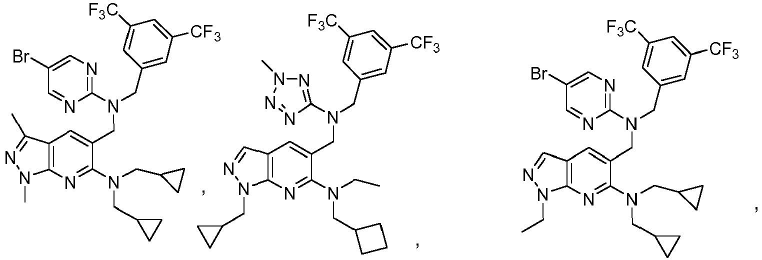 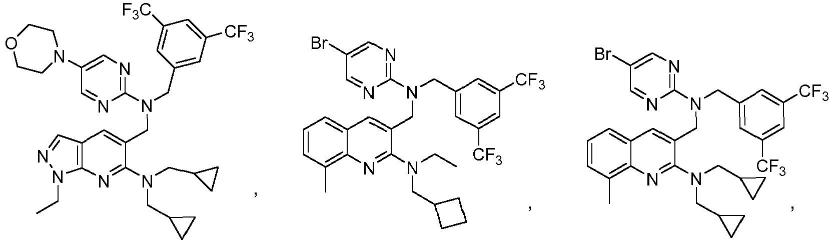 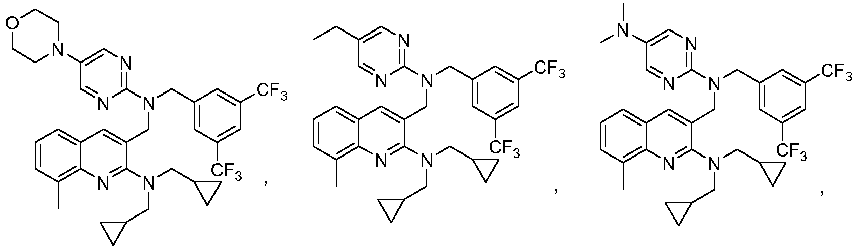 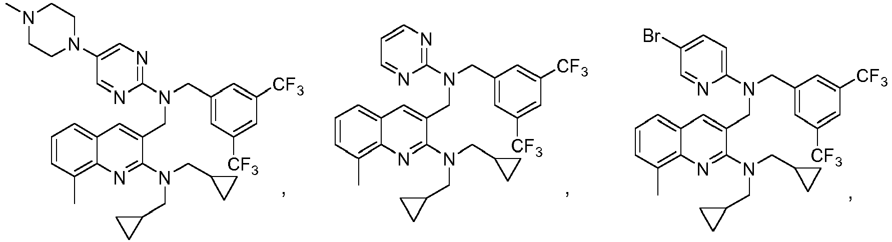 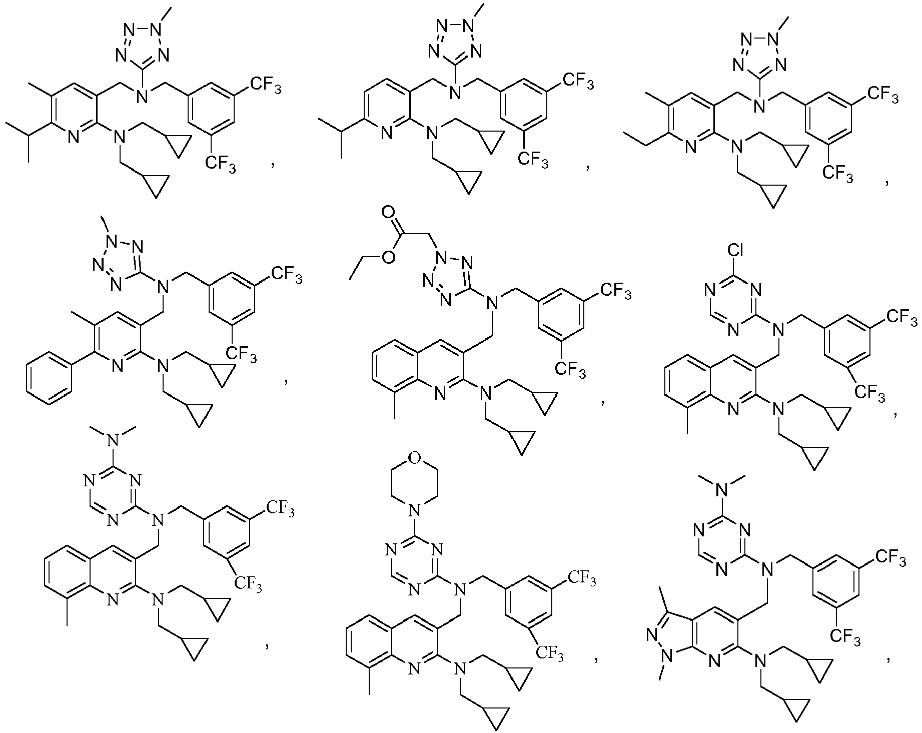 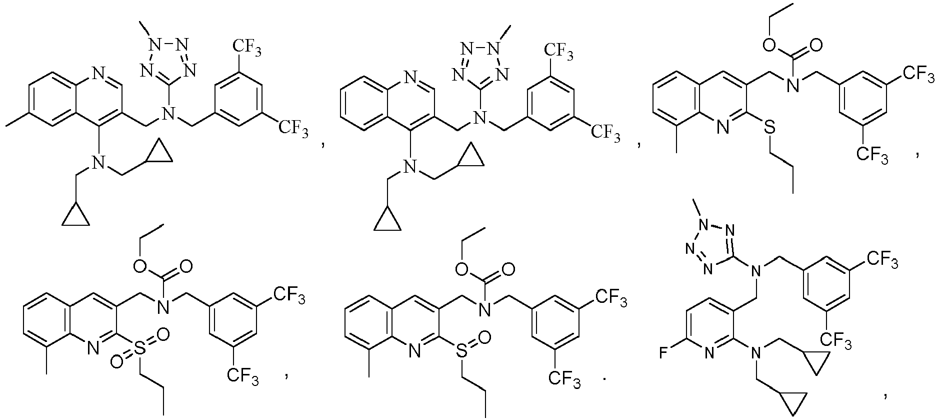 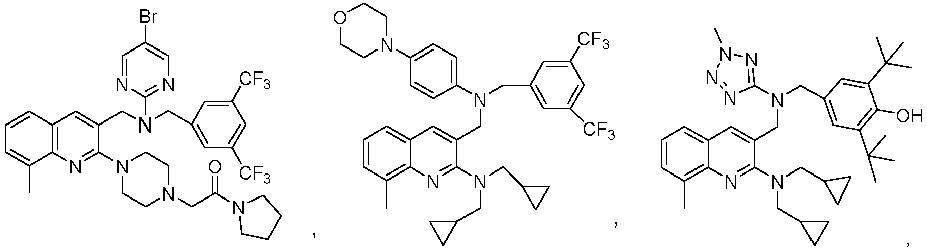 or a salt, a prodrug, a diastereomeric mixture, an enantiomer, a tautomer, or a racemic mixture thereof.

The present invention is also directed to a pharmaceutical composition comprising a pharmaceutically acceptable carrier and at least one of the above compounds.

The pharmacetutical composition may further comprise at least one of the following:
a pharmaceutically acceptable auxiliary;
a pharmaceutically acceptable preservative;
a pharmaceutically acceptable excipient;
a pharmaceutically acceptable diluent;
a pharmaceutically acceptable solvate; or
any combination thereof.

The pharmaceutical composition may be in the form of a tablet, a capsule, a cachet, a powder, a granule, a solution, a suspension, an emulsion, a bolus, a lozenge, a suppository, a pessary, a tampon, a cream, a gel, a paste, a foam, a spray, an aerosol, a microcapsule, a liposome, a transdermal patch, a pastille, a paste, or a mouthwash.

The present invention is also direceted to a compound set out above which is a cholesteryl ester transfer protein (CETP) inhibitor.

The present disclosure is directed to novel benzylamine compounds, novel compositions comprising these benzylamine compounds, and novel methods employing such benzylamine compounds and their compositions. Disclosed herein are methods for making benzylamine compounds compounds, compositions comprising these benzylamines, and methods and compositions for using these benzylamines. The benzylamine compounds and compositions comprising these compounds have utility in treatment of a variety of diseases. Certain aspects of benzylamine compounds have been disclosed in PCT Publication WO 2004/020393, and in U.S. Patent Numbers 6,710,089 and 6,723,753.

In one aspect, the present disclosure provides for compounds and compositions comprising these compounds, in which the compounds have the following formula: 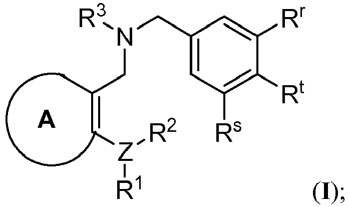 or a salt, including a pharmaceutically acceptable or a non-pharmaceutically acceptable salt, a prodrug, a diastereomeric mixture, an enantiomer, a tautomer, a racemic mixture, or any combination thereof, wherein:
A is selected from 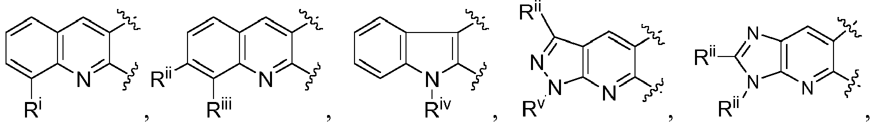 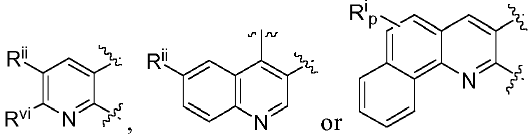 Wherein
   R¹, in each occurrence, is selected independently from hydrogen, halogen, alkyl, haloalkyl, alkoxy;
   Rⁱⁱ, in each occurrence, is selected independently from hydrogen or alkyl;
   Rⁱⁱⁱ, in each occurrence, independently represents halogen, alkyl, haloalkyl or alkoxy;
   R^{iv}, in each occurrence, is selected independently from hydrogen, alkyl, or - COR⁴ or CO₂R⁵;
   R^{v}, in each occurrence, is selected independently from alkyl, aryl, or -(CH₂)ₘcycloalkyl;
   R^{vi}, in each occurrence, is selected independently from halogen, alkyl or aryl;
   R⁴ represents cycloalkyl;
   R⁵ represents alkyl;
   m represents 0-2, inclusive;
   p is an integer from 0 to 3, inclusive;
   R^{r} and R^{s} independently represents -C(CH₃)₃ or -CF₃;
   R^{t} represents hydrogen or hydroxyl;
   R³, in each occurrence, is selected independently from
      (i) a cyclic group selected from 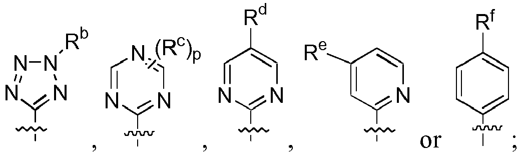 or
      (ii) -CO₂Et, when ZR¹ moiety represents S, SO, or SO₂;
   R^{b}, in each occurrence, is selected independently from (i) methyl, isopropyl,-(CH₂)₂OH, -(CH₂)₂OCH₃, -(CH₂)₂NHCO₂CH(CH₃)₃, -(CH₂)₂NH₂, -CH₂CH(CH₃)OH, -CH₂CO₂Et; (ii) 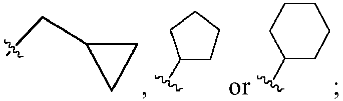
   R^{c} and R^{f}, in each occurrence, is selected independently from (i) hydrogen, halogen, alkyl, heterocyclyl comprising at least one heteroatom selected independently from >O, >N, or >S, , any of which having up to 6 carbon atoms; or (ii) NR^{y}R^{Z}, wherein R^{y} and R^{z} independently represents hydrogen or alkyl group;
   R^{d}, in each occurrence, is selected independently from hydrogen, halogen, (C₁-C₃)alkyl, -N(CH₃)₂, 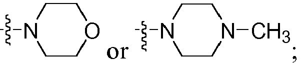
   R^{e} independently represents halogen;
   Z represents N; or the ZR¹ moiety represents S, SO or SO₂; or
   the ZR¹R² moiety independently represents
      (i) -C(O)Z¹R^{g} wherein R^{g} is hydrogen, cycloalkyl, aryl or (cycloalkyl)alkyl, Z¹ represents O or NR^{h}, wherein R^{h} is hydrogen, alkyl, cycloalkyl, or (cycloalkyl)alkyl; or
      (ii) 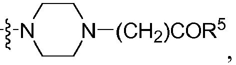 R⁵ represents heterocyclyl comprises comprising at least one heteroatom selected independently from >O, >N, or >S;
   R¹ and R², in each occurrence, is selected independently from ethyl,

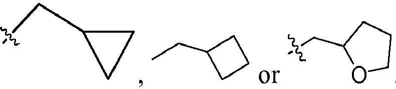

In another aspect, the present disclosure provides for compounds and compositions, in which the compounds have the following formula: 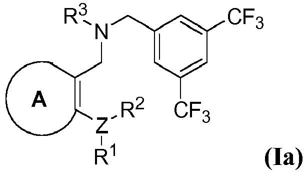 or a salt, a prodrug, a diastereomeric mixture, an enantiomer, a tautomer, a racemic mixture, or any combination thereof, wherein all symbols are as defined above. Various aspects of the invention are provided.

In yet another aspect, the present disclosure is also directed to methods or processes for the preparation of the benzylamine compounds disclosed herein, including compounds of the general formula (I). In another aspect, this disclosure is also directed to compositions comprising the benzylamine compounds disclosed herein, including compounds of the general formula (I).
The pharmaceutical composition of the present invention, comprises a pharmaceutically acceptable carrier and at least one compound according to this invention, and further comprises: optionally, a pharmaceutically acceptable auxiliary; optionally, a pharmaceutically acceptable preservative; optionally, a pharmaceutically acceptable excipient; optionally, a pharmaceutically acceptable diluent; and optionally, a pharmaceutically acceptable solvate.

The present invention also is directed to a composition for use in a method for treating a condition or disease in a mammalian subject, including a human. In some aspects, the method comprises administering to the subject a composition comprising a therapeutically-effective amount of at least one compound of the invention, or their pharmaceutically-acceptable salts thereof. Besides being useful for treating a human subject, the compositions of the present invention are useful for treating a variety of mammals such as, for example, companion animals such as cats or dogs, primates, ruminant animals, and rodents.

The present invention also is directed to a composition for use in a method for treating or preventing a condition or disease in a human or an animal subject, the method comprising administering to the subject a composition comprising a prophylactically- or therapeutically-effective amount of at least one compound of the invention, or their pharmaceutically-acceptable salts thereof. In some aspects, for example, this invention provides compositions for use in the treatment and/or prevention of conditions or disease states in a human or animal, such as dyslipidemia, atherosclerosis, peripheral vascular disease, hypertryglyceridemia, hypercholesterolemia, hyperbetalipoproteinemia, hypoalphalipoprotenemia, cardiovascular disorders such as angina, ischemia, stroke, myocardial infarction (MI), reperfusion injury, restenosis and hypertension, and diabetic vascular diseases such as diabetic retinopathy, and endotoxemia, comprising administering a therapeutically-effective amount of at least one compound of the invention.

### DETAILED DESCRIPTION OF THE INVENTION

In accordance with the present invention, novel benzylamine compounds and novel compositions comprising these benzylamine compounds are described. In one aspect of the disclosure, compounds in accordance with the present invention can comprise benzylamine compounds having the following formula: 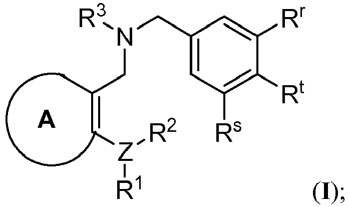 or a salt, a prodrug, a diastereomeric mixture, an enantiomer, a tautomer, a racemic mixture, or any combination thereof, wherein:
A is selected from 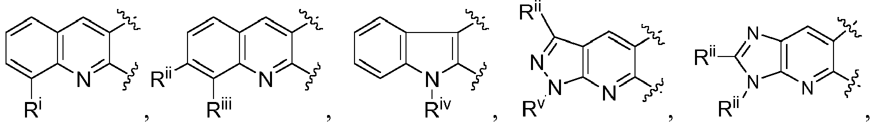 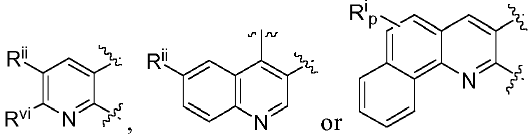 Wherein
   R¹, in each occurrence, is selected independently from hydrogen, halogen, alkyl, haloalkyl, alkoxy;
   Rⁱⁱ, in each occurrence, is selected independently from hydrogen or alkyl;
   Rⁱⁱⁱ, in each occurrence, independently represents halogen, alkyl, haloalkyl or alkoxy;
   R^{iv}, in each occurrence, is selected independently from hydrogen, alkyl, or - COR⁴ or CO₂R⁵;
   R^{v}, in each occurrence, is selected independently from alkyl, aryl, or -(CH₂)ₘcycloalkyl;
   R^{vi}, in each occurrence, is selected independently from halogen, alkyl or aryl;
   R⁴ represents cycloalkyl;
   R⁵ represents alkyl;
   m represents 0-2, inclusive;
   p is an integer from 0 to 3, inclusive;
   R^{r} and R^{s} independently represents -C(CH₃)₃ or -CF₃;
   R^{t} represents hydrogen or hydroxyl;
   R³, in each occurrence, is selected independently from
      (i) a cyclic group selected from 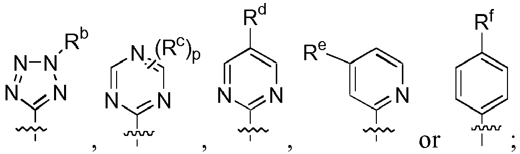 or
      (ii) -CO₂Et, when ZR¹ moiety represents S, SO, or SO₂;
   R^{b}, in each occurrence, is selected independently from (i) methyl, isopropyl,-(CH₂)₂OH, -(CH₂)₂OCH₃, -(CH₂)₂NHCO₂CH(CH₃)₃, -(CH₂)₂NH₂, -CH₂CH(CH₃)OH, -CH₂CO₂Et; (ii) 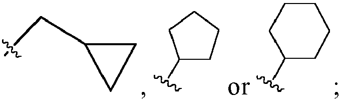
   R^{c} and R^{f}, in each occurrence, is selected independently from (i) hydrogen, halogen, alkyl, heterocyclyl comprising at least one heteroatom selected independently from >O, >N, or >S, , any of which having up to 6 carbon atoms; or (ii) NR^{y}R^{z}, wherein R^{y} and R^{z} independently represents hydrogen or alkyl group;
   R^{d}, in each occurrence, is selected independently from hydrogen, halogen, (C₁-C₃)alkyl, -N(CH₃)₂, 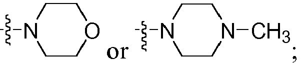
   R^{e} independently represents halogen;
   Z represents N; or the ZR¹ moiety represents S, SO or SO₂; or
      the ZR¹R² moiety independently represents
      (i) -C(O)Z¹R^{g} wherein R^{g} is hydrogen, cycloalkyl, aryl or (cycloalkyl)alkyl, Z¹ represents O or NR^{h}, wherein R^{h} is hydrogen, alkyl, cycloalkyl, or (cycloalkyl)alkyl; or
      (ii) 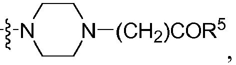 R⁵ represents heterocyclyl comprises comprising at least one heteroatom selected independently from >O, >N, or >S;
   R¹ and R², in each occurrence, is selected independently from ethyl,

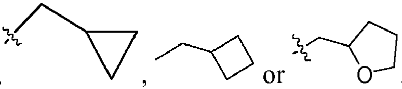

Another aspect of the present disclosure provides benzylamine compounds according to formula **(I),** having the formula: 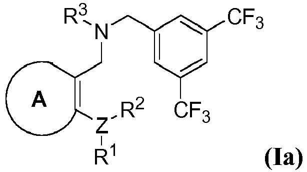 or a salt, a prodrug, a diastereomeric mixture, an enantiomer, a tautomer, a racemic mixture, or any combination thereof, wherein all symbols are as defined above.

Another aspect of the present disclosure provides benzylamine compounds according to formula **(Ia),** wherein A is selected from 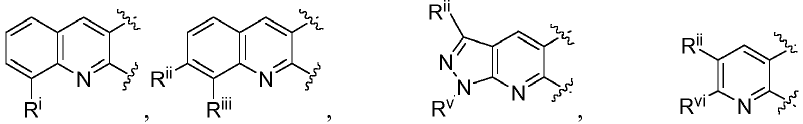 or 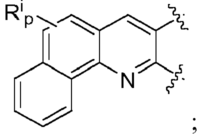 and all other symbols are as defined above.

Another aspect of the present disclosure provides benzylamine compounds according to formula **(Ia),** having the following formula: 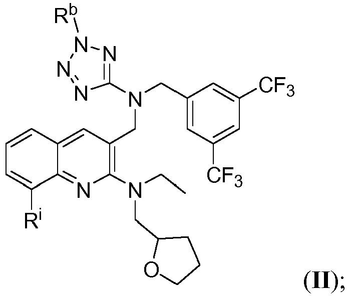 or a salt, a prodrug, a diastereomeric mixture, an enantiomer, a tautomer, a racemic mixture, or any combination thereof, wherein:
Rⁱ, is Cl, -OCH₃, -CH₃, -CH(CH₃)₂, or -CF₃;
R^{b} represents a) methyl, isopropyl, -CH₂-CH₂-OH, -CH₂-CH₂-OCH₃, -CH₂-CH₂-NH-CO₂CH(CH₃)₃, -CH₂-CH₂-NH₂, -CH₂-CH(CH₃)-OH, or -CH₂-CO₂Et; b)

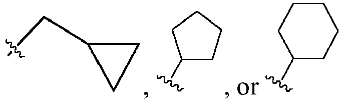

Another aspect of the present invention provides benzylamine compound according to formula **(II),** wherein Rⁱ and R^{b} independently represent -CH₃.

Another aspect of the present invention provides benzylamine compound according to formula **(II),** which is (*S*)-(+)-(3-{[(3,5-bis-trifluoromethyl-benzyl)-(2-methyl-2H-tetrazol-5-yl)-amino]methyl} -8-methyl-quinolin-2-yl)-ethyl-(tetrahydrofuran-2-ylmethyl)-amine

Another aspect of the present invention provides benzylamine compound according to formula **(II),** which is (*R*)-(-)-(3-{[(3,5-bis-trifluoromethyl-benzyl)-(2-methyl-2H-tetrazol-5-yl)-amino]methyl}-8-methyl-quinolin-2-yl)-ethyl-(tetrahydrofuran-2-ylmethyl)-amine

Another aspect of the present invention provides benzylamine compounds according to formula **(Ia),** having the following formula: 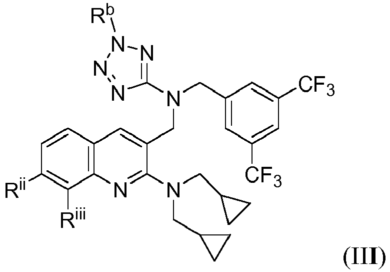 or a salt, a prodrug, a diastereomeric mixture, an enantiomer, a tautomer, a racemic mixture, or any combination thereof, wherein:
Rⁱⁱⁱ is -CH₃, CF₃, Cl, -OCH₃, or -CH(CH₃)₂;
Rⁱⁱ is H or -CH₃;
R^{b}, in each occurrence, is selected independently from (i) methyl, isopropyl,-(CH₂)₂OH, -(CH₂)₂OCH₃, -(CH₂)₂NHCO₂CH(CH₃)₃, -(CH₂)₂NH₂, -CH₂CH(CH₃)OH, -CH₂CO₂Et; (ii)

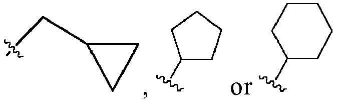

Another aspect of the present invention provides benzylamine compound according to formula (III), wherein Rⁱⁱ is hydrogen.

Another aspect of the present invention provides benzylamine compound according to formula (III), which is (3-{[3,5-bis trifluoromethyl-benzyl)-(2-cyclopropylmethyl-2H-tetrazole-5-yl)-amino]-methyl}-8-methyl-quinoline-2-yl)- bis-cyclopropylmethyl-amine; or a salt, a prodrug, a diastereomeric mixture, an enantiomer, a tautomer, or a racemic mixture.

Another aspect of the present invention provides benzylamine compound according to formula (III), which is (3-{[3,5-bistrifluoromethyl-benzyl)-(2-cyclopentyl -2H-tetrazole -5-yl)-amino]methyl-}-8-methyl-quinoline-2-yl)- bis-cyclopropylmethyl-amine; or a salt, a prodrug, a diastereomeric mixture, an enantiomer, a tautomer, or a racemic mixture.

Another aspect of the present invention provides benzylamine compound according to formula (III), which is (3-{[3,5-bistrifluoromethyl-benzyl)-(2-cyclohexyl -2H-tetrazole-5-yl)-amino]-methyl}-8-methyl-quinoline-2-yl)-bis-cyclopropylmethyl-amine; or a salt, a prodrug, a diastereomeric mixture, an enantiomer, a tautomer, or a racemic mixture.

Another aspect of the present invention provides benzylamine compound according to formula (III), which is (3-{[3,5-bis trifluoromethyl-benzyl)-(2-isopropyl -2H-tetrazole-5-yl)-amino]-methyl-}-8-methyl-quinoline-2-yl)-bis-cyclopropylmethyl-amine; or a salt, a prodrug, a diastereomeric mixture, an enantiomer, a tautomer, or a racemic mixture.

Another aspect of the present invention provides benzylamine compound according to formula (III), which is (2-{5-[[2-(bis-Cyclopropylmethyl-amino)-8-methyl-quinolin-3-ylmethyl]-(3,5-bis-trifluoromethyl-benzyl)-amino]-tetrazol-2-yl}-ethyl)-carbamic acid tert-butyl ester; or a salt, a prodrug, a diastereomeric mixture, an enantiomer, a tautomer, or a racemic mixture.

Another aspect of the present invention provides benzylamine compound according to formula (III), which is (3-{[[2-amino-ethyl)-2H-tetrazol-5-yl]-(3,5-bistrifluoromethyl-benzyl)-amino]-methyl}-8-methyl-quinolin-2-yl)-bis-cyclopropylmethyl-amine; or a salt, a prodrug, a diastereomeric mixture, an enantiomer, a tautomer, or a racemic mixture.

Another aspect of the present invention provides benzylamine compound according to formula (III), which is 1-{5-[[2-(bis-cyclopropylmethyl-amino)8-methyl-quinoline-3-ylmethyl]-(3,5-bistrifluoro methyl-benzyl)-amino]-tetrazol-2-yl}-propane-2-ol; or a salt, a prodrug, a diastereomeric mixture, an enantiomer, a tautomer, or a racemic mixture.

Another aspect of the present invention provides benzylamine compound according to formula (III), which is (3-{[(3,5-Bis-trifluoromethyl-benzyl)-(2-methyl-2H-tetrazol-5-yl)-amino]-methyl}-8-trifluoromethyl-quinolin-2-yl)-bis-cyclopropylmethyl-amine; or a salt, a prodrug, a diastereomeric mixture, an enantiomer, a tautomer, or a racemic mixture.

Another aspect of the present invention provides benzylamine compound according to formula (III), which is (3-{[3,5-bis trifluoromethyl-benzyl)-(2-methyl -2H-tetrazole -5-yl)-amino]-methyl-}-8-Chloro-quinoline-2-yl)- bis-cyclopropylmethyl-amine; or a salt, a prodrug, a diastereomeric mixture, an enantiomer, a tautomer, or a racemic mixture.

Another aspect of the present invention provides benzylamine compound according to formula (III), which is 3-{[3,5-bis trifluoromethyl-benzyl)-(2-methyl -2H-tetrazole -5-yl)-amino]-methyl-}-8-methoxy-quinoline-2-yl)- bis-cyclopropylmethyl-amine; or a salt, a prodrug, a diastereomeric mixture, an enantiomer, a tautomer, or a racemic mixture.

Another aspect of the present invention provides benzylamine compound according to formula (III), which is 3-{[3,5-bis trifluoromethyl-benzyl)-(2-methyl-2H-tetrazole -5-yl)-amino]-methyl-}-8-isopropyl-quinoline-2-yl)- bis-cyclopropylmethyl-amine; or a salt, a prodrug, a diastereomeric mixture, an enantiomer, a tautomer, or a racemic mixture.

Another aspect of the present invention provides benzylamine compound according to formula (III), which is 2-{5-[[2-(bis-cyclopropylmethyl-amino)-8-methyl-quinoline-3-ylmethyl]-(3,5-bis trifluoromethyl-benzyl)-amino]-tetrazol-2-yl}-ethanol; or a salt, a prodrug, a diastereomeric mixture, an enantiomer, a tautomer, or a racemic mixture.

Another aspect of the present invention provides benzylamine compound according to formula (III), which is [3-({(3,5-bis-trifluoromethyl-benzyl)-[2-(2-methoxy-ethyl)-2H-tetrazol-5-yl]-amino}-methyl)-8-methyl-quinolin-2-yl]-bis-cyclopropylmethyl-amine; or a salt, a prodrug, a diastereomeric mixture, an enantiomer, a tautomer, or a racemic mixture.

Another aspect of the present invention provides benzylamine compound according to formula (III), which is 3-{[3,5-bis trifluoromethyl-benzyl)-(2-methyl - 2H-tetrazole -5-yl)-amino]-methyl-}-7,8-dimethyl-quinoline-2-yl)- bis-cyclopropylmethyl-amine; or a salt, a prodrug, a diastereomeric mixture, an enantiomer, a tautomer, or a racemic mixture.

Another aspect of the present invention provides benzylamine compound according to formula (III), which is {5-[[2-(bis-cyclopropylmethyl-amino)-8-methylquinolin-3-ylmethyl]-(3,5-bis-trifluoromethyl-benzyl)-amino]-tetrazol-2-yl}-acetic acid ethyl ester; or a salt, a prodrug, a diastereomeric mixture, an enantiomer, a tautomer, or a racemic mixture.

Another aspect of the present disclosure provides benzylamine compounds according to formula **(Ia),** having the following formula: 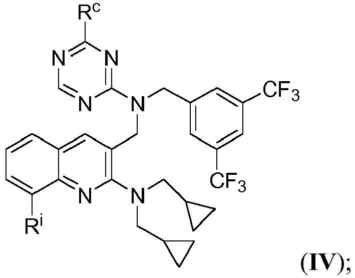 or a salt, a prodrug, a diastereomeric mixture, an enantiomer, a tautomer, a racemic mixture, or any combination thereof, wherein:
Rⁱ, is Cl, -OCH₃, -CH₃, -CH(CH₃)₂, or -CF₃;
R^{c} represents halogen, -N(CH₃)₂, or

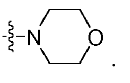

Another aspect of the present invention provides benzylamine compound according to formula (IV), wherein Rⁱ is -CH₃.

Another aspect of the present disclosure provides benzylamine compound according to formula (IV), which is (3-{[(3,5-Bis-trifluoromethyl-benzyl)-(4-chloro-[1,3,5]triazin-2-yl)-amino]-methyl}-8-methyl-quinolin-2-yl)-bis-cyclopropylmethyl-amine; or a salt, a prodrug, a diastereomeric mixture, an enantiomer, a tautomer, or a racemic mixture.

Another aspect of the present invention provides benzylamine compound according to formula (IV), which is N-[2-(Bis-cyclopropylmethyl-amino)-8-methyl-quinolin-3-ylmethyl]-N-(3,5-bis-trifluoromethyl-benzyl)-N',N'-dimethyl-[1,3,5]triazine-2,4-diamine; or a salt, a prodrug, a diastereomeric mixture, an enantiomer, a tautomer, or a racemic mixture.

Another aspect of the present invention provides benzylamine compound according to formula (IV), which is (3-{[(3,5-Bis-trifluoromethyl-benzyl)-(4-morpholin-4-yl-[1,3,5]triazin-2-yl)-amino]-methyl}-8-methyl-quinolin-2-yl)-bis-cyclopropylmethyl-amine; or a salt, a prodrug, a diastereomeric mixture, an enantiomer, a tautomer, or a racemic mixture.

Another aspect of the present disclosure provides benzylamine compounds according to formula **(Ia),** having the following formula: 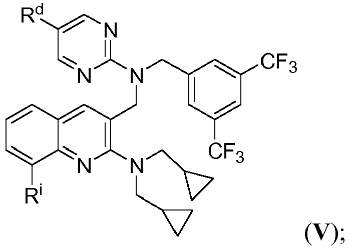 or a salt, a prodrug, a diastereomeric mixture, an enantiomer, a tautomer, a racemic mixture, or any combination thereof, wherein:
Rⁱ, is Cl, -OCH₃, -CH₃, -CH(CH₃)₂, -CF₃;
R^{d} represents hydrogen, -Br, - CH₂CH₃, -N(CH₃)₂,

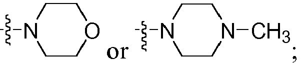

Another aspect of the present invention provides benzylamine compound according to formula (V), wherein Rⁱ represents -CH₃.

Another aspect of the present invention provides benzylamine compound according to formula (V), which is (3-{[(3,5-bis-trifluoromethyl-benzyl)-(5-bromo-pyrimidin-2-yl)-amino]-methyl}-8-methyl-quinolin-2-yl)-bis-cyclopropylmethyl-amine; or a salt, a prodrug, a diastereomeric mixture, an enantiomer, a tautomer, or a racemic mixture.

Another aspect of the present invention provides benzylamine compound according to formula (V), which is (3-{[(3,5-bis-trifluoromethyl-benzyl)-(5-morpholin-4-yl-pyrimidin-2-yl)-amino]-methyl}-8-methyl-quinolin-2-yl)-bis-cyclopropylmethyl-amine; or a salt, a prodrug, a diastereomeric mixture, an enantiomer, a tautomer, or a racemic mixture.

Another aspect of the present invention provides benzylamine compound according to formula (V), which is (3-{[(3,5-bis-trifluoromethyl-benzyl)-(5-ethyl-pyrimidin-2-yl)-amino]-methyl}-8-methyl-quinolin-2-yl)-bis-cyclopropylmethyl-amine; or a salt, a prodrug, a diastereomeric mixture, an enantiomer, a tautomer, or a racemic mixture.

Another aspect of the present invention provides benzylamine compound according to formula (V), which is *N*-[2-(bis-cyclopropylmethyl-amino)-8-methyl-quinolin-3-ylmethyl]-*N-(3,5*-bis-trifluoromethyl-benzyl)-*N'*,*N'*-dimethyl-pyrimidine-2,5-diamine; or a salt, a prodrug, a diastereomeric mixture, an enantiomer, a tautomer, or a racemic mixture.

Another aspect of the present invention provides benzylamine compound according to formula (V), which is [3-({((3,5-bis-trifluoromethyl-benzyl)-[5-(4-methyl-piperazin-1-yl)-pyrimidin-2-yl]-amino}-methyl)-8-methyl-quinolin-2-yl]-bis-cyclopropylmethyl-amine; or a salt, a prodrug, a diastereomeric mixture, an enantiomer, a tautomer, or a racemic mixture.

Another aspect of the present invention provides benzylamine compound according to formula (V), which is (3-{[(3,5-bis-trifluoromethyl-benzyl)-pyrimidin-2-yl-amino]-methyl}-8-methyl-quinolin-2-yl)-bis-cyclopropylmethyl-amine; or a salt, a prodrug, a diastereomeric mixture, an enantiomer, a tautomer, or a racemic mixture.

Another aspect of the present disclosure provides benzylamine compounds according to formula (Ia), having the formula: 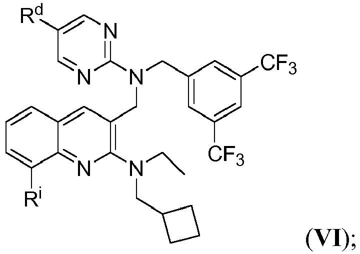 or a salt, a prodrug, a diastereomeric mixture, an enantiomer, a tautomer, a racemic mixture, or any combination thereof, wherein:
Rⁱ, is Cl, -OCH₃, -CH₃, -CH(CH₃)₂, -CF₃;
R^{d} represents hydrogen, -Br, -N(CH₃)₂,

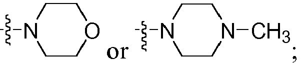

Another aspect of the present disclosure provides benzylamine compound according to formula (VI), wherein Rⁱ is -OCH₃ and R^{d} is -Br.

Another aspect of the present invention provides benzylamine compound according to formula (VI), which is (3-{[(3,5-bis-trifluoromethyl-benzyl)-(5-bromo-pyrimidin-2-yl)-amino]-methyl}-8-methyl-quinolin-2-yl)-cyclobutylmethyl-ethyl-amine; or a salt, a prodrug, a diastereomeric mixture, an enantiomer, a tautomer, or a racemic mixture.

Another aspect of the present disclosure provides benzylamine compounds according to formula (Ia), having the formula: 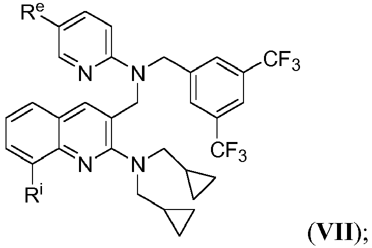 or a salt, a prodrug, a diastereomeric mixture, an enantiomer, a tautomer, a racemic mixture, or any combination thereof, wherein:
Rⁱ, is Cl, -OCH₃, -CH₃, -CH(CH₃)₂, or -CF₃;
R^{e} represents -Br.

Another aspect of the present invention provides benzylamine compound according to formula (VII), wherein Rⁱ is -CH₃ and R^{e} is -Br.

Another aspect of the present invention provides benzylamine compound according to formula (VII), which is (3-{[(3,5-bis-trifluoromethyl-benzyl)-(5-bromo-pyridin-2-yl)-amino]-methyl}-8-methyl-quinolin-2-yl)-bis- cyclopropylmethyl-amine; or a salt, a prodrug, a diastereomeric mixture, an enantiomer, a tautomer, or a racemic mixture.

Another aspect of the present disclosure provides benzylamine compounds according to formula (Ia), having the formula: 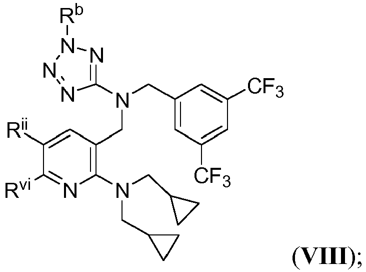 or a salt, a prodrug, a diastereomeric mixture, an enantiomer, a tautomer, a racemic mixture, or any combination thereof, wherein:
R^{vi}, is F, Cl, -CH₃, -CH₂CH₃, -CH(CH₃)₂ or phenyl;
Rⁱⁱ, is H or -CH₃;
R^{b} is -CH₃.

Another aspect of the present invention provides benzylamine compound according to formula (VIII), wherein
R^{vi}, is F, -CH₂CH₃, -CH(CH₃)₂ or phenyl;
Rⁱⁱ, is H or -CH₃;
R^{b} is -CH₃.

Another aspect of the present invention provides benzylamine compound according to formula (VIII), which is (3-{[(3,5-Bis-trifluoromethyl-benzyl)-(2-methyl-2H-tetrazol-5-yl)-amino]-methyl}-6-isopropyl-5-methyl-pyridin-2-yl)-bis-cyclopropylmethyl-amine; or a salt, a prodrug, a diastereomeric mixture, an enantiomer, a tautomer, or a racemic mixture.

Another aspect of the present invention provides benzylamine compound according to formula (VIII), which is (3-{[(3,5-Bis-trifluoromethyl-benzyl)-(2-methyl-2H-tetrazol-5-yl)-amino]-methyl}-6-isopropyl-pyridin-2-yl)-bis-cyclopropylmethyl-amine; or a salt, a prodrug, a diastereomeric mixture, an enantiomer, a tautomer, or a racemic mixture.

Another aspect of the present invention provides benzylamine compound according to formula (VIII), which is (3-{[(3,5-Bis-trifluoromethyl-benzyl)-(2-methyl-2H-tetrazol-5-yl)-amino]-methyl}-6-ethyl-5-methyl-pyridin-2-yl)-bis-cyclopropylmethyl-amine; or a salt, a prodrug, a diastereomeric mixture, an enantiomer, a tautomer, or a racemic mixture.

Another aspect of the present invention provides benzylamine compound according to formula (VIII), which is (3-{[3,5-Bis-trifluoromethyl-benzyl)-(2H-tetrazol-5-yl)-amino]-methyl}-5-methyl-6-phenyl-pyridin-2-yl)-bis-cyclopropylmethyl-amine; or a salt, a prodrug, a diastereomeric mixture, an enantiomer, a tautomer, or a racemic mixture.

Another aspect of the present invention provides benzylamine compound according to formula (VIII), which is (3-{[(3,5-Bis-trifluoromethyl-benzyl)-(2-methyl-2H-tetrazol-5-yl)-amino]-methyl}-6-fluoro-pyridin-2-yl)-bis-cyclopropylmethyl-amine; or a salt, a prodrug, a diastereomeric mixture, an enantiomer, a tautomer, or a racemic mixture.

Another aspect of the present disclosure provides benzylamine compounds according to formula (Ia), having the formula: 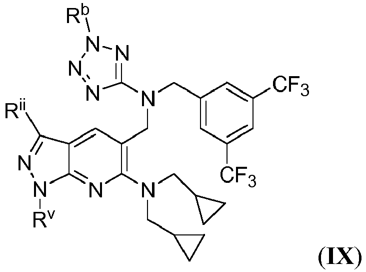 or a salt, a prodrug, a diastereomeric mixture, an enantiomer, a tautomer, a racemic mixture, or any combination thereof, wherein:
R^{v}, is -CH₃, -CH₂CH₃, -CH(CH₃)₂, phenyl, or 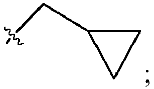
Rⁱⁱ is -H or -CH₃;
R^{b}, in each occurrence, is selected independently from (i) methyl, isopropyl,-(CH₂)₂OH, -(CH₂)₂OCH₃, -(CH₂)₂NHCO₂CH(CH₃)₃, -(CH₂)₂NH₂, -CH₂CH(CH₃)OH, -CH₂CO₂Et; (ii)

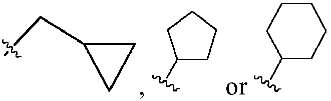

Another aspect of the present invention provides benzylamine compound according to formula (IX), wherein
R^{v}, is -CH₃ or phenyl;
Rⁱⁱ is -H or -CH₃;
R^{b} is methyl.

Another aspect of the present invention provides benzylamine compound according to formula (IX), which is (5-{[(3,5-bis-trifluoromethyl-benzyl)-(2-methyl-2H-tetrazol-5-yl)-amino-methyl}-1-phenyl-1H-pyrazolo[3,4-b]pyridin-6-yl)-bis-cyclopropylmethyl-amine; or a salt, a prodrug, a diastereomeric mixture, an enantiomer, a tautomer, or a racemic mixture.

Another aspect of the present disclosure provides benzylamine compounds according to formula (Ia), having the formula: 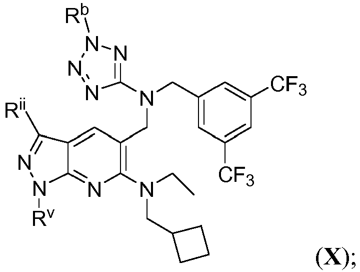 or a salt, a prodrug, a diastereomeric mixture, an enantiomer, a tautomer, a racemic mixture, or any combination thereof, wherein:
R^{v}, is -CH₃, -CH₂CH₃, -CH(CH₃)₂, phenyl, or 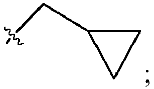
Rⁱⁱ is -H or -CH₃;
R^{b}, in each occurrence, is selected independently from (i) methyl, isopropyl,-(CH₂)₂OH, -(CH₂)₂OCH₃, -(CH₂)₂NHCO₂CH(CH₃)₃, -(CH₂)₂NH₂, -CH₂CH(CH₃)OH, -CH₂CO₂Et; (ii)

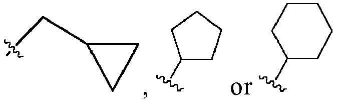

Another aspect of the present disclosure provides benzylamine compound according to formula (X), wherein
R^{v} is 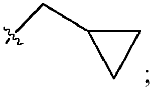 Rⁱⁱ is -H or -CH₃;
R^{b is} methyl.

Another aspect of the present invention provides benzylamine compound according to formula (X), which is 5-{[(3,5-bis-trifluoromethyl-benzyl)-(2-methyl-2H-tetrazol-5-yl)-amino]methyl}-1-cyclopropylmethyl-1H-pyrazolo[3,4-b]pyridin-6-yl)-cyclobutylmethyl-ethyl-amine; or a salt, a prodrug, a diastereomeric mixture, an enantiomer, a tautomer, or a racemic mixture.

Another aspect of the present disclosure provides benzylamine compounds according to formula (Ia), having the formula: 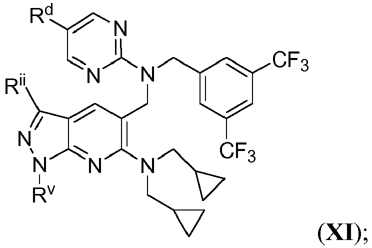 or a salt, a prodrug, a diastereomeric mixture, an enantiomer, a tautomer, a racemic mixture, or any combination thereof, wherein:
R^{v}, is -CH₃, -CH₂CH₃, -CH(CH₃)₂, phenyl, or 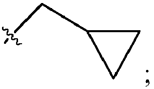
Rⁱⁱ is -H or -CH₃;
R^{d} represents hydrogen, -Br, -N(CH₃)₂,

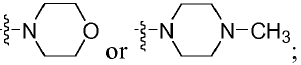

Another aspect of the present invention provides benzylamine compound according to formula (XI), wherein
R^{v}, is -CH₃ or -CH₂CH₃;
Rⁱⁱ is -H or -CH₃;
R^{d} is -Br or

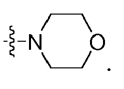

Another aspect of the present disclosure provides benzylamine compound according to formula (XI), which is (5-{[(3,5-bis-trifluoromethyl-benzyl)-(5-bromo-pyrimidin-2-yl)-amino]-methyl}-1,3-dimethyl-1H-pyrazolo[3,4-b]pyridin-6-yl)-bis-cyclopropylmethyl-amine; or a salt, a prodrug, a diastereomeric mixture, an enantiomer, a tautomer, or a racemic mixture.

Another aspect of the present invention provides benzylamine compound according to formula (XI), which is (5-{[(3,5-bis-trifluoromethyl-benzyl)-(5-bromo-pyrimidin-2-yl)-amino]-methyl}-1-ethyl-1H-pyrazolo[3,4-b]pyridin-6-yl)-bis-cyclopropylmethyl-amine; or a salt, a prodrug, a diastereomeric mixture, an enantiomer, a tautomer, or a racemic mixture.

Another aspect of the present invention provides benzylamine compound according to formula (XI), which is (5-{[(3,5-bis-trifluoromethyl-benzyl)-(5-(4-morpholin-4-yl-phenyl)-pyrimidin-2-yl]-amino}-methyl)-1-ethyl-1H-pyrazolo[3,4-b]pyridin-6-yl)-bis-cyclopropylmethyl-amine; or a salt, a prodrug, a diastereomeric mixture, an enantiomer, a tautomer, or a racemic mixture.

Another aspect of the present disclosure provides benzylamine compounds according to formula (Ia), having the formula: 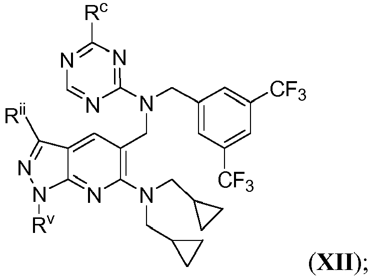 or a salt, a prodrug, a diastereomeric mixture, an enantiomer, a tautomer, a racemic mixture, or any combination thereof, wherein:
R^{v} is -CH₃, -CH₂CH₃, -CH(CH₃)₂, phenyl, or 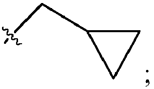
Rⁱⁱ is -H or -CH₃;
R^{c} represents halogen, -N(CH₃)₂,

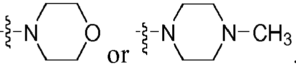

Another aspect of the present invention provides benzylamine compound according to formula (XII), wherein
R^{v} is -CH₃;
Rⁱⁱ is -CH₃;
R^{c} is -N(CH₃)₂..

Another aspect of the present invention provides benzylamine compound according to formula (XII), which is (5-{[(3,5-Bis-trifluoromethyl-benzyl)-(4-N,N dimethylamino-[1,3,5]triazin-2-yl)-amino]-methyl}-1,3-dimethyl-1H-pyrazolo[3,4-b]pyridin-6-yl)-bis-cyclopropylmethyl-amine; or a salt, a prodrug, a diastereomeric mixture, an enantiomer, a tautomer, or a racemic mixture.

Another aspect of the present disclosure provides benzylamine compounds according to formula (Ia), having the formula: 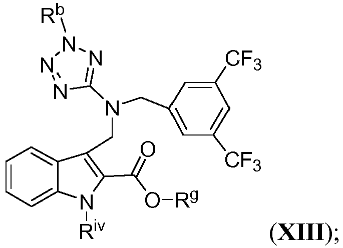 or a salt, a prodrug, a diastereomeric mixture, an enantiomer, a tautomer, a racemic mixture, or any combination thereof, wherein:
R^{iv} is H, -CH₃, -CH₂CH₃, -CH₂CH₂CH₃, -CO₂CH₂CH₃, 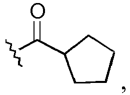 or 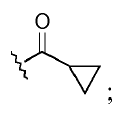
R^{b} is -CH₃;
R^{g} is -CH₂CH₃.

Another aspect of the present disclosure provides benzylamine compound according to formula (XIII), which is 3-{[(3,5-Bis-trifluormethyl-benzyl)-(2-methyl-2H-tetrazol-5-yl)-amino]-methyl}-1H-indole-2-carboxylic acid ethyl ester; or a salt, a prodrug, a diastereomeric mixture, an enantiomer, a tautomer, or a racemic mixture.

Another aspect of the present disclosure provides benzylamine compound according to formula (XIII), which is 3-{[(3,5-bis-trifluoromethyl-benzyl)-(2-methyl-2H-tetrazol-5-yl)-amino]-methyl}-1-methyl-1H-indole-2-carboxylic acid ethyl ester; or a salt, a prodrug, a diastereomeric mixture, an enantiomer, a tautomer, or a racemic mixture.

Another aspect of the present disclosure provides benzylamine compound according to formula (XIII), which is 3-{[(3,5-bis-trifluoromethyl-benzyl)-(2-methyl-2H-tetrazol-5-yl)-amino]-methyl}-1-propyl-1H-indole-2-carboxylic acid ethyl ester; or a salt, a prodrug, a diastereomeric mixture, an enantiomer, a tautomer, or a racemic mixture.

Another aspect of the present disclosure provides benzylamine compound according to formula (XIII), which is 3-{[(3,5-bis-trifluoromethyl-benzyl)-(2-methyl-2H-tetrazol-5-yl)-amino]-methyl} -1-cyclopentanecarbonyl-1H-indole-2-carboxylic acid ethyl ester; or a salt, a prodrug, a diastereomeric mixture, an enantiomer, a tautomer, or a racemic mixture.

Another aspect of the present disclosure provides benzylamine compound according to formula (XIII), which is 3-{[(3,5-bis-trifluoromethyl-benzyl)-(2-methyl-2H-tetrazol-5-yl)-amino]-methyl}-1-ethyl-1H-indole-2-carboxylic acid ethyl ester; or a salt, a prodrug, a diastereomeric mixture, an enantiomer, a tautomer, or a racemic mixture.

Another aspect of the present disclosure provides benzylamine compound according to formula (XIII), which is 3-{[(3,5-bis-trifluoromethyl-benzyl)-(2-methyl-2H-tetrazol-5-yl)-amino]-methyl}-indole-1,2-carboxylic acid ethyl ester; or a salt, a prodrug, a diastereomeric mixture, an enantiomer, a tautomer, or a racemic mixture.

Another aspect of the present disclosure provides benzylamine compound according to formula (XIII), which is 3-{[(3,5-bis-trifluoromethyl-benzyl)-(2-methyl-2H-tetrazol-5-yl)-amino]-methyl} -1-cyclopropanecarbonyl-1H-indole-2-carboxylic acid ethyl ester; or a salt, a prodrug, a diastereomeric mixture, an enantiomer, a tautomer, or a racemic mixture.

Another aspect of the present disclosure provides benzylamine compounds according to formula (Ia), having the formula: 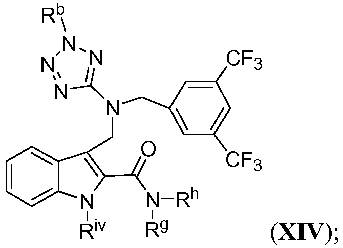 or a salt, a prodrug, a diastereomeric mixture, an enantiomer, a tautomer, a racemic mixture, or any combination thereof, wherein:
R^{iv} is H, -CH₃, -CH₂CH₃, -CH₂CH₂CH₃, CO₂ CH₂CH₃, 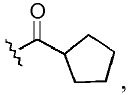 or 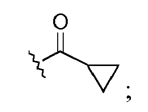
R^{b} is -CH₃;
R^{g} is -CH₂CH₃ or 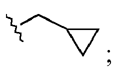
R^{h} is -CH₂CH₃ or

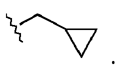

Another aspect of the present disclosure provides benzylamine compound according to formula (XIV), wherein R^{iv} is H, -CH₃ or -CH₂CH₂CH₃.

Another aspect of the present disclosure provides benzylamine compound according to formula (XIV), which is 3-{[(3,5-Bis-trifluoromethyl-benzyl)-(2-methyl-2H-tetrazol-5-yl)-amino]-methyl}-3H-indole-2-carboxylic acid diethylamide; or a salt, a prodrug, a diastereomeric mixture, an enantiomer, a tautomer, or a racemic mixture.

Another aspect of the present disclosure provides benzylamine compound according to formula (XIV), which is 3-{[(3,5-bis-trifluoromethyl-benzyl)-(2-methyl-2H-tetrazol-5-yl)-amino]-methyl}-1-methyl-1H-indole-2-carboxylic acid diethylamide; or a salt, a prodrug, a diastereomeric mixture, an enantiomer, a tautomer, or a racemic mixture.

Another aspect of the present disclosure provides benzylamine compound according to formula (XIV), which is 3-{[(3,5-bis-trifluoromethyl-benzyl)-(2-methyl-2H-tetrazol-5-yl)-amino]-methyl}-1-methyl-1H-indole-2-carboxylic acid bis-cyclopropylmethyl-amide; or a salt, a prodrug, a diastereomeric mixture, an enantiomer, a tautomer, or a racemic mixture.

Another aspect of the present disclosure provides benzylamine compound according to formula (XIV), which is 3-{[(3,5-bis-trifluoromethyl-benzyl)-(2-methyl-2H-tetrazol-5-yl)-amino]-methyl}-1-propyl-1H-indole-2-carboxylic acid bis-cyclopropylmethyl-amide; or a salt, a prodrug, a diastereomeric mixture, an enantiomer, a tautomer, or a racemic mixture.

Another aspect of the present disclosure provides benzylamine compounds according to formula (Ia), having the formula: 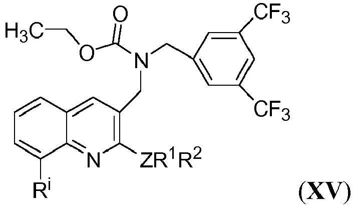 or a salt, a prodrug, a diastereomeric mixture, an enantiomer, a tautomer, a racemic mixture, or any combination thereof, wherein:
Rⁱ is hydrogen or -CH₃;
ZR¹ moiety represents S, SO or SO₂;
R² is -CH₃, -CH₂CH₃ or -CH₂CH₂CH₃.

Another aspect of the present invention provides benzylamine compound according to formula (XV), wherein
Rⁱ is -CH₃;
R² is -CH₂CH₃.

Another aspect of the present invention provides benzylamine compound according to formula (XV), which is (3,5-bis-trifluoromethyl-benzyl)-(8-methyl-2-propylsulfanyl-quinolin-3-ylmethyl)-carbamic acid ethyl ester; or a salt, a prodrug, a diastereomeric mixture, an enantiomer, a tautomer, or a racemic mixture.

Another aspect of the present invention provides benzylamine compound according to formula (XV), which is (3,5-Bis-trifluoromethyl-benzyl)-[8-methyl-2-(propane-1-sulfonyl)-quinolin-3-ylmethyl]-carbamic acid ethyl ester; or a salt, a prodrug, a diastereomeric mixture, an enantiomer, a tautomer, or a racemic mixture.

Another aspect of the present invention provides benzylamine compound according to formula (XV), which is (3,5-Bis-trifluoromethyl-benzyl)-[8-methyl-2-(propane-1-sulfinyl)-quinolin-3-ylmethyl]-carbamic acid ethyl ester; or a salt, a prodrug, a diastereomeric mixture, an enantiomer, a tautomer, or a racemic mixture.

Another aspect of the present disclosure provides benzylamine compounds according to formula (Ia), having the formula: 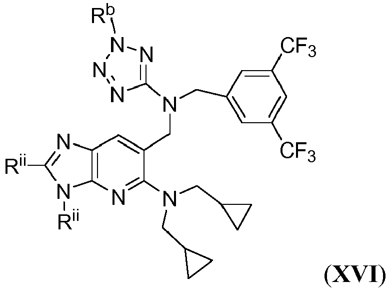 or a salt, a prodrug, a diastereomeric mixture, an enantiomer, a tautomer, a racemic mixture, or any combination thereof, wherein:
Rⁱⁱ, in each occurrence, independently represent hydrogen, -CH₃, -CH₂CH₃, or -CH(CH₃)₂;
R^{b}, in each occurrence, is selected independently from (i) methyl, isopropyl,-(CH₂)₂OH, -(CH₂)₂OCH₃, -(CH₂)₂NHCO₂CH(CH₃)₃, -(CH₂)₂NH₂, -CH₂CH(CH₃)OH, -CH₂CO₂Et; (ii)

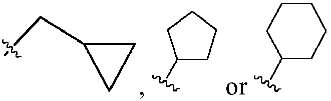

Another aspect of the present disclosure provides benzylamine compound according to formula (XVI), wherein
Rⁱⁱ is -CH₃, R^{b} is methyl.

Another aspect of the present disclosure provides benzylamine compound according to formula (XVI), which is (6-{[(3,5-Bis-trifluoromethyl-benzyl)-(2-methyl-2H-tetrazol-5-yl)-amino]-methyl}-2,3-dimethyl-3H-imidazo[4,5-b]pyridin-5-yl)-bis-cyclopropylmethyl-amine; or a salt, a prodrug, a diastereomeric mixture, an enantiomer, a tautomer, or a racemic mixture.

Another aspect of the present disclosure provides benzylamine compounds according to formula (Ia), having the formula: 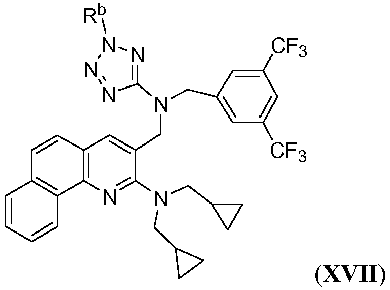 or a salt, a prodrug, a diastereomeric mixture, an enantiomer, a tautomer, a racemic mixture, or any combination thereof, wherein:
R^{b}, in each occurrence, is selected independently from (i) methyl, isopropyl,-(CH₂)₂OH, -(CH₂)₂OCH₃, -(CH₂)₂NHCO₂CH(CH₃)₃, -(CH₂)₂NH₂, -CH₂CH(CH₃)OH, -CH₂CO₂Et; (ii)

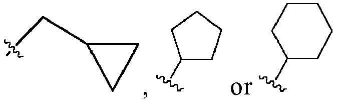

Another aspect of the present disclosure provides benzylamine compound according to formula (XVI), which is 3-{[3,5-bis trifluoromethyl-benzyl)-(2-methyl -2H-tetrazole -5-yl)-amino]-methyl-}-benzo[h]quinolin-2-yl)- bis-cyclopropylmethyl-amine; or a salt, a prodrug, a diastereomeric mixture, an enantiomer, a tautomer, or a racemic mixture.

Another aspect of the present disclosure provides benzylamine compounds according to formula (Ia), having the formula: 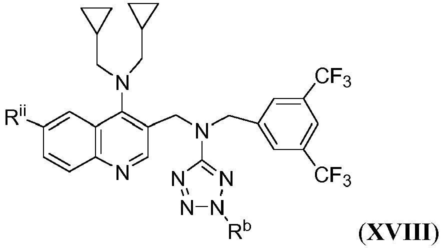 or a salt, a prodrug, a diastereomeric mixture, an enantiomer, a tautomer, a racemic mixture, or any combination thereof, wherein
Rⁱⁱ is H, -CH₃ or -CH(CH₃)₂;
R^{b}, in each occurrence, is selected independently from (i) methyl, isopropyl,-(CH₂)₂OH, -(CH₂)₂OCH₃, -(CH₂)₂NHCO₂CH(CH₃)₃, -(CH₂)₂NH₂, -CH₂CH(CH₃)OH, -CH₂CO₂Et; (ii)

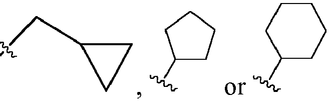

Another aspect of the present disclosure provides benzylamine compound according to formula (XVIII), wherein
Rⁱⁱ is H or -CH₃;
R^{b} is methyl.

Another aspect of the present disclosure provides benzylamine compound according to formula (XVIII), which is 3-{[(3,5-Bis-trifluoromethyl-benzyl)-(2-methyl-2H-tetrazol-5-yl)-amino]-methyl}-6-methyl-quinolin-4-yl)-bis-cyclopropylmethyl-amine; or a salt, a prodrug, a diastereomeric mixture, an enantiomer, a tautomer, or a racemic mixture.

Another aspect of the present invention provides benzylamine compound according to formula (XVIII), which is (3-{[(3,5-Bis-trifluoromethyl-benzyl)-(2-methyl-2H-tetrazol-5-yl)-amino]-methyl}-quinolin-4-yl)-bis-cyclopropylmethyl-amine; or a salt, a prodrug, a diastereomeric mixture, an enantiomer, a tautomer, or a racemic mixture.

Another aspect of the present disclosure invention provides benzylamine compound according to formula (I), which is 2-[4-(3-{[(3,5-Bis-trifluoromethylbenzyl)-(5-bromo-pyrimidin-2-yl)-amino]-methyl}-8-methyl-quinolin-2-yl)-piperazin-1-yl]-1-pyrrolidin-1-yl-ethanone; or a salt, a prodrug, a diastereomeric mixture, an enantiomer, a tautomer, or a racemic mixture.

Another aspect of the present invention provides benzylamine compound according to formula (I), which is [3-({((3,5-bis-trifluoromethyl-benzyl)-[(4-morpholin-4-yl-phenyl)-amino]-methyl)-8-methyl-quinolin-2-yl]-bis-cyclopropylmethyl-amine; or a salt, a prodrug, a diastereomeric mixture, an enantiomer, a tautomer, or a racemic mixture.

Another aspect of the present invention provides benzylamine compound according to formula (I), which is 4-{[[2-(bis-cyclopropylmethyl-amino)8-methyl-quinoline-3-ylmethyl]-(2-methyl-2H-tetrazol-5-yl)-amino]-methyl}-2,6-di-*tert*-butylphenol; or a salt, a prodrug, a diastereomeric mixture, an enantiomer, a tautomer, or a racemic mixture.

Another aspect of the present invention provides benzylamine compound according to formula (I), which is selected from
(3-{[3,5 -bis trifluoromethyl-benzyl)-(2-cyclopropylmethyl -2H-tetrazole -5-yl)-amino]-methyl-}-8-methyl-quinoline-2-yl)- bis-cyclopropylmethyl-amine;
(3-{[3,5 -bis trifluoromethyl-benzyl)-(2-cyclopentyl -2H-tetrazole -5-yl)-amino]-methyl-}-8-methyl-quinoline-2-yl)- bis-cyclopropylmethyl-amine;
(3-{[3,5 -bis trifluoromethyl-benzyl)-(2-cyclohexyl -2H-tetrazole -5-yl)-amino]-methyl-}-8-methyl-quinoline-2-yl)- bis-cyclopropylmethyl-amine;
(3-{[3,5 -bis trifluoromethyl-benzyl)-(2-isopropyl -2H-tetrazole -5-yl)-amino]-methyl-}-8-methyl-quinoline-2-yl)- bis-cyclopropylmethyl-amine;
(2-{5-[[2-(bis-Cyclopropylmethyl-amino)-8-methyl-quinolin-3-ylmethyl]-(3,5-bis-trifluoromethyl-benzyl)-amino]-tetrazol-2-yl}-ethyl)-carbamic acid tert-butyl ester;
(3-{[[2-amino-ethyl)-2H-tetrazol-5-yl]-(3,5-bistrifluoromethyl-benzyl)-amino]-methyl}-8-methyl-quinolin-2-yl)-bis-cyclopropylmethyl-amine;
1-{5-[[2-(bis-cyclopropylmethyl-amino)8-methyl-quinoline-3-ylmethyl]- (3,5-bistrifluoromethyl-benzyl)-amino]-tetrazol-2-yl}-propane-2-ol;
(3-{[(3,5-Bis-trifluoromethyl-benzyl)-(2-methyl-2H-tetrazol-5-yl)-amino]-methyl}-8-trifluoromethyl-quinolin-2-yl)-bis-cyclopropylmethyl-amine;
(3-{[3,5 -bis trifluoromethyl-benzyl)-(2-methyl -2H-tetrazole -5-yl)-amino]-methyl-}-8-Chloro-quinoline-2-yl)- bis-cyclopropylmethyl-amine;
3-{[3,5 -bis trifluoromethyl-benzyl)-(2-methyl -2H-tetrazole -5-yl)-amino]-methyl-}-8-methoxy-quinoline-2-yl)- bis-cyclopropylmethyl-amine;
3-{[3,5 -bis trifluoromethyl-benzyl)-(2-methyl -2H-tetrazole -5-yl)-amino]-methyl-}-8-isopropyl-quinoline-2-yl)- bis-cyclopropylmethyl-amine;
2-{5-[[2-(bis-cyclopropylmethyl-amino)-8-methyl-quinoline-3-ylmethyl]- (3,5-bistrifluoromethyl-benzyl)-amino]-tetrazol-2-yl}-ethanol;
[3-({(3,5-bis-trifluoromethyl-benzyl)-[2-(2-methoxy-ethyl)-2H-tetrazol-5-yl]-amino}-methyl)-8-methyl-quinolin-2-yl]-bis-cyclopropylmethyl-amine;
3-{[3,5 -bis trifluoromethyl-benzyl)-(2-methyl -2H-tetrazole -5-yl)-amino]-methyl-}-7,8-dimethyl-quinoline-2-yl)- bis-cyclopropylmethyl-amine;
(S)-(+)-(3-{[(3,5-bis-trifluoromethyl-benzyl)-(2-methyl-2H-tetrazol-5-yl)-amino]methyl}-8-methyl-quinolin-2-yl)-ethyl-(tetrahydro-furan-2-ylmethyl)-amine;
(R)-(-)-(3-{[(3,5-bis-trifluoromethyl-benzyl)-(2-methyl-2H-tetrazol-5-yl)-amino]methyl}-8-methyl-quinolin-2-yl)-ethyl-(tetrahydro-furan-2-ylmethyl)-amine;
(5-{[(3,5-bis-trifluoromethyl-benzyl)-(2-methyl-2H-tetrazol-5-yl)-amino]-methyl}-1-phenyl-1H-pyrazolo[3,4-b]pyridin-6-yl)-bis-cyclopropylmethyl-amine;
(5-{[(3,5-bis-trifluoromethyl-benzyl)-(5-bromo-pyrimidin-2-yl)-amino]-methyl}-1,3-dimethyl-1H-pyrazolo[3,4-b]pyridin-6-yl)-bis-cyclopropylmethyl-amine;
(5-{[(3,5-bis-trifluoromethyl-benzyl)-(2-methyl-2H-tetrazol-5-yl)-amino]methyl}-1-cyclopropylmethyl-1H-pyrazolo [3,4-b]pyridin-6-yl)-cyclobutylmethyl-ethyl-amine;
(5-{[(3,5-bis-trifluoromethyl-benzyl)-(5-bromo-pyrimidin-2-yl)-amino]-methyl}-1-ethyl-1H-pyrazolo [3,4-b]pyridin-6-yl)-bis-cyclopropylmethyl-amine;
(5-{[(3,5-bis-trifluoromethyl-benzyl)-(5-(4-morpholin-4-yl-phenyl)-pyrimidin-2-yl]-amino}-methyl)-1-ethyl-1H-pyrazolo[3,4-b]pyridin-6-yl)-bis-cyclopropylmethyl-amine;
(3-{[(3,5-bis-trifluoromethyl-benzyl)-(5-bromo-pyrimidin-2-yl)-amino]-methyl}-8-methyl-quinolin-2-yl)-cyclobutylmethyl-ethyl-amine;
(3-{[(3,5-bis-trifluoromethyl-benzyl)-(5-bromo-pyrimidin-2-yl)-amino]-methyl}-8-methyl-quinolin-2-yl)-bis-cyclopropylmethyl-amine;
(3-{[(3,5-bis-trifluoromethyl-benzyl)-(5-morpholin-4-yl-pyrimidin-2-yl)-amino]-methyl}-8-methyl-quinolin-2-yl)-bis-cyclopropylmethyl-amine;
(3-{[(3,5-bis-trifluoromethyl-benzyl)-(5-ethyl-pyrimidin-2-yl)-amino]-methyl}-8-methyl-quinolin-2-yl)-bis-cyclopropylmethyl-amine;
N-[2-(bis-cyclopropylmethyl-amino)-8-methyl-quinolin-3-ylmethyl]-N-(3,5-bis-trifluoromethyl-benzyl)-N',N'-dimethyl-pyrimidine-2,5-diamine;
[3-({((3,5-bis-trifluoromethyl-benzyl)-[5-(4-methyl-piperazin-1-yl)-pyrimidin-2-yl]-amino}-methyl)-8-methyl-quinolin-2-yl]-bis-cyclopropylmethyl-amine;
(3-{[(3,5-bis-trifluoromethyl-benzyl)-pyrimidin-2-yl-amino]-methyl}-8-methyl-quinolin-2-yl)-bis-cyclopropylmethyl-amine;
(3-{[(3,5-bis-trifluoromethyl-benzyl)-(5-bromo-pyridin-2-yl)-amino]-methyl}-8-methyl-quinolin-2-yl)-bis-cyclopropylmethyl-amine;
(3-{[(3,5-Bis-trifluoromethyl-benzyl)-(2-methyl-2H-tetrazol-5-yl)-amino]-methyl}-6-isopropyl-5-methyl-pyridin-2-yl)-bis-cyclopropylmethyl-amine;
(3-{[(3,5-Bis-trifluoromethyl-benzyl)-(2-methyl-2H-tetrazol-5-yl)-amino]-methyl}-6-isopropyl-pyridin-2-yl)-bis-cyclopropylmethyl-amine;
(3-{[(3,5-Bis-trifluoromethyl-benzyl)-(2-methyl-2H-tetrazol-5-yl)-amino]-methyl}-6-ethyl-5-methyl-pyridin-2-yl)-bis-cyclopropylmethyl-amine;
(3-{[3,5-Bis-trifluoromethyl-benzyl)-(2H-tetrazol-5-yl)-amino]-methyl}-5-methyl-6-phenyl-pyridin-2-yl)-bis-cyclopropylmethyl-amine;
{5-[[2-(bis-cyclopropylmethyl-amino)-8-methyl-quinolin-3-ylmethyl]-(3,5-bis-trifluoromethyl-benzyl)-amino]-tetrazol-2-yl}-acetic acid ethyl ester;
(3-{[(3,5-Bis-trifluoromethyl-benzyl)-(4-chloro-[1,3,5]triazin-2-yl)-amino]-methyl}-8-methyl-quinolin-2-yl)-bis-cyclopropylmethyl-amine;
N-[2-(Bis-cyclopropylmethyl-amino)-8-methyl-quinolin-3-ylmethyl]-N-(3,5-bis-trifluoromethyl-benzyl)-N',N'-dimethyl-[1,3,5]triazine-2,4-diamine;
(3-{[(3,5-Bis-trifluoromethyl-benzyl)-(4-morpholin-4-yl-[1,3,5]triazin-2-yl)-amino]-methyl}-8-methyl-quinolin-2-yl)-bis-cyclopropylmethyl-amine;
(5-{[(3,5-Bis-trifluoromethyl-benzyl)-(4-N,N,dimethylamino-[1,3,5]triazin-2-yl)-amino]-methyl}-1,3-dimethyl-1H-pyrazolo[3,4-b]pyridin-6-yl)-bis-cyclopropylmethyl-amine;
3-{[(3,5-Bis-trifluoromethyl-benzyl)-(2-methyl-2H-tetrazol-5-yl)-amino]-methyl}-6-methyl-quinolin-4-yl)-bis-cyclopropylmethyl-amine;
(3,5-bis-trifluoromethyl-benzyl)-(8-methyl-2-propylsulfanyl-quinolin-3-ylmethyl)-carbamic acid ethyl ester;
(3,5-Bis-trifluoromethyl-benzyl)-[8-methyl-2-(propane-1-sulfonyl)-quinolin-3-ylmethyl]-carbamic acid ethyl ester;
(3,5-Bis-trifluoromethyl-benzyl)-[8-methyl-2-(propane-1-sulfinyl)-quinolin-3-ylmethyl]-carbamic acid ethyl ester;
(3-{[(3,5-Bis-trifluoromethyl-benzyl)-(2-methyl-2H-tetrazol-5-yl)-amino]-methyl}-6-fluoro-pyridin-2-yl)-bis-cyclopropylmethyl-amine;
2-[4-(3-{[(3,5-Bis-trifluoromethyl-benzyl)-(5-bromo-pyrimidin-2-yl)-amino]-methyl}-8-methyl-quinolin-2-yl)-piperazin-1-yl]-1-pyrrolidin-1-yl-ethanone;
[3-({((3,5-bis-trifluoromethyl-benzyl)-[(4-morpholin-4-yl-phenyl)-amino]-methyl)-8-methyl-quinolin-2-yl]-bis-cyclopropylmethyl-amine;
4-{[[2-(bis-cyclopropylmethyl-amino) -8-methyl-quinolin-3-ylmethyl]-(2-methyl-2H-tetrazol-5-yl)-amino]-methyl}-2,6-di-*tert*-butyl-phenol; or
a salt, a prodrug, a diastereomeric mixture, an enantiomer, a tautomer, or a racemic mixture.

Another aspect of the present disclosure provides benzylamine compound according to formula (I), which is selected from
3-{[3,5 -bis trifluoromethyl-benzyl)-(2-methyl -2H-tetrazole -5-yl)-amino]-methyl-}-benzo[h]quinolin-2-yl)- bis-cyclopropylmethyl-amine;
(3-{[(3,5-Bis-trifluoromethyl-benzyl)-(2-methyl-2H-tetrazol-5-yl)-amino]-methyl}-quinolin-4-yl)-bis-cyclopropylmethyl-amine;
(6-{[(3,5-Bis-trifluoromethyl-benzyl)-(2-methyl-2H-tetrazol-5-yl)-amino]-methyl}-2,3-dimethyl-3H-imidazo[4,5-b]pyridin-5-yl)-bis-cyclopropylmethyl-amine;
3-{[(3,5-Bis-trifluormethyl-benzyl)-(2-methyl-2H-tetrazol-5-yl)-amino]-methyl}-1H-indole-2-carboxylic acid ethyl ester;
3-{[(3,5-bis-trifluoromethyl-benzyl)-(2-methyl-2H-tetrazol-5-yl)-amino]-methyl}-1-methyl-1H-indole-2-carboxylic acid ethyl ester;
3-{[(3,5-bis-trifluoromethyl-benzyl)-(2-methyl-2H-tetrazol-5-yl)-amino]-methyl}-1-propyl-1H-indole-2-carboxylic acid ethyl ester;
3-{[(3,5-bis-trifluoromethyl-benzyl)-(2-methyl-2H-tetrazol-5-yl)-amino]-methyl}-1-cyclopentanecarbonyl-1H-indole-2-carboxylic acid ethyl ester;
3-{[(3,5-bis-trifluoromethyl-benzyl)-(2-methyl-2H-tetrazol-5-yl)-amino]-methyl}-1-ethyl-1H-indole-2-carboxylic acid ethyl ester;
3-{[(3,5-bis-trifluoromethyl-benzyl)-(2-methyl-2H-tetrazol-5-yl)-amino]-methyl}-indole-1,2-carboxylic acid ethyl ester;
3-{[(3,5-bis-trifluoromethyl-benzyl)-(2-methyl-2H-tetrazol-5-yl)-amino]-methyl}-1-cyclopropanecarbonyl-1H-indole-2-carboxylic acid ethyl ester;
3-{[(3,5-Bis-trifluoromethyl-benzyl)-(2-methyl-2H-tetrazol-5-yl)-amino]-methyl}-3H-indole-2-carboxylic acid diethylamide;
3-{[(3,5-bis-trifluoromethyl-benzyl)-(2-methyl-2H-tetrazol-5-yl)-amino]-methyl}-1-methyl-1H-indole-2-carboxylic acid diethylamide;
3-{[(3,5-bis-trifluoromethyl-benzyl)-(2-methyl-2H-tetrazol-5-yl)-amino]-methyl}-1-methyl-1H-indole-2-carboxylic acid bis-cyclopropylmethyl-amide;
3-{[(3,5-bis-trifluoromethyl-benzyl)-(2-methyl-2H-tetrazol-5-yl)-amino]-methyl}-1-propyl-1H-indole-2-carboxylic acid bis-cyclopropylmethyl-amide; or
a salt, a prodrug, a diastereomeric mixture, an enantiomer, a tautomer, or a racemic mixture.

By the disclosure of these specific compounds, it is intended to include any salt, including a pharmaceutically acceptable or a non-pharmaceutically acceptable salt, any prodrug, and any stereoisomer, including diastereomeric mixtures, enantiomers, tautomers, racemic mixtures, or any combinations thereof, of the disclosed compounds.

The present disclosure also encompasses any combination of compounds provided herein, including any salts, including pharmaceutically acceptable and non-pharmaceutically acceptable salts, or any mixture thereof. The present invention also encompasses any stereoisomers of compounds provided herein, including any combination of stereoisomers.

In this aspect of the present invention, compounds provided herein can be chiral or achiral, or they may exist as racemic mixtures, diastereomers, pure enantiomers, a prodrug, a tautomer or any mixture thereof. For chiral compounds, separate enantiomers, separate diastereomers, and any mixture of enantiomers, diastereomers, or both are encompassed herein, such as, for example, (R), (S), or a mixture of (R) and (S) isomers. In this aspect, individual optical isomers or a particular desired isomer may be obtained by using chiral reagents to obtain a single isomeric form in a resolution process wherever applicable, or by conducting the reaction in the presence of reagents or catalysts in their single enantiomeric or diasteromeric form.

Methods for the resolution of racemic compounds include, but are not limited to: using microbial resolution; resolving the diastereomeric salts formed with chiral acids such as mandelic acid, camphorsulfonic acid, tartaric acid, lactic acid, and the like wherever applicable; or resolving the diastereomeric salts formed with chiral bases such as brucine, cinchona alkaloids and their derivatives; and the like. Commonly used methods are compiled in Jaques, et al. in Enantiomers, Racemates and Resolution; Wiley-Interscience, 1981. For example, where appropriate, compounds of formula (I) can be resolved by treating with chiral amines, aminoacids, or aminoalcohols derived from aminoacids; by using conventional reaction conditions to convert an acid into an amide; by separation of diastereomers by fractional crystallization or by chromatography; or by preparing the stereoisomers of formula (I) by hydrolyzing the pure diastereomeric amide.

As used herein, the terms "pharmaceutically acceptable" salt or "pharmacologically acceptable" salt refers generally to a salt or complex of the compound or compounds in which the compound can be either anionic or cationic, and have associated with it a counter cation or anion, respectively, that is generally considered suitable for human or animal consumption. For example, a pharmaceutically acceptable salt can refer to a salt of a compound disclosed herein that forms upon reaction or complexation with an acid whose anion is generally considered suitable for human or animal consumption. In this aspect, pharmacologically acceptable salts include salts with organic acids or inorganic acids. Examples of pharmacologically acceptable salts include, but are not limited to, hydrochloride, hydrobromide, hydroiodide, sulfate, phosphate, propionate, lactate, maleate, malate, succinate, tartarate, and the like.

Salts may also be formed by deprotonating an acid moiety of the compound, such as a carboxylic acid moiety, OH, or NH, and the like, using a base such as an organic base, an inorganic base, an organometallic base, a Lewis base, a Bronsted base, or any combination thereof. In cases where compounds carry an acidic moiety, suitable pharmaceutically acceptable salts can include alkali metal salts, alkaline earth metal salts, or salts with organic basis, and the like. In this aspect, examples of alkali metal salts include, but are not limited to, sodium and potassium salts, and examples of salts with organic basis include, but are not limited to, meglumine salts, and the like. The pharmacologically acceptable salts can be prepared by conventional means. Additional examples of pharmaceutically acceptable salts, and methods of preparing such salts, are found, for example, in Berg et.al., J. Pharma. Sci, 66, 1-19 (1977).

In a further aspect, this invention also provides a composition comprising a pharmaceutically acceptable carrier and at least one compound of the invention. In this aspect, the at least one compound can be present as a neutral compound, as a salt, or as any combination thereof. This invention also encompasses a composition comprising at least one compound of the invention, and optionally comprising a pharmaceutically acceptable additive selected from a carrier, an auxiliary, a diluent, an excipient, a preservative, a solvate, or any combination thereof. In another aspect, this invention encompasses a pharmaceutical composition comprising at least one compound of the invention, and optionally further comprising an agent selected from a chemotherapeutic agent, an immunosuppressive agent, a cytokine, a cytotoxic agent, an anti-inflammatory agent, an antidyspilidemic agent, an antirheumatic agent, a cardiovascular agent, or any combination thereof.

Further, this invention encompasses a pharmaceutical composition, comprising at least one compound of the invention, and optionally comprising a pharmaceutically acceptable additive selected from a carrier, an auxiliary, a diluent, an excipient, a preservative, a solvate, or any combination thereof, wherein the pharmaceutical composition is in the form of a tablet, a capsule, a syrup, a cachet, a powder, a granule, a solution, a suspension, an emulsion, a bolus, a lozenge, a suppository, a cream, a gel, a paste, a foam, a spray, an aerosol, a microcapsule, a liposome, or a transdermal patch.

In another aspect of this disclosure, alternatively, the compounds can be formulated and administered in a prodrug form. In general, prodrugs comprise functional derivatives of the claimed compounds which are capable of being enzymatically activated or converted into the more active parent form. Thus, in the treatment methods of the present invention, the term "administering" encompasses the treatment of the various disorders described with the compound specifically disclosed or with a compound which may not be specifically disclosed, but which converts to the specified compound *in vivo* after administration to the patient. Conventional procedures for the selection and preparation of suitable prodrug derivatives are described, for example, in Wihnan, 14 Biochem. Soc. Trans. 375-82 (1986); Stella et al., Prodrugs: A Chemical Approach to Targeted Drug Delivery, in Directed Drug Delivery 247-67 (1985).

Thus, in one aspect, "prodrugs" of the compounds disclosed herein refers to species that have chemically- or metabolically-cleavable groups wherein, under physiological conditions, the species become, provide, release, or are transformed into the compounds disclosed herein. In this manner, prodrugs can release the pharmaceutically *in vivo* active compounds disclosed herein. For example, prodrugs of present invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs, optionally substituted phenylacetamide-containing prodrugs, 5-fluorocytosine or other 5-fluorouridine prodrugs which may be converted into the more active species, and the like. In another aspect, prodrugs of present invention include, but are not limited to derivatives of carboxylic acid, sulfonamide, amine, hydroxyl, and the like, including other functional groups and including any combination thereof.

In another aspect, this invention provides a pharmaceutical composition, comprising one or more compounds of the invention and optionally comprising a pharmaceutically acceptable additive selected from a carrier, an auxiliary, a diluent, an excipient, a preservative, a solvate, or any combination thereof. In a related aspect, this invention affords a composition for use in a method of treating a condition or disease state such as dyslipidemia, atherosclerosis, peripheral vascular disease, hypertryglyceridemia, hypercholesterolemia, hyperbetalipoproteinemia, hypoalphalipoprotenemia, cardiovascular disorders such as angina, ischemia, stroke, myocardial infarction (MI), reperfusion injury, restenosis and hypertension, and diabetic vascular diseases such as diabetic retinopathy, and endotoxemia, comprising administering an effective amount of at least one compound as disclosed herein.

### SYNTHETIC METHODS

General reaction schemes are provided herein that detail the synthetic approaches to the benzylamine compounds disclosed herein. Thus, compounds in accordance with this disclosure could be prepared as shown in the specific Schemes and/or as illustrated in the Examples by using standard synthetic methods and starting materials, which are either commercially available or can be synthesized from commercially available precursors using synthetic methods known in the art, or variations thereof as appreciated by those skilled in the art. Each variable in the following schemes refer to any group consistent with the description of the compounds provided herein. In each synthetic scheme or example provided, substitutents in any structure that are illustrated in the scheme or example that are not specified are selected as disclosed according to the general formulas of the compounds provided herein.

The following general procedures could be used in the reactions schemes and in the Examples provided herein.

Halogenation could be carried out by using reagents such as phosphorus oxychloride (POCl₃), thionyl chloride (SOCl₂), and the like, for example, at a temperature from about 80°C to about 120°C, for about 4 to about 8 hours, followed by pH adjustment of resultant mixture to a pH from about 6 to about 7.

Amination could be carried out by using amines in presence of a solvent chosen from acetone, acetonitrile, dimethylformamide, dimethylacetamide and the like, with or with out a base. Suitable bases include triethylamine, N,N-diisopropyl ethyl amine, potassium carbonate, sodium carbonate, sodium hydride, and the like. The reaction temperature was typically from about 20°C to about 120°C, and the duration of the reaction was typically in the range of from about 4 hours to about 20 hours.

Thus one further aspect of the invention relates to the processes of preparing compounds of formulas provided herein. Any compound of any formula disclosed herein can be obtained using procedures provided in the reaction Schemes, as well as procedures provided in the Examples, by selecting suitable starting materials and following analogous procedures. Thus, any compound of any formula disclosed or exemplified herein, can be obtained by using the appropriate starting materials and appropriate reagents, with the desired substitutions, and following procedures analogous to those described herein. Therefore, it will be readily understood by one of ordinary skill, that the reaction schemes disclosed herein can be adapted to prepare any compound of this disclosure, therefore any discussion of a particular step in a reaction scheme is intended to reflect one method or one set of considitions that can be used to carry out that step. This discussion of a particular step is not intended to be limiting, but rather exemplary, of one particular method and set of conditions by which that step can be effected.

In one aspect of this disclosure, compounds of formula (I) according to this invention could be prepared as illustrated in at least one of the following Schemes 1-5. In some cases, the relevant reagents and starting materials were commercially available. In other cases, the relevant reagents and starting materials were made by standard synthetic procedures in organic and heterocyclic chemistry, and known by one of ordinary skill in the relevant art. These techniques were analogous to the synthesis of known structurally similar intermediates or starting materials and the procedures described in preparations and examples below. For example the following references disclosed the exact procedures or analogues procedures for the preparation of many of the intermediates described in Schemes 1-5: Synlett., 2001, No: 2, 251-253; Synthesis 2001, 1185-1196; Journal of Heterocyclic Chemistry, 1987, 351-355; Journal of Organic Chemistry Vol: 30, 1965, 3593-3596; Journal of Heterocyclic Chemistry 1982, 809-811. Such known procedures include the reduction of aldehydes, cyanation, alkylation of amines, benzylation, acylation of amines, sulfonylation of amines, reductive amination, hydrolysis of nitriles, esterification of carboxylic acids and carboxylic acids to amide conversions, and the like.

Thus, in the following representative synthetic schemes, the starting materials were either commercially available or readily prepared using well known procedures, using starting materials and/or reagents having the appropriate substitution. As the context of any scheme or Example demands or allows, substitutents in any structure that are not specified are selected as provided herein in the general description of the disclosed compounds.

In one aspect, compounds according to the present invention could be prepared according to the following scheme.

In one aspect, compounds according to the present invention could be prepared according to the following scheme.

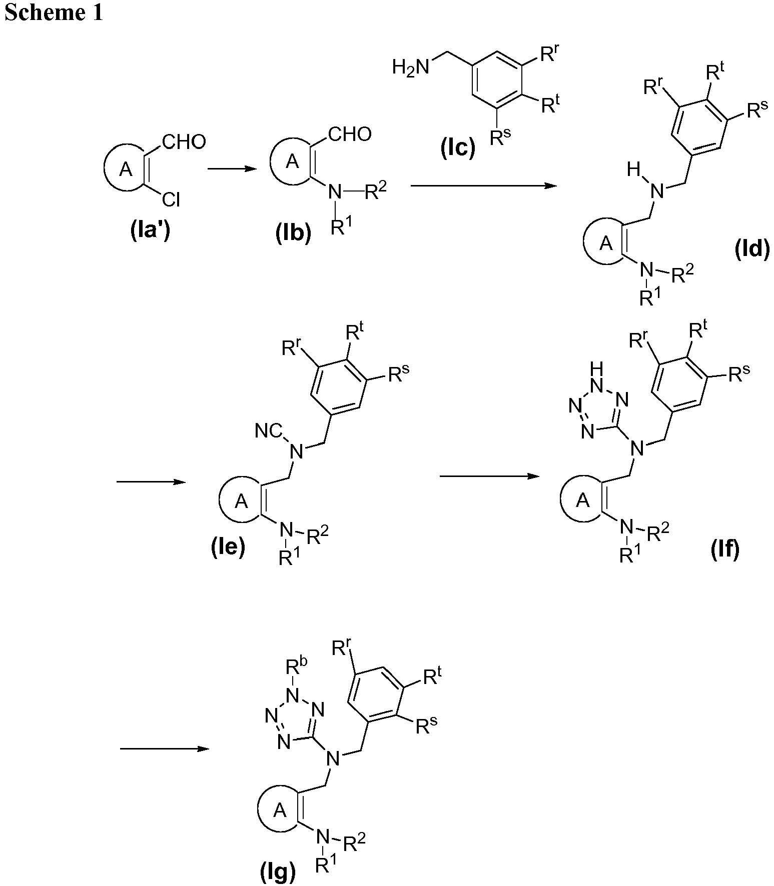

Representative steps of Scheme 1 include the following. The compound of formula **(Ia')** could be converted to a compound of formula (Ib) by an amination reaction using HNR¹R², in a polar solvent such as N,N-dimethylformamide (DMF) and a base such as sodium carbonate or potassium carbonate. The reaction could also be carried out in the presence of a solvent such as acetonitrile, tetrahydrofuran, or toluene. The base could also be selected from cesium carbonate, potassium tertiary butoxide, and the like.

Reductive amination of compound of formula (Ib) with a compound of formula **(Ic),** in presence of a reducing agent such as Na(CN)BH₃, Na(OAc)₃BH, NaBH₄ and the like, in a (C₁-C₁₀) alcohol solvent such as methanol, ethanol, propanol, isopropanol, and the like, or a chlorinated solvent such as dichloromethane, chloroform, 1,2-dichloroethane, and the like, along with an acid such as acetic acid or diluted hydrochloric acid, yields a compound of formula (I), wherein R³ is typically hydrogen. In one aspect, the temperature of the reaction could be maintained from about 25 °C to about 35 °C, and the duration of the reaction typically could range from about 30 minutes to about 5 hours.

The compound of formula (Id), could be converted to a compound of formula (Ie), in the presence of cyanogen bromide (CNBr), by using a suitable solvent such as dimethylformamide, acetonitrile, a (C₁-C₁₀) alcohol, or the like, along with a base such as sodium bicarbonate, sodium carbonate, potassium carbonate, cesium carbonate, potassium bicarbonate, and the like. In one aspect, the temperature of the reaction could be maintained from about 25 °C to about 55 °C, and the duration of the reaction typically could range from about 20 minutes to about 5 hours.

The compound of formula **(Ie),** can be converted to a compound of formula **(If),** by reacting the cyano compound with sodium azide or potassium azide, in the presence zinc salts such as ZnBr₂. Suitable solvents for this reaction include N,N-dimethylformamide, acetonitrile, (C₁-C₁₀) alcohols, and the like.

Compounds of formula **(If)** could be converted into a compound of formula **(Ig)** where R^{b} is as defined above, by reacting the tetrazolyl compound with corresponding reagents such as alkyl halides or dialkyl sulphates, in the presence of a base such as sodium hydroxide, potassium hydroxide, potassium carbonate, sodium hydride and the like, along with a phase-transfer catalyst such as tetraalkylammoniumhalide or tetraarylammoiumhalide, in a solvent medium such as water, dimethylformamide, acetonitrile, and the like.

In yet another aspect, compounds according to the present invention could be prepared according to the following scheme.

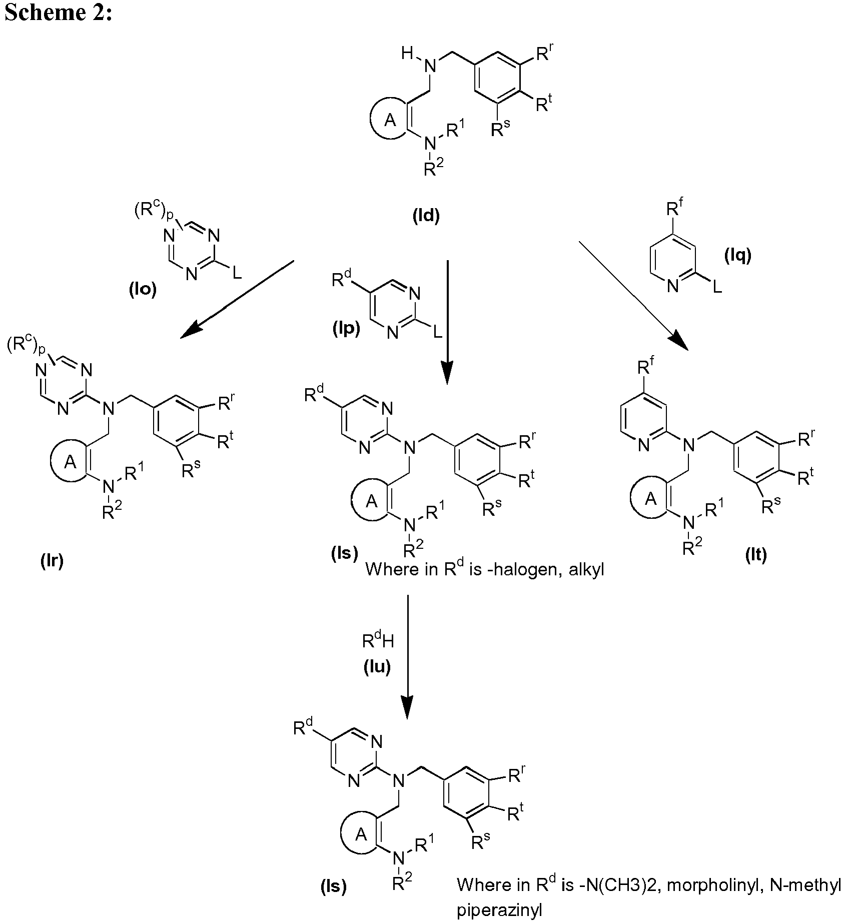

The compound of formula **(Id)** could be converted to a compound of formulae **(Ir), (Is)** and **(It)** by reacting with a compound of formulae (**Io**), (**Ip**) and (Iq) respectively wherein L represents a leaving group such as halide. The base such as triethylamine, disiopropylethylamine, potassium carbonate, sodiumcarbonate can be used. The reaction could also be carried out in the presence of different solvents, including but not limited to, acetone, acetonitrile, N,N-dimethylformamide, dimethylsulfoxide and the like.

The compound of formula **(Is)** wherein R^{d} is halogen could be coverted to a compound of formula **(Is),** wherein R^{d} is -N(CH₃)₂, morpholinyl, N-methyl piperazinyl by reacting with compound of formula (Iu) wherein R^{d} is -N(CH₃)₂, morpholinyl, N-methyl piperazinyl by known reactions like Buchwald-Hartwig Cross Coupling Reaction

In yet another aspect, compounds according to the present invention could be prepared according to the following scheme.

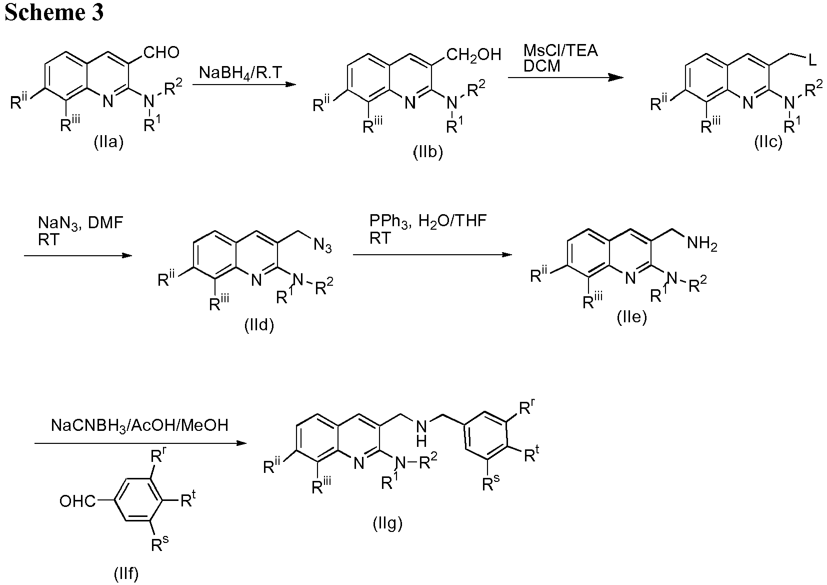

Representative steps of Scheme 3 include the following:
The compound of formula (IIa) could be converted to a compound of formula (IIb) by carring out reduction, in a polar solvent such as methanol and a reagent such as sodiumborohydrate.

The compound of formula (IIb) could be converted to a compound of formula (IIc), wherein L is leaving group, by recting with a reagent such as methansulfonylchlride or p-toluensulfonylchloride, in a solvent such as dichloromethane.

The compound of formula (IIc) could be converted to a compound of formula (IIe) by recting with sodiumazide or potasiumazide, followed by coverting azide group to -NH₂ group by conventional methods.

The compound of formula (IIe) could be converted to a compound of formula (IIg) by carrying out reductive amination with compound of formula (IIf).

Compounds disclosed herein to control CETP activity can be used for preventing or treating a variety of conditions or diseases such as ones associated with lipoprotein metabolism. Without being held to a particular theory, it is believed that CETP activity can affect the level of circulating cholesterol-containing HDL. Increased CETP can produce a decrease in HDL-C levels relative to LDL-C and/or VLDL-C levels. For example, CETP plays a role in transferring cholesteryl ester from HDL to VLDL and LDL, and thereby in altering the relative profile of circulating lipoproteins to one which is associated with an increased risk of cardiovascular disease (for example, decreased levels of HDL-C and increased levels of VLDL-C and LDL-C). Further, increased levels of CETP activity can be predictive of increased risk of cardiovascular disease. Modulation or inhibition of CETP activity, therefore, can be a prophylactic or therapeutic method for modulating the relative levels of lipoproteins to reduce or prevent the progression of, to induce regression of, or reduce risk of development of a variety of conditions or diseases including cardiovascular diseases, such as atherosclerosis.

Effective amounts are administered to the subject in dosages and formulations that are safe and effective, including, but not limited to, the ranges taught herein. As disclosed herein, compositions comprising at least one compound having a formula as disclosed herein, and/or their pharmaceutically-acceptable salts, can be used in conjunction with other prophylactic or therapeutic agents or in methods optionally comprising steps such as altered patient activities, including, but not limited to, changes in exercise or diet.

In one aspect, the present invention provides a composition for use in a method of treating or preventing a condition or disease in a mammalian subject, the method comprising administering to the subject a composition comprising a prophylactically- or therapeutically-effective amount of at least one compound of the invention, and/or their pharmaceutically-acceptable salts. In various aspects, the condition or disease is dyslipidemia, atherosclerosis, a peripheral vascular disease, hypertryglyceridemia, hypercholesterolemia, hyperbetalipoproteinemia, hypoalphalipoprotenemia, a cardiovascular disorder (i.e., angina, ischemia, stroke, myocardial infarction (MI), reperfusion injury, restenosis, hypertension) or diabetic vascular diseases (i.e., diabetic retinopathy, endotoxemia).

In one other aspect, the present invention provides a composition for use in a method of decreasing or inhibiting CETP activity in a mammalian subject, the method comprising administering to the subject an amount of a composition comprising at least one compound of the invention, and/or their pharmaceutically-acceptable salts, wherein the amount is sufficient to decrease or inhibit CETP activity in the subject.

In still another aspect, the present invention provides a composition for use in a method of increasing high density lipoprotein (HDL) in a mammalian subject, the method comprising administering to the subject an amount of a composition comprising at least one compound of the invention, and/or their pharmaceutically-acceptable salts, wherein the amount is sufficient to increase high density lipoprotein (HDL) in the subject.

In another aspect, the present invention provides a composition for use in a method of elevating the ratio of circulating HDL to circulating LDL, VLDL, or total cholesterol in a mammalian subject, the method comprising administering to the subject a prophylactically- or therapeutically-effective amount of at least one compound of the invention, and/or their pharmaceutically-acceptable salts.

In yet another aspect, the present invention provides a composition for use in a method of altering catabolism of HDL-cholesterol to decrease development of atherosclerotic lesions in a mammalian subject, the method comprising administering to the subject an amount of a composition comprising at least one compound of the invention, and/or their pharmaceutically-acceptable salts, wherein the amount is sufficient to alter the catabolism of HDL-cholesterol thereby leading to decreased development of atherosclerotic lesions.

In still another aspect, the present invention provides a composition for use in a method of decreasing low density lipoprotein (LDL) in a mammalian subject, the method comprising administering to the subject an amount of a composition comprising at least one compound of the invention, and/or their pharmaceutically-acceptable salts, wherein the amount is sufficient to decrease low density lipoprotein (LDL).

In another aspect, the present invention provides a composition for use in a method of treating or preventing atherosclerosis in a mammalian subject, the method comprising administering to the subject a prophylactically- or therapeutically-effective amount of at least one compound of the invention, and/or their pharmaceutically-acceptable salts.

In yet another aspect, the present invention provides a composition for use in a method of treating or preventing hyperlipidemia in a mammalian subject, the method comprising administering to the subject a prophylactically- or therapeutically-effective amount of at least one compound of the invention, and/or their pharmaceutically-acceptable salts.

In another aspectthis invention provides a composition for use in a method of treating or preventing a CETP-mediated disorder in a mammalian subject, the method comprising administering to the subject a prophylactically- or therapeutically-effective amount of at least one compound of the invention, and/or their pharmaceutically-acceptable salts.

In yet another aspect, the present invention provides a composition for use in a method of treating or preventing dyslipidemia, atherosclerosis, a peripheral vascular disease, hypertryglyceridemia, hypercholesterolemia, hyperbetalipoproteinemia, hypoalphalipoprotenemia, a cardiovascular disorder, a diabetic vascular disease, or endotoxemia. In one aspect, the cardiovascular disorder is angina, ischemia, stroke, myocardial infarction (MI), reperfusion injury, restenosis or hypertension.

The compounds of the present invention are useful in the treatment and / or prophylaxis of the above said diseases in combination / concomittant with one or more LDL-cholesterol lowering agents such as HMG CoA reductase inhibitors; cholesterol absorption inhibitors; antiobesity drugs; lipoprotein disorder treatment drugs; hypoglycemic agents: insulin; biguanides; sulfonylureas; thiazolidinediones; dual PPAR agonists; and/or mixtures thereof. The compounds of the present invention in combination with HMG CoA reductase inhibitors, microsomal triglyceride transfer protein (MTP) /ApoB secretion inhibitors, cholesterol absorption inhibitors, antiobesity drugs, hypoglycemic agents can be administered together or within in such a period of time so as to act synergistically.

In one aspect, the present invention provides a prophylactic or therapeutic composition comprising at least one compound of the invention, and/or their pharmaceutically-acceptable salts and, optionally an antihypertensive agent. Hypertension can be characterized as persistently high blood pressure. Illustratively, an adult having a systolic blood pressure that is persistently at least about 140 mmHg or a diastolic blood pressure that is at least about 90 mmHg can be classified as hypertensive. Hyperlipidemic conditions such as atherosclerosis can have an affect on hypertension.

The dosage regimen utilizing, the compounds of the present invention is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician, veterinarian or clinician can readily determine and prescribe the effective amount of the drug required to prevent, counter or arrest the progress of the condition.

Compounds and compositions of the present invention can be administered by any appropriate route, including, for example, orally, parenterally, intravenously, intradermally, intramuscularly, subcutaneously, sublingually, transdermally, bronchially, pharyngolaryngeal, intranasally, topically such as by a cream or ointment, rectally, intraarticular, intracisternally, intrathecally,
intravaginally, intraperitoneally, intraocularly, by inhalation, bucally or as an oral or nasal spray.

Oral dosages of compositions of the present invention, when used for the indicated effects, will range from about 0.01 mg per kg of body weight per day (mg/kg/day) to about 100 mg/kg/day. Advantageously, compounds of the present invention can be administered in a single daily dose, or the total daily dosage may be administered in divided doses of two, three or four times daily. Furthermore, preferred compounds for the present invention can be administered in intranasal form *via* topical use of suitable intranasal vehicles, or *via* transdermal routes, using those forms of transdermal skin patches well known to those of ordinary skill in the art. To be administered in the form of a transdermal delivery system, the dosage administration will, of course, be substantially continuous rather than intermittent throughout the dosage regimen.

The compounds herein described in detail can form the active ingredient, and are typically administered in admixture with suitable pharmaceutical diluents, excipients or carriers (collectively referred to herein as 'carrier' materials) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic, pharmaceutically acceptable, inert carrier such as lactose, starch, sucrose, glucose, methyl cellulose, magnesium stearate, dicalcium phosphate, calcium sulfate, mannitol, sorbitol and the like; for oral administration in liquid form, the oral drug components can be combined with any oral, non-toxic, pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include starch, gelatin, natural sugars such as glucose or betalactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

In one aspect, the present invention provides a composition comprising at least one compound of the invention.

In another aspect, this invention provides a pharmaceutical composition, comprising:
at least one compound of the invention; and
optionally comprising a pharmaceutically acceptable additive selected from a carrier, an auxiliary, a diluent, an excipient, a preservative, a solvate, or any combination thereof.

In yet another aspect, this invention provides a pharmaceutical composition, comprising:
at least one compound of the invention; and
optionally comprising a pharmaceutically acceptable additive selected from a carrier, an auxiliary, a diluent, an excipient, a preservative, a solvate, or any combination thereof;
wherein the pharmaceutical composition is in the form of a tablet, a capsule, a syrup, a cachet, a powder, a granule, a solution, a suspension, an emulsion, a bolus, a lozenge, a suppository, a cream, a gel, a paste, a foam, a spray, an aerosol, a microcapsule, a liposome, or a transdermal patch.

In still another aspect, this invention provides a pharmaceutical composition, comprising:
at least one compound of the invention;
optionally comprising a pharmaceutically acceptable additive selected from a carrier, an auxiliary, a diluent, an excipient, a preservative, a solvate, or any combination thereof; and
further comprising an agent selected from a chemotherapeutic agent, an immunosuppressive agent, a cytokine, a cytotoxic agent, an anti-inflammatory agent, an antirheumatic agent, an antidyspilidemic agent, a cardiovascular agent, or any combination thereof

Accordingly, in addition to the compounds of the invention, the pharmaceutical compositions of the present invention can further comprise at least one of any suitable auxiliary such as, but not limited to, diluent, binder, stabilizer, buffers, salts, lipophilic solvents, preservative, adjuvant, or the like. In one aspect of the present invention, pharmaceutically acceptable auxiliaries are employed. Examples and methods of preparing such sterile solutions are well known in the art and can be found in well known texts such as, but not limited to, REMINGTON'S PHARMACEUTICAL SCIENCES (Gennaro, Ed., 18th Edition, Mack Publishing Co. (1990)). Pharmaceutically acceptable carriers can be routinely selected that are suitable for the mode of administration, solubility and/or stability of the compound.

For oral administration in the form of a tablet or capsule, a compound can be combined with an oral, non-toxic pharmaceutically acceptable inert carrier such as ethanol, glycerol, water, and the like. Moreover, when desired or necessary, suitable binders, lubricants, disintegrating agents, and coloring agents may also be incorporated into the mixture. Suitable binders include, without limitation, starch; gelatin; natural sugars such as glucose or beta-lactose; corn sweeteners; natural and synthetic gums such as acacia, tragacanth, or sodium alginate, carboxymethylcellulose; polyethylene glycol; waxes; and the like. Lubricants used in these dosage forms include, without limitation, sodium oleate, sodium stearate, magnesium stearate, sodium benzoate, sodium acetate, sodium chloride, and the like. Disintegrators include, without limitation, starch, methyl cellulose, agar, bentonite, xanthan gum, and the like.

The pharmaceutical preparations contain at least one compound of the present invention, and/or a pharmaceutically acceptable salt thereof, in an amount effective to inhibit CETP activity and prevent or treat the various conditions or diseases attributable to CETP activity. One skilled in the art can easily determine such an effective amount. The preparations optionally can contain other ingredients including, for example, an antihypertensive drug.

Formulations of the present invention suitable for oral administration can be presented as discrete units such as capsules, cachets, or tablets each containing a predetermined amount of the active ingredient; as a powder or granules; as a solution or a suspension in an aqueous liquid or a non-aqueous liquid; or as an oil-in-water liquid emulsion or a water-in-oil emulsion and as a bolus, and the like.

The invention further relates to the administration of at least one compound of the invention by the following routes, including, but not limited to oral, parenteral, subcutaneous, intramuscular, intravenous, intrarticular, intrabronchial, intraabdominal, intracapsular, intracartilaginous, intracavitary, intracelial, intracelebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, bolus, vaginal, rectal, buccal, sublingual, intranasal, iontophoretic means, or transdermal means.

A composition comprising at least one compound of the present invention can be administered at a frequency and for a period of time effective to achieve a therapeutic effect, which should be understood in the context of a regimen of repeated administration at such a frequency and over such a period. In some aspects, a composition is administered at a frequency and for a period of time effective to increase a HSPG expression. In some aspects, a composition can be administered in a single daily dose, or a total daily dosage can be administered in divided doses of two, three, or four times daily. Typically and most conveniently, a composition is administered at least once daily, but in certain situations less frequent, e.g., twice weekly or weekly, administration can be effective. For greatest benefit, administration should continue for a prolonged period, for example at least about 3 months, or at least about 6 months, or at least about 1 year, or at least about 2 years, or at least about 3 years. In one aspect,
administration continues from a time of initiation for substantially the remainder of the mammal's life.

The selection and/or amounts of individual compounds can, if desired vary over the period of administration. In one aspect, a single composition of this invention is administered to a mammal for the entire period of administration. In other aspects, different compositions comprising at least one compound are administered to the mammal at different times.

The dosages of compounds can be adjusted on a per body weight basis and may thus be suitable for any subject regardless of the subject's size.

In one aspect of this invention, daily oral dose comprises a total compound amount of at least about 0.0001 mg per kg body weight, illustratively about 0.0001 mg to about 1000 mg, about 0.001 mg to about 100 mg, about 0.01 mg to about 10 mg, about 0.1 mg to about 5 mg, or about 1 to about 3 mg per kg body weight.

In another aspect, a daily intravenous injection comprises a total compound amount of at least about 0.0001 mg per kg body weight, illustratively about 0.0001 mg to about 0.5 mg, about 0.001 mg to about 0.25, or about 0.01 to about 0.03 mg per kg body weight.

Illustratively, a tablet for oral administration can be manufactured to comprise a total compound amount of about 0.001 mg, about 0.1 mg, about 0.2 mg, about 0.5 mg, about 1 mg, about 2 mg, about 5 mg, about 10 mg, about 15 mg, about 100 mg, about 150 mg, about 200 mg, about 250 mg, about 300 mg, about 350 mg, about 400 mg, about 450 mg, about 500 mg, about 550 mg, about 600 mg, about 650 mg, about 700 mg, about 800 mg, about 900 mg, or about 1000 mg.

In one aspect, a composition comprises an active ingredient content of at least about 0.01% by weight of the composition, illustratively about 0.01% to about 99%, about 0.05% to about 90%, about 0.1% to about 80%, about 0.5% to about 50% by weight of the composition. The amount of active ingredient that can be combined with other materials to produce a single dosage form varies depending upon the subject treated and the particular mode of administration.

An effective amount of the drug is ordinarily supplied at a dosage level of from about 0.1 mg/kg to about 20 mg/kg of body weight per day. In one aspect, the range is from about 0.2 mg/kg to about 10 mg/kg of body weight per day. In another aspect, the range is from about 0.5 mg/kg to about 10 mg/kg of body weight per day. The compounds can be administered on a regimen of about 1 to about 10 times per day.

Co-administration or sequential administration of the compounds of the present invention and other therapeutic agents can be employed, such as chemotherapeutic agents, immunosuppressive agents, cytokines, cytotoxic agents, nucleolytic compounds, radioactive isotopes, receptors, and pro-drug activating enzymes, which can be naturally occurring or produced by recombinant methods. The combined administration includes co-administration, using separate formulations or a single pharmaceutical formulation, and consecutive administration in either order, wherein preferably there is a time period while both (or all) active therapeutic agents simultaneously exert their biological activities.

It is to be understood that this invention is not limited to the particular methodology, syntheses, formulations, protocols, cell lines, constructs, and reagents described herein and as such can vary. It is also to be understood that the terminology used herein is for the purpose of describing particular aspects only, and is not intended to limit the scope of the present invention.

All publications, patents, and other references mentioned herein are provided for the purpose of describing and disclosing, for example, the constructs and methodologies that are described in these references, which might be used in connection with the presently described invention.

### DEFINITIONS AND TERMINOLOGY

The groups defined for various symbols used in the formulas of this disclosure, as well as the optional substituents defined on those groups, can be defined as follows. Unless otherwise specified, any recitation of the number of carbon atoms in a particular group is intended to refer to the unsubstituted "base" group, therefore, any substituent recited on a base group is described by its own definition, including its own limitation of the number of carbon atoms. Unless otherwise specified, all structural isomers of a given structure, for example, all enantiomers, diasteriomers, and regioisomers, are included within this definition.

The terms "halogen" or "halo" includes fluorine, chlorine, bromine, or iodine.

The term "alkyl" group is used to refer to both linear and branched alkyl groups. Exemplary alkyl groups include, but are not limited to, methyl, ethyl, propyl, butyl, pentyl, hexyl, heptyl, octyl, nonyl, or decyl, and the like. Unless otherwise specified, an alkyl group has from 1 to 12 carbon atoms. Also unless otherwise specified, all structural isomers of a given structure, for example, all enantiomers and all diasteriomers, are included within this definition. For example, unless otherwise specified, the term propyl is meant to include *n*-propyl and *iso*-propyl, while the term butyl is meant to include *n*-butyl, *iso*-butyl, *t*-butyl, *sec*-butyl, and so forth.

The term "aryl" refers to an optionally substituted monocylic or polycyclic aromatic ring system of 6 to 14 carbon atoms. Exemplary groups include phenyl, naphthyl, 1,2,3,4-tetrahydronaphthalene, indane, fluorene, and the like. Unless otherwise specified, an aryl group typically has from 6 to 14 carbon atoms.

The term "haloalkyl" refers to a group containing at least one halogen and an alkyl portion as define above, that is, a haloalkyl is a substituted alkyl group that is substituted with one or more halogens. Unless otherwise specified, all structural isomers of a given structure, for example, all enantiomers and all diasteriomers, are included within this definition. Exemplary haloalkyl groups include fluoromethyl, chloromethyl, fluoroethyl, chloroethyl, trifluoromethyl, and the like. Unless otherwise specified, a haloalkyl group has from 1 to 12 carbon atoms.

A "cycloalkyl" group refers to a cyclic alkyl group which can be mono or polycyclic. Exemplary cycloalkyl groups include cyclopropyl, cyclobutyl, cyclopentyl, cyclohexyl, cycloheptyl, cyclooctyl, cyclononyl, and cyclodecyl. Unless otherwise specified, a cycloalkyl group has from 3 to 15 carbon atoms.

An "alkoxy" group refers to an -O(alkyl) group, where alkyl is as defined herein. Therefore, unless otherwise specified, all isomers of a given structure are included within a definition. Exemplary alkyl groups include methoxy, ethoxy, n-propoxy, *iso*-propoxy, *n*-butoxy, *iso*-butoxy, *t*-butoxy, and the like. Unless otherwise specified, an alkoxy group has from 1 to 12 carbon atoms. Unless otherwise specified, all structural isomers of a given structure, for example, all enantiomers and all diasteriomers, are included within this definition. For example, unless otherwise specified, the term propoxy is meant to include *n*-propoxy and *iso-*propoxy.

"Heterocyclyl" is a non-aromatic, saturated or unsaturated, monocyclic or polycyclic ring system of 3 to 10 member having at least one heteroatom or heterogroup selected from -O-, >N-, -S-, >NR, >SO₂ >CO, and the like, wherein R is hydrogen or a substituted or an unstubstituted alkyl, aryl, or acyl, as defined herein. Exemplary heterocyclyl groups include aziridinyl, imidazolidinyl, 2,5-dihydro-[1,2,4]oxadiazolenyl, oxazolidinyl, isooxazolidinyl, pyrrolidinyl, piperdinyl, piperazinyl, morpholinyl, thiomorpholinyl, thiazolidinyl, 1,3-dioxolanyl, 1,4-dioxanyl, 2,5-dihydro-1H-imidazolyl, and the like. Unless otherwise specified, a heterocyclyl group typically has from 2 to 10 carbon atoms. A heterocyclyl group can be bonded through a heteroatom that is formally deprotonated or a heterocyclyl group can be bonded through a carbon atom of the heterocyclyl group.

A "cyclic" moiety, including a monocyclic moiety or a bicyclic moiety, unless otherwise specified, is intended to be inclusive of all the cyclic groups disclosed herein, for example, a heteroaryl group, a heterocyclyl group, a heterocycloalkyl group, and/or a heteroaryloxy group.

A "(cycloalkyl)alkyl" group, refers to an alkyl group that is substituted with a cycloalkyl substituent, wherein alkyl and cycloalkyl are defined herein. Thus, the cycloalkyl group portion can be a mono or polycyclic alkyl group. Unless otherwise specifed, a (cycloalkyl)alkyl group can have up to 20 carbon atoms, regardless of how the carbon atoms are distributed between the alkyl portion and the cycloalkyl portion of the group, and including all possible sterochemistries and all regiochemistries. For example, in one aspect, a cycloalkyl-substitued alkyl can comprise a (C₃-C10) cycloalkyl bonded to a C₁-C₁₀ alkyl group, wherein the cycloalkyl portion can be mono or polycyclic. Exemplary (cycloalkyl)alkyl groups include, but are not limited to, cyclopropylmethyl, cyclopropylethyl, cyclopropylpropyl, cyclobutylmethyl, cyclobutylethyl, cyclobutylpropyl, cyclopentylmethyl, cyclopentylethyl, cyclopentylpropyl, cyclohexylmethyl, cyclohexylethyl, cyclohexylpropyl, cycloheptylmethyl, cycloheptylethyl, cyclooctylmethyl, cyclooctylethyl, cyclooctylpropyl, and the like.

Further, the meaning of certain additional terms and phrases employed in the specification, can be defined as follows.

As used herein, the term "compound" includes both the singular and the plural, and includes any single entity or combined entities that have at least the affect disclosed herein and combinations, fragments, analogs or derivatives of such entities.

As used herein, the term "substance" refers broadly to any material of a particular kind or constitution. Examples of a "substance" can include, without limitation, a chemical element, a molecule, a compound, a mixture, a composition, an emulsion, a chemotherapeutic agent, a pharmacological agent, a hormone, an antibody, a growth factor, a cellular factor, a nucleic acid, a protein, a peptide, a peptidomimetic, a nucleotide, a carbohydrate, and combinations, fragments, analogs or derivatives of such entities.

The terms "treatment", "treating", "treat", and the like are used herein to refer generally to any process, application, therapy, etc., wherein a mammal is subject to medical attention with the object of obtaining a desired pharmacological and/or physiological effect for improving the mammal's condition or disease, directly or indirectly. The effect can be therapeutic in terms of a partial or complete stabilization or cure for a disease and/or adverse effect attributable to the disease. The effect also can include, for example, inhibition of disease symptom (*i.e.,* arresting its development) or relieving disease symptom *(i.e.,* causing regression of the disease or symptom).

A used herein, the term "therapeutically-effective amount" refers to that amount of at least one compound as disclosed herein, or their pharmaceutically-acceptable salts thereof, that is sufficient to bring about the biological or medical effect that is being sought in a mammal, system, tissue, or cell.

The term "preventing", "prevent", "prevention", and the like are used herein to refer generally to any process, application, therapy, etc., wherein a mammal is subject to medical attention with the object of obtaining a desired pharmacological and/or physiological effect for preventing onset of clinically evident condition or disease or preventing onset of a preclinically evident stage of a condition or disease. The effect can be prophylactic in terms of completely or partially preventing or reducing the risk of occurance of a condition or disease or symptom thereof.

A used herein, the term "prophylactically-effective amount" refers to that amount of a drug or pharmaceutical agent that will prevent or reduce the risk of occurrence of the biological or medical effect that is sought to be prevented in the cell, tissue, system, or mammal.

As used herein, the term "activation" refers to any alteration of a signaling pathway or biological response including, for example, increases above basal levels, restoration to basal levels from an inhibited state, and stimulation of the pathway above basal levels.

Generally a skill in the art is known that valance must be conserved for all the stable molecules. Therefore, the necessary implication that hydrogen atoms are necessary and available to complete the valance in all structures including Formula I unless expressly indicated otherwise.

Publications and patents mentioned herein are disclosed for the purpose of describing, for example, the constructs and methodologies that are provided in the publications and patents, which might be used in connection with the present invention. Nothing herein is to be construed as an admission that the inventors are not entitled to antedate such publications, patents, or other disclosure by virtue of prior invention.

To the extent that any definition or usage provided by any document incorporated herein by reference conflicts with the definition or usage provided herein, the definition or usage provided herein controls.

When Applicants disclose or claim a range of any type, for example a range of temperatures, a range of numbers of atoms, a molar ratio, or the like, Applicants' intent is to disclose or claim individually each possible number that such a range could reasonably encompass, as well as any sub-ranges and combinations of sub-ranges encompassed therein. For example, when the Applicants disclose or claim a chemical moiety having a certain number of carbon atoms, Applicants' intent is to disclose or claim individually every possible number that such a range could encompass, consistent with the disclosure herein. For example, the disclosure that R is selected independently from an alkyl group having up to 12 carbon atoms, or in alternative language a C₁ to C₁₂ alkyl group, as used herein, refers to an R group that can be selected independently from a hydrocarbyl group having 1, 2, 3, 4, 5, 6, 7, 8, 9, 10, 11, or 12 carbon atoms, as well as any range between these two numbers for example a C₃ to C₈ alkyl group, and also including any combination of ranges between these two numbers for example a C₃ to C₅ and C₇ to C₁₀ hydrocarbyl group. In another example, by the disclosure that the molar ratio typically spans the range from about 0.1 to about 1.1, Applicants intend to recite that the molar ratio can be selected from about 0.1:1, about 0.2:1, about 0.3:1,
about 0.4:1, about 0.5:1, about 0.6:1, about 0.7:1, about 0.8:1, about 0.9:1, about 1.0:1, or about 1.1:1.

Applicants reserve the right to proviso out or exclude any individual members of any such group, including any sub-ranges or combinations of sub-ranges within the group, that may be claimed according to a range or in any similar manner, if for any reason Applicants choose to claim less than the full measure of the disclosure, for example, to account for a reference that Applicants may be unaware of at the time of the filing of the application. Further, Applicants reserve the right to proviso out or exclude any individual substituents, compounds, ligands, structures, or groups thereof, or any members of a claimed group, if for any reason Applicants choose to claim less than the full measure of the disclosure, for example, to account for a reference that Applicants may be unaware of at the time of the filing of the application.

The following references disclose certain heterocyclic compounds.

**Table-1. References disclosing heterocyclic compounds.**

| **Publication or Patent No**. | **Title** |
|---|---|
| WO2005097806 | Preparation of heterocyclic piperidine derivatives as inhibitor of cholesterol ester transfer protein |
| WO2005095395 | Preparation of 1,2,3,4-tetrahydro-1,5-naphthyridin-4-amines as cholesteryl ester transfer protein inhibitors |
| WO2005095409 | Preparation of 1,2,3,4-tetrahydroquinolin-4-amines as cholesteryl ester transfer protein inhibitors |
| WO2005030185 | Method using cholesteryl ester transfer protein (CETP) inhibitors for inhibiting remnant lipoprotein production |
| US2004039018 | Use of cholesteryl ester transfer protein (CETP) inhibitors and antihypertensive agents and optional HMG-CoA |
| | reductase inhibitors for the treatment of cardiovascular conditions |
| WO2000017165 | Preparation of 4-amino-substituted 2-substituted 1,2,3,4-tetrahydroquinolines as CEPT inhibitors |
| US2004053842 | Therapeutic use and pharmaceutical compositions of cholesterol ester transfer protein (CETP) inhibitors and optional HMG-CoA reductase inhibitors and/or antihypertensive agents |
| WO2003000295 | Self-emulsifying formulations of cholesteryl ester transfer protein inhibitors and surfactants |
| WO2002011710 | Pharmaceutical compositions of cholesteryl ester transfer protein inhibitors |
| US2003198674 | Controlled release dosage forms containing cholesteryl ester transfer protein inhibitor |
| WO2003063832 | Pharmaceutical compositions comprising a solid amorphous dispersion of CETP inhibitors |
| US2003104063 | Pharmaceutical compositions containing a solid dispersion of a poorly-soluble drug in a matrix and a solubility-enhancing polymer |
| US2003054037 | Pharmaceutical compositions of adsorbates of amorphous drug |
| US2003170309 | Pharmaceutical compositions containing polymer and drug assemblies |
| US2003072801 | Pharmaceutical compositions comprising concentration-enhancing polymers |
| US2004185102 | Dosage forms comprising a CETP inhibitor and an HMG-CoA reductase inhibitor |
| WO2005000811 | Preparation of 3-aminopyrrolidines as inhibitors of monoamine uptake |
| US2005049239 | Preparation of aroylpiperidines and related compounds as selective inhibitors of the type 2 glycine transporter (GlyT2) |
| WO2005021525 | Preparation of aroylpiperidines and related compounds as selective inhibitors of the type 2 glycine transporter (GlyT2) |
| WO2004078169 | Use of EP2 selective receptor agonists in medical treatment of pulmonary hypertension and other conditions |
| WO2004078128 | Preparation of pyridine-containing diaryl ureas useful in the treatment of cancer and other disorders |
| WO2004073709 | Preparation of tertiary amino compounds as antimicrobial |
| | agents |
| WO2003087088 | Preparation of pyridone and pyrimidone compounds as inhibitors of the enzyme Lp-PLA2 |
| WO2003030909 | Preparation of 2- and 4-aminopyrimidines N-substituted by a bicyclic ring for use as kinase inhibitors in the treatment of cancer |
| WO2002090349 | Pyridylmethylanthranilamide N-oxides as inhibitors of VEGFR II kinase |
| WO2002070462 | Preparation of aminodicarboxylic acids for the treatment of cardiovascular diseases |
| WO2002068417 | Heteropolycyclic compounds, particularly pyridyl- and phenyl-substituted 1,2,4-oxadiazoles and analogs, and their use as metabotropic glutamate receptor antagonists for inhibiting neuronal damage |
| WO2002042273 | Preparation of aromatic acid derivatives useful as serine protease inhibitors |
| WO2002022584 | Preparation of substituted heterocyclic aryl-alkyl-aryl compounds as thrombin inhibitors |
| WO2001085671 | Preparation of (heterocyclyl)anthranylamides as inhibitors of vascular endothelial growth factor receptors |
| WO2001060458 | Piperazine inhibitors of prenyl-protein transferase for antitumor use |
| WO2001060369 | Piperazine inhibitors of prenyl-protein transferase for antitumor therapy |
| WO2001056560 | Preparation of substituted amino acids as neutral sphingomyelinase inhibitors |
| WO2001022954 | Indolyl-3-glyoxylic acid derivatives comprising therapeutically valuable properties |
| WO2001000623 | Method of producing nitroguanidine and nitroenamine derivatives |
| JP2001163779 | Prostaglandin receptor agonists and prodrugs for treatment of male erectile disorder |
| EP1108426 | Prostaglandin receptor agonists and prodrugs for treatment of male erectile disorder |
| DE19962300 | Preparation of N-(pyridin-4-yl) [1-(4-aminobenzyl)indol-3-yl]glyoxylamides as antitumor agents |
| US2001014690 | Preparation ofN-(pyridin-4-yl) [1-(4-aminobenzyl)indol-3-yl]glyoxylamides as antitumor agents |
| US6432987 | Preparation ofN-(pyridin-4-yl) [1-(4-aminobenzyl)indol-3-yl]glyoxylamides as antitumor agents |
| CA2395259 | Preparation of N-(pyridin-4-yl) [1-(4-aminobenzyl)indol-3-yl]glyoxylamides as antitumor agents |
| WO2001047913 | Preparation of N-(pyridin-4-yl) [1-(4-aminobenzyl)indol-3-yl]glyoxylamides as antitumor agents |
| DE19930075 | Preparation of aminoarylsulfonamides as antivirals |
| WO2001002350 | Preparation of aminoarylsulfonamides as antivirals |
| WO2000050398 | Preparation of phenyl and pyridinyl derivatives as NK-1 receptor antagonists |
| US6121271 | Preparation of naphtho[2,3-b]heteroar-4-yl derivatives for treating metabolic disorders related to insulin resistance or hyperglycemia |
| DE19845202 | Hair growth atimulant |
| WO2000019969 | Hair growth atimulant |
| WO9951224 | Preparation of indolylglyoxylamides as antitumor agents |
| WO9919300 | Preparation of prostaglandin agonists and their use to treat bone disorders |
| US6008362 | Elevation of HDL cholesterol by 2-(4-chloro-1-aryl-butylidene)- hydrazinecarbothioamides |
| US5977170 | Preparation of 4- [(aminothioxomethyl)hydrazono]-4-arylbutyl carbamates as elevators of HDL cholesterol |
| JP11209366 | Preparation of chromans and pharmaceuticals for treatment of heart failure |
| WO9857928 | Elevation of HDL cholesterol by 2-(4-chloro-1-aryl-butylidene)hydrazinecarbothioamides |
| WO9857927 | Elevation of HDL cholesterol by 4-[(aminothioxomethyl)hydrazono]-4-arylbutyl carbamates |
| WO9857925 | Elevation of HDL cholesterol by 2-[(aminothioxomethyl)hydrazono]-2-arylethyl carbamates |
| WO9827053 | Preparation of sulfonamide and carboxamide derivatives as drugs |
| WO9809946 | Preparation of new, N-substituted indole-3-glyoxylamides as antiasthmatics, antiallergic agents and immunosuppressants/immunomodulators |
| DE19615262 | Phenylglycinol amides as antiatherosclerotic agents |
| EP802188 | Phenylglycinol amides as antiatherosclerotic agents |
| WO9616650 | Antibacterial or bactericide comprising 2-aminothiazole derivative and salts thereof |
| US5491152 | ACAT-inhibiting derivatives of cyclic ethers and sulfides |
| | for the treatment of atherosclerosis |
| JP08092225 | Preparation of O-(1,2,4-triazol-5-yl)glycolamide derivatives as herbicides |
| JP08092224 | Preparation of 3,5-diphenyl-1,2,4-triazole derivatives as insecticides and acaricides |
| WO9505363 | Amidine derivatives with nitric oxide synthetase activities |
| US5422355 | Antidepressant (arylalkyl)amines as GABA autoreceptor agonists |
| DD294706 | Antidepressant (arylalkyl)amines as GABA autoreceptor agonists |
| US5086073 | Antidepressant (arylalkyl)amines as GABA autoreceptor agonists |
| US5260331 | Antidepressant (arylalkyl)amines as GABA autoreceptor agonists |
| JP07285962 | Preparation of (pyridyloxy)pyrazole derivatives as herbicides |
| WO9413636 | Preparation of N-carbamoyl-2-[(aminoalkyl)carbamoylalkoxy]azetidinones and analogs as elastase inhibitors |
| US5348953 | Preparation of azetidinones as antiinflammatory and antidegenerative agents |
| CN1068815 | Preparation of azetidinones as antiinflammatory and antidegenerative agent |
| ZA9204659 | Preparation of azetidinones as antiinflammatory and antidegenerative agent |
| AU9218582 | Preparation of azetidinones as antiinflammatory and antidegenerative agent |
| AU660026 | Preparation of azetidinones as antiinflammatory and antidegenerative agent |
| WO9413636 | Preparation of azetidinones as antiinflammatory and antidegenerative agent |
| EP604798 | N-arylhydrazine derivatives as insecticides and acaricides |
| EP585500 | Diaryl piperazineacetamides as antimuscarinic agents |
| WO9405648 | Diaryl piperazineacetamides as antimuscarinic agents |
| WO9310099 | Pyrazoleglycolamide derivatives as agrochemicals |
| EP553016 | Preparation of naphthalene amides and sulfonamides, their pharmaceutical formulations, and their affinity for serotoninergic receptors |
| JP01104052 | Preparation of pyridine derivatives as leukotriene |
| | antagonists and vasodilators |

Applicants reserve the right to proviso out or to restrict from any claim currently presented, or from any claim that may be presented in this or any further application based upon this disclosure, including claims drawn any genus or subgenus disclosed herein, any compound or group of compounds disclosed in any reference, including any reference provided herein.

The following acronyms, abbreviations, terms and definitions have been used throughout this disclosure. The following acronyms, abbreviations, terms and definitions have been used throughout the experimental section. **Acronyms and abbreviations:** THF (tetrahydrofuran), DMF (*N*,*N-*dimethylformamide), IPA (isopropanol), TBAB (tetra-*n*-butylammonium bromide), TBTU [O-(Benzotriazol-1-yl)-N,N,N',N'-tetramethyluronium tetrafluoroborate] DIPEA (diisopropylethylamine) DCM (dichloromethane), DCE (dichloroethane), EDCI [1-(3-dimethylaminopropyl)-3-ethylcarbodiimide hydrochloride], DIBAL (diisobutyl aluminum hydride), LAH (lithium aluminum hydride), POCl₃ (phosphorousoxychloride) g or gm (grams), L (liter), mL (milliliters), mp (melting point), rt or RT (room temperature 20-40 °C), overnight (8-12 hours) aq (aqueous), min (minute), h or hr (hour), min (minutes), atm (atmosphere), conc. (concentrated), MS or mass spec (mass spectroscopy/spectrometry), NMR (nuclear magnetic resonance), IR (infrared spectroscopy), RB (round bottom), RBF (round bottom flask). In addition to these abbreviations, standard chemical abbreviations for chemical moieties, such as Me for methyl, Et for ethyl, and the like, are used throughout. **NMR abbreviations:** br (broad), apt (apparent), s (singlet), d (doublet), t (triplet), q (quartet), dq (doublet of quartets), dd (doublet of doublets), dt (doublet of triplets), m (multiplet).

### General Synthetic Procedures.

Room temperature is defined as an ambient temperature range, typically from about 20°C to about 35°C. An ice bath (crushed ice and water) temperature is defined as a range, typically from about -5°C to about 0 °C. Temperature at reflux is defined as ±15°C of the boiling point of the primary reaction solvent. Overnight is defined as a time range of from about 8 to about 16 hours. Vacuum filtration (water aspirator) is defined as occurring over a range of pressures, typically from about 5 mm Hg to about 15 mm Hg. Dried under vacuum is defined as using a high vacuum pump at a range of pressures, typically from about 0.1 mm Hg to about 5 mm Hg. Neutralization is defined as a typical acid-based neutralization method and measured to a pH range of from about pH 6 to about pH 8, using pH-indicating paper. Brine is defined as a saturated aqueous sodium chloride. Nitrogen atmosphere is defined as positive static pressure of nitrogen gas passed through a Drierite™ column with an oil bubbler system. Concentrated ammonium hydroxide is defined as an approximately 15 M solution. Melting points were measured against a mercury thermometer and are not corrected.

All eluents for column or thin layer chromatography were prepared and reported as volume:volume (v:v) solutions. The solvents, reagents, and the quantities of solvents and/or reagents used for reaction work-up or product isolation can be those that typically would be used by one of ordinary skill in organic chemical synthesis, as would be determined for the specific reaction or product to be isolated. For example: 1) crushed ice quantity typically ranged from about 10 g to about 1000 g depending on reaction scale; 2) silica gel quantity used in column chromatography depended on material quantity, complexity of mixture, and size of chromatography column employed and typically ranged from about 5 g to about 1000 g; 3) extraction solvent volume typically ranged from about 10 mL to about 500 mL, depending upon the reaction size; 4) washes employed in compound isolation ranged from about 10 mL to about 100 mL of solvent or aqueous reagent, depending on scale of reaction; and 5) drying reagents (potassium carbonate, sodium carbonate or magnesium sulfate) ranged from about 5 g to about 100 g depending on the amount of solvent to be dried and its water content.

### Spectroscopic and other Instrumental Procedures

NMR. The ¹H spectra described herein were obtained using Varian Gemini 200 MHz spectrometers. Spectrometer field strength and NMR solvent used for a particular sample are indicated in the examples, or on any NMR spectra that are shown as Figures. Typically, ¹H NMR chemical shifts are reported as δ values in parts per million (ppm) downfield from tetramethylsilane (TMS) (δ = 0 ppm) as an internal standard. Solid or liquid samples were dissolved in an appropriate NMR solvent (typically CDCl₃ or DMSO-d₆), placed in a NMR sample tube, and data were collected according to the spectrometer instructional manuals. Most samples were analyzed in Variable Temperature mode, typically at about 55 °C, though some data for some samples were collected with the probe at ambient probe temperature. NMR data were processed using the software provided by Varian, VNMR 6.1 G version.

The present invention is further illustrated by the following examples, which are not to be construed in any way as imposing limitations upon the scope of this disclosure, but rather are intended to be illustrative only. On the contrary, it is to be clearly understood that resort may be had to various other embodiments, modifications, and equivalents thereof which, after reading the description herein, may suggest themselves to one of ordinary skill in the art without departing from the spirit of the present invention. Thus, the skilled artisan will appreciate how the experiments and Examples may be further implemented as disclosed by variously altering the following examples, substituents, reagents, or conditions. In the specification, including the following examples, in the disclosure of any measurements, including temperatures, pressures, times, weights, percents, concentrations, ranges, chemical shifts, frequencies, molar ratios, and the like, it is to be understood that such measurements are respectively, "about."

### Example 1

### Synthesis of (3-{[3,5-bistrifluoromethyl-benzyl)-(2-cyclopropylmethyl-2H-tetrazole -5-yl)-amino]-methyl-}-8-methyl-quinoline-2-yl)-bis-cyclopropylmethyl-amine

### Step (i): Synthesis of 2-chloro-8-methyl-quinoline-3-carbaldehyde

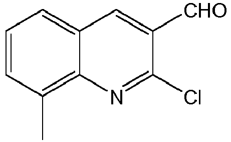

DMF (1.22 g, 16.7 mmol) was taken in a flask equipped with a drying tube and POCl₃ (7.32 g, 46.7 mmol) was added dropwise with stirring at 0° C. To this solution, *N-*o-Tolyl acetamide (1.00 g, 6.7 mmol) was added and the solution was refluxed for 6 h at 90°C. The excess POCl₃ was distilled off, water was added to the residue and this was stirred at room temperature for 10 min. The solid was filtered and dried under vacuum..This crude compound was purified over silica gel (100-200 mesh) using 6% ethyl acetate and petroleum ether to give the product as a yellowish solid (yield: 78%). ¹H NMR (CDCl₃, 200 MHz): δ 10.5 (s, 1H), 8.71 (s, 1H), 7.83- 7.79 (m, 1H), 7.74-7.70 (m, 1H), 7.56-7.49 (m, 1H), 2.79 (s, 3H); *m*/*z* (EI-MS): 206 (M⁺, 100%).

### Step (ii): Synthesis of 2-(bis(cyclopropylmethyl)amino)-8-methylquinoline-3-carbaldehyde:

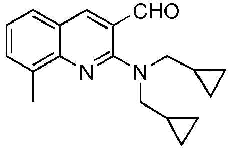

2-Chloro-8-methyl-quinoline-3-carbaldehyde (.115 g, 0.559 mmol), and potassium carbonate (0.231 g, 1.67 mmol) were put in a 25 mL two necked RB flask. To this, 3 mL of DMF was added followed by dropwise addition of bis-cyclopropylmethyl amine (0.083 g, 0.67 mmol). The reaction mixture was refluxed for 2 h and was cooled to RT. It was then poured on crushed ice (10 mL) and extracted with EtOAc (3 × 10 mL). The organic layer was washed with brine and dried over sodium sulphate. The solvent was evaporated under vacuum to give a yellow colored oil (0.081 g, 50%).
¹H NMR (CDCl₃, 400 MHz): δ 10.5 (s, 1H), 8.71 (s, 1H), 7.83- 7.79 (m, 1H), 7.74-7.70 (m, 1H), 7.56-7.49 (m, 1H), 3.55-3.47 (m, 4H), 2.79 (s, 3H), 1.73-1.72 (m, 2H), 1.70-1.46 (m, 4H), 1.20-1.11 (m, 4H); *m*/*z* (ES-MS): 295 (M⁺+1, 100%); IR (neat, cm⁻¹): 3385, 2948, 1691.

### Step (iii): Synthesis of 3-((3,5-bis(trifluoromethyl)benzylamino)methyl)-N,N-bis(cyclopropylmethyl)-8-methylquinolin-2-amine

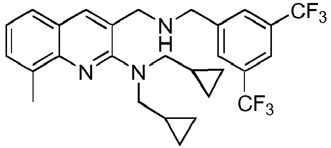

2-(Bis(cyclopropylmethyl)amino)-8-methylquinoline-3-carbaldehyde (0.081 g, 0.39 mmol), 3,5-bis-trifluoromethylbenzylamine (0.096 g, 0.39 mmol) and acetic acid (0.047 g, 0.78 mmol) were put in a 25 mL RB flask. To this, 2 mL of methanol was added and stirred at RT for 15 min. Sodium cyanoborohydride (0.075 g, 0.77 mmol) was added portionwise and stirring was continued at RT for another 1 h. Methanol was removed from the reaction mixture under vacuum, water was added to this crude and was extracted with ethyl acetate (3 x 50 mL). The organic layer was washed with saturated NaHCO₃ solution, brine and dried over sodium sulphate. The solvent was evaporated and the crude residue was purified by column chromatography over silica gel (100-200 mesh) eluting with 4% ethyl acetate in petroleum ether to give the title amine (0.142 g, yield: 99%).
¹H NMR (CDCl₃, 400 MHz): δ 7.89-7.86 (m, 1H), 7.80 (m, 1H), 7.75-7.74 (m, 1H), 7.60-7.40 (m, 3H), 7.30-7.26 (m,1H), 4.12 (s, 2H), 3.88 (s, 2H), 3.24-3.22 (m, 4H), 2.72 (s, 3H), 0.99-0.92 (m, 2H), 0.44-0.35 (m, 4H), 0.11-0.05 (m, 4H); *m*/*z* (EI-MS): 522 (M⁺+1, 100%); IR (neat, cm⁻¹): 3357, 2929, 2851.

### Step (iv): Synthesis of N-(3,5-bis(trifluoromethyl)benzyl)-N-((2-(bis(cyclopropylmethyl)amino)-8-methylquinolin-3-yl)methyl)cyanamide

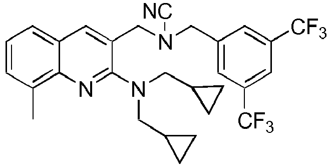

To a solution of 3-((3,5-bis(trifluoromethyl)benzylamino)methyl)-N,N-bis(cyclopropylmethyl)-8-methylquinolin-2-amine (0.176 g , 0.33 mmol ), obtained in step (iii) , in MeOH (4 mL) under N₂ atmosphere was added sodium bicarbonate (0.056 g, 0.67 mmol) followed by the addition of cyanogen bromide (0.063 g, 0.60 mmol). The reaction mixture was stirred at RT for 2 h. The solvent was removed under vacuum to give the crude residue which was dissolved in water, extracted with ethyl acetate and dried over sodium sulphate. The solvent was evaporated and concentrated in *vacuo* to afford N-(3,5-bis(trifluoromethyl)benzyl)-N-((2-(bis(cyclopropylmethyl)amino)-8-methylquinolin-3-yl)methyl)cyanamide (0.118 g, 64%).
¹H NMR (CDCl₃, 400 MHz): δ 8.07 (s, 1H), 7.82 (s, 1H), 7.70 (s, 2H), 7.56-7.55 (m, 1H), 7.50-7.49 (m, 1H), 4.49 (s, 2H), 4.23 (s, 2H), 3.17 -3.15 (m, 4H), 2.71 (s, 3H), 0.097-0.085 (m, 2H), 0.405-0.401 (m, 4H), 0.385-0.381 (m, 4H); *m*/*z* (ES-MS): 547 (M⁺+1, 100%); IR(KBr ,cm⁻¹) : 2273, 1280.

### Step (v): Synthesis of (3-{[(3,5-bistrifluoromethyl-benzyl)-(2H-tetrazol-5-yl)-amino]-methyl}-8-methyl-quinolin-2-yl)-bis-cyclopropylmethyl-amine

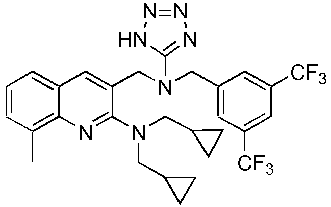

*N-*(3,5-Bis(trifluoromethyl)benzyl)-N-((2-(bis(cyclopropylmethyl)amino)-8-methylquinolin-3-yl)methyl)cyanamide (0.118 g, 0.216 mmol), sodium azide (0.70 g 1.08 mmol) and ammonium chloride (.058 g, 1.08 mmol) were put in a RB flask under N₂ atmosphere. To this reaction mixture, DMF (2 mL) was added and was refluxed for 1 h. The reaction mixture was cooled to RT and ice was added to this and extracted with ethylacetate (3x10 mL). The combined organic layer was washed with brine, dried over sodium sulphate and then concentrated under vacuum to afford of (3-[(3,5-bistrifluoromethyl-benzyl)-(2H-tetrazol-5-yl)-amino]-methyl}-8-methyl-quinolin-2-yl)-bis-cyclopropylmethyl-amine as a yellow solid (0.125 g, 99%).
¹H NMR (CDCl₃, 400 MHz): δ 7.99 (s, 1H), 7.79 -7.74 (m, 4H), 7.41-7.40 (m, 1H ), 7.33-7.31 (m, 1H), 4.99 (s, 2H), 4.80 (s, 2H), 3.68 (s, 4H), 2.16 (s, 1H) 1.56-1.06 (m, 11H); *m*/*z* (ES-MS): 578 (M⁺+1, 100%); IR (KBr , cm⁻¹) 3680 , 2922 , 1660 , 1616.

### Step (vi): Synthesis of (3-{[3,5-bis trifluoromethyl-benzyl)-(2-cyclopropylmethyl-2H-tetrazole -5-yl)-amino]-methyl-}-8-methyl-quinoline-2-yl)-bis-cyclopropylmethyl-amine

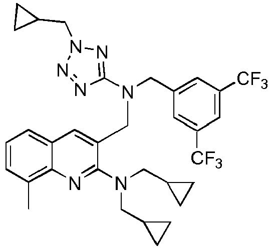

(3-{[(3,5-Bistrifluoromethyl-benzyl)-(2H-tetrazol-5-yl)-amino]-methyl}-8-methyl-quinolin-2-yl)-bis-cyclopropylmethyl-amine (0.4 g, 0.679 mmol) and potassium carbonate (0.28 g, 2.03 mmol) were taken in a 25 mL RB . To this, 4 mL of DMF was added and the reaction mixture was stirred at RT for 0.5 h. Then cycloproanelmethylbromide (0.11g, 0.814 mmol) was added dropwise. The reaction temperature was raised to 60°C and stirring was continued for another 2 h. The reaction mixture was cooled to RT, ice was added to this and was extracted with ethyl acetate (3x10 mL). The combined organic layer was washed with brine, dried over sodium sulphate and was concenterated under vacuum to afford the crude which was purified over silica gel (100-200 mesh) using 4% ethyl acetate and petroleum ether to give the desired product (0.25 g, yield: 57%).
.¹H NMR (CDCl₃, 400 MHz): δ 7.87 (s, 1H), 7.70 (s, 2H), 7.44-7.39 (m, 2H), 7.23-7.19 (m, 1H), 4.93 (s, 2H), 4.64 (s, 2H), 4.30 (d, *J =* 7.25 Hz, 2H), 3.20-3.18 (m, 4H), 2.71 (s, 3H), 1.04-0.99 (m, 2H), 0.98-0.95 (m, 1H), 0.69-0.64 (m, 2H), 0.48-0.44 (m, 2H), 0.39-0.36 (m, 4H), 0.08-0.07 (m, 4H); (ES-MS): *m*/*z* 644 (M⁺+1, 100%); IR (neat, cm⁻¹) 3384, 1618, 1580, 1278.

### Example 2

### Synthesis of (3-{[3,5-bis trifluoromethyl-benzyl)-(2-cyclopentyl -2H-tetrazole-5-yl)-amino]-methyl-}-8-methyl-quinoline-2-yl)-bis-cyclopropylmethyl-amine

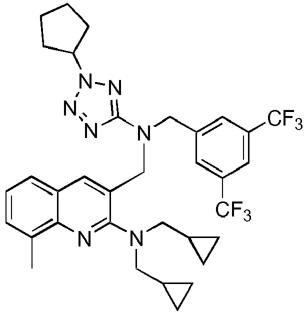

(3-{[(3,5-Bistrifluoromethyl-benzyl)-(2H-tetrazol-5-yl)-amino]-methyl}-8-methyl-quinolin-2-yl)-bis-cyclopropylmethyl-amine (0.4 g, 0.679 mmol) and potassium carbonate (0.28 g, 2.03 mmol) were taken in a 25 mL RB. To this, 4 mL of DMF was added and the reaction mixture was stirred at RT for 0.5 h.Then cyclopentylbromide (0.12g, 0.814 mmol) was added dropwise. The reaction temperature was raised to 60°C and stirring was continued for another 2 h. The reaction mixture was cooled to RT, ice was added to this and was extracted with ethyl acetate (3 x 10 mL). The combined organic layer was washed with brine, dried over sodium sulphate and was concenterated under vacuum to afford the crude which was purified over silica gel (100-200 mesh) using 4% ethylacetate and petroleum ether to afford the required product (0.3 g, yield: 67%).
¹H NMR (CDCl₃, 400 MHz): δ 7.87 (s, 1H), 7.70 (s, 3H), 7.44-7.41 (m, 2H), 7.23-7.20 (m, 1H), 5.09-5.05 (m, 1H), 4.92 (s, 2H), 4.62 (s, 2H), 3.20-3.19 (m, 4H), 2.71 (s, 3H), 2.23-2.18 (m, 4H), 1.94-1.89 (m, 2H), 1.76-1.71 (m, 2H), 1.03-0.99 (m, 2H), 0.39-0.35 (m, 4H), 0.09-0.07 (m, 4H); (ES-MS): *m*/*z* 658 (M⁺+1, 100%); IR (neat, cm⁻¹): 3383, 1578, 1278.

### Example 3

### Synthesis of (3-{[3,5-bis trifluoromethyl-benzyl)-(2-cyclohexyl -2H-tetrazole-5-yl)-amino]-methyl-}-8-methyl-quinoline-2-yl)-bis-cyclopropylmethyl-amine

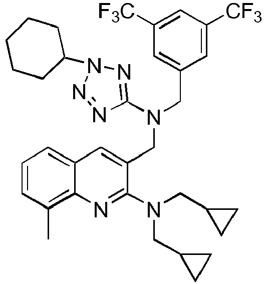

(3-{[(3,5-Bistrifluoromethyl-benzyl)-(2H-tetrazol-5-yl)-amino]-methyl}-8-methyl-quinolin-2-yl)-bis-cyclopropylmethyl-amine (0.275 g, 0.46 mmol) and potassium carbonate (0.19 g, 1.40 mmol) were taken in a 25 mL RB. To this, 4 mL of DMF was added and the reaction mixture was stirred at RT for 0.5 h. Then cyclohexylbromide (0.09 g, 0.552 mmol) was added dropwise. The reaction temperature was raised to 60°C and stirring was continued for another 2 h. The reaction was cooled to RT, ice was added to this and was extracted with ethyl acetate (3 x 10 mL). The combined organic layer was washed with brine, dried over sodium sulphate and was concentrated under vacuum to afford the crude which was purified over silica gel (100-200 mesh) using 4% ethyl acetate and petroleum ether to get the required product (0.125 g, yield: 39%).
¹H NMR (CDCl₃, 400 MHz): δ 7.87 (s, 1H), 7.70 (s, 3H), 7.44-7.40 (m, 2H), 7.23-7.19 (m, 1H), 4.92 (s, 2H), 4.62 (s, 2H), 4.56-4.50 (m, 1H), 3.20-3.19 (m, 4H) 2.71 (s, 3H), 2.24-2.20 (m, 2H), 1.98-1.66 (m, 4H), 1.76-1.71 (m, 2H), 1.53-1.24 (m, 2H), 1.05-0.98 (m, 2H), 0.39-0.35 (m, 4H), 0.09-0.06 (m, 4H); (ES-MS): *m*/*z* 672 (M⁺+1, 100%); IR (neat, cm⁻¹): 3417, 1576, 1278.

### Example 4

### Synthesis of (3-{[3,5-bis trifluoromethyl-benzyl )-(2-isopropyl -2H-tetrazole -5-yl)-amino]-methyl-}-8-methyl-quinoline-2-yl)- bis-cyclopropylmethyl-amine

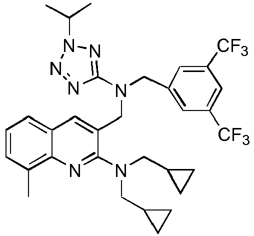

This compound (0.12 g, yield: 44%) was prepared following the same procedure as in Example 1 by using isopropylbromide instead of cyclopropanemethyl bromide.
¹H NMR (CDCl₃, 400 MHz): δ 7.87 (s, 1H), 7.71 (s, 3H), 7.43-7.41 (m, 2H), 7.22-7.20 (m, 1H), 4.93 (s, 2H), 4.92-4.88 (m, 1H), 4.63 (s, 2H), 3.20 (d, *J* = 6.4 Hz, 4H), 2.72 (s, 3H), 1.62 (d, *J =* 6.7 Hz, 6H), 1.04-1.00 (m, 2H), 0.40-0.38 (m, 4H), 0.10-0.07 (m, 4H).
(ES-MS): *m*/*z* 632 (M⁺+1, 100%); IR (neat, cm⁻¹): 3079, 2925, 1578, 1278, 1135.

### Example 5

### Synthesis of (2- {5-[[2-(bis-cyclopropylmethyl-amino)-8-methyl-quinolin-3-ylmethyl]-(3,5-bis-trifluoromethyl-benzyl)-amino]-tetrazol-2-yl}-ethyl)-carbamic acid tert-butyl ester

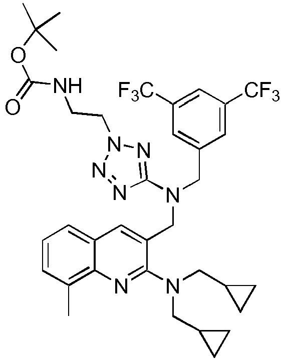

This compound (0.35 g, yield: 95%) was prepared following the same procedure as in Example 1 by using (2-bromoethyl)-carbamic acid-*tert*-butyl ester instead of cyclopropanemethyl bromide.
¹H NMR (CDCl₃, 400 MHz): δ 7.84 (s, 1H), 7.70 (s, 1H), 7.68 (s, 2H), 7.44-7.42 (m, 2H), 7.24-7.20 (m, 1H), 4.92 (s, 2H), 4.8 (br s, 1H), 4.66 (s, 2H), 4.59-4.56 (m, 2H), 3.73-3.69 (m, 2H), 3.19-3.12 (m, 4H), 2.71 (s, 3H), 1.53 (s, 9H), 0.89-0.85 (m, 2H), 0.39-0.35 (m, 4H), 0.09-0.05 (m, 4H); (ES-MS): *m*/*z* 733 (M⁺+1, 100%); IR (neat, cm⁻¹): 3358, 2927, 1582, 1278.

### Example 6

### Synthesis of (3-{[[2-amino-ethyl)-2H-tetrazol-5-yl]-(3,5-bistrifluoromethyl-benzyl)-amino]-methyl}-8-methyl-quinolin-2-yl)-bis-cyclopropylmethyl-amine

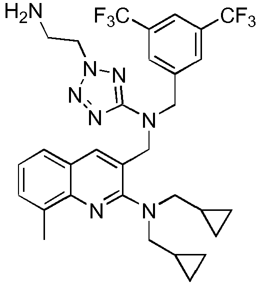

(2- {5-[[2-(bis-cyclopropylmethyl-amino)-8-methyl-quinolin-3-ylmethyl]-(3,5-bis-trifluoromethyl-benzyl)-amino]-tetrazol-2-yl}-ethyl)-carbamic acid tert-butyl ester (Example 5) (0.242 g, 0.33 mmol) was dissolved in 1 mL of DCM in a 25 mL RB. This solution was cooled to 0°C, and was added 0.4 mL of a mixture of TFA and DCM (1:1). Stirring was continued for 1 h at RT. Then water was added to reaction mixture and this was extracted with DCM (3 x 10 mL). The combined organic layer was washed with brine and was concentrated under vacuum to get pure compound (0.15 g, yield: 72%).
¹H NMR (CDCl₃, 400 MHz): δ 7.85 (s, 1H), 7.70-7.69 (m, 3H), 7.44-7.41 (m, 2H), 7.24-7.20 (m, 1H), 4.93 (s, 2H), 4.66 (s, 2H), 4,54-4.51 (m, 2H) 3.31-3.28 (m, 2H), 3.20-3.18 (m, 4H), 2.71 (s, 3H), 0.99-0.86 (m, 2H), 0.40-0.35 (m ,4H), 0.10-0.07 (m, 4H); (ES-MS): *m*/*z* 633 (M⁺+1, 100%); IR (neat, cm⁻¹): 2926, 1580, 1278.

### Example 7

### Synthesis of 1-{5-[[2-(bis-cyclopropylmethyl-amino)8-methyl-quinoline-3-ylmethyl]- (3,5-bistrifluoromethyl-benzyl)-amino]-tetrazol-2-yl}-propane-2-ol

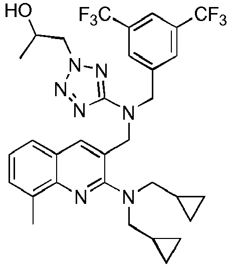

3-{[(3,5-Bistrifluoromethyl-benzyl)-(2H-tetrazol-5-yl)-amino]-methyl}-8-methyl-quinolin-2-yl)-bis-cyclopropylmethyl-amine (0.4g, 0.679 mmol), tris-*t*-butyl tin oxide (0.202 g, 0.33 mmol) were refluxed in ethanol for 4 h. Ethanol was removed from the reaction mixture under vacuum and diethyl ether was added followed by the addition of methyl propylene oxide (0.058g, 1.00 mmol). The reaction was refluxed for overnight and ether was removed under vacuum. The crude was purified by column chromatography over silica gel (100-200 mesh) using chloroform to afford the required compound (0.1 g, yield: 23%).
¹H NMR (CDCl₃, 400 MHz): δ 7.97 (s, 1H), 7.79 (s, 2H), 7.74 (s, 1H), 7.50-7.47 (m, 2H), 7.30-7.28 (m, 1H), 4.95-4.85 (m, 2H), 4,74-4.69 (m, H), 4.36-4.31 (m ,1H), 4.14-4.10 (m, 1H), 4.00-3.94 (m, 1H), 3.21-3.11 9(m, 4H), 2.71 (s, 3H), 1.19 (d, *J* = 6.4 Hz, 3H), 1.02-0.96 (m, 2H), 0.41-0.36 (m, 4H); (ES-MS): *m*/*z* 648 (M⁺+1, 100%); IR (neat, cm⁻¹) :3411, 1593, 1281.

### Example 8

### Synthesis of (3-1[(3,5-Bis-trifluoromethyl-benzyl)-(2-methyl-2H-tetrazol-5-yl)-amino]-methyl}-8-trifluoromethyl-quinolin-2-yl)-bis-eyclopropylmethyl-amine

### Step (i): Synthesis of 3-phenyl-N-(2-trifluoromethyl-phenyl)-acrylamide

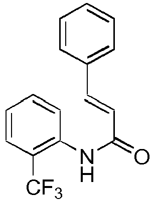

A mixture of cinnamoyl chloride (20.7g, 124.3 mmol), 2-trifluoromethylamine (20.0g, 124.2 mmol), and potassium carbonate (25.6g, 185.5 mmol) in 60 mL of acetone and 50 mL of water were stirred at 0 °C for 2 h. The reaction mixture was poured into ice and stirred for 10 min. The solid was filtered and dried under vacuum to afford the said compound (25 g, yield: 69%).
¹H NMR (CDCl₃, 400 MHz): δ 8.37 (d, *J=* 8.06, 1H), 7.79-7.75 (m,1H), 7.64-7.55 (m, 4H), 7.42-7.38 (m, 3H), 7.25-7.22 (m, 1H), 6.55-6.51 (m, 1H); (ES-MS): *m*/*z* 292 (M+1, 100%);.IR (KBr, cm⁻¹): 3227, 3012, 1632, 1318.

### Step (ii): Synthesis of 8-trifluoromethyl -1H-quinolin-2-one

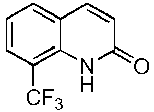

3-Phenyl-*N*-(2-trifluoromethyl-phenyl)-acrylamide (18.0 g, 61.8 mmol), which was obtained in step (i), and aluminium chloride (49.3 g, 371.1mmol) were taken in a 250 mL RB flask containing 50 mL of chlorobenzene. The reaction mixture was refluxed for 2 h and then cooled to room temperature. The mixture was poured in ice cold water. The precipitate was filtered off, washed with water and dried under vacuum to afford the title compound (9.0 g, yield: 68%).
¹H NMR (CDCl₃, 200 MHz): δ 12.01 (bs, 1H), 7.98-7.95 (m, 1H), 7.63-7.56 (m, 1H), 7.43-7.28 (m, 2H), 6.88 (s, 1H); (ES-MS): *m*/*z* 302 (M+89, 100%); IR (KBr, cm⁻¹): 3007, 2850, 1664.

### Step (iii): Synthesis of 2-chloro-8-trifluoromethyl-quinoline

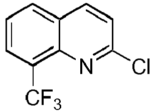

8-Trifluoromethyl -1H-quinolin-2-one(8.35g, 39.2 mmol) was taken in a 25 mL RB POCl₃ (10 mL) was added, and then refluxed for 1 h. The excess POCl₃ was distilled off and ice was added to the reaction mixture. This was stirred for 15 min, the precipitate was filtered off and then dried under vacuum to afford the title compound (3 g, yield: 33%).
(ES-MS): *m*/*z* 197(M-34, 100%); IR (KBr, cm⁻¹): 3061, 1575, 1145.

### Step (iv): Synthesis of 2-chloro-8-trifluoromethyl-quinoline-3-carbaldehyde

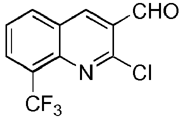

To a cold solution of butyllithium (0.498g, 1.7 mmol) in 2 mL dry THF at - 20C was added diisopropyl amine (0.192g, 1.9mmol) drop wise and reaction mixture was allowed to stir at 0 °C for 1 h. then rection mixture was again cooled to -78 °C and a solution of 2-chloro-8-trifluoromethyl-quinoline(0.4g, 1.70 mmol) in 4 mL dry THF was added drop wise continued stirring for 5-10 min then added ethyl formate (0.12 g, 1.7 mmol) drop wise, continued stirring for 0.5 h. n-Butyllithium was quenched with ammonium chloride solution and extracted with ether (3 x 10 mL). The combined organic layers were washed with brine solution, dried over sodium sulfate, concentrated under vacuum to get the crude compound. The crude residue was purified over silica gel (100-200 mesh) using 10% ethyl acetate: petroleum ether to afford the desired compound (0.15g, 34%).
¹H NMR (CDCl₃, 200 MHz): δ 10.6 (s,1H), 8.40 (d, J=7.5 Hz, 1H), 8.07 (d, J=7.9 Hz, , 1H), 7.96-7.88 (m,1H), 7.79-7.71 (m,1H); (ES-MS): *m*/*z* 260 (M+1, 60%), 258 (M-1, 100%); IR (KBr, cm⁻¹): 1710, 1580.

### Step (v): Synthesis of 2-(bis(cyclopropylmethyl)amino)-8-trifluoromethylquinoline-3-carbaldehyde

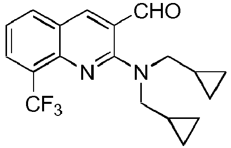

This compound (0.92 g, 69 %) was prepared by following same procedure as given in step (ii) of Example 1 by using 2-chloro-8-trifluoromethyl-quinoline-3-carbaldehyde instead of 2-Chloro-8-methyl-quinoline-3-carbaldehyde.
¹H NMR (CDCl₃, 400 MHz): δ 10.47 (s,1H), 8.12-8.10 (m,1H), 7.96-7.94 (m,1H), 7.74-7.70 (m,1H), 7.52-7.48 (m,1H), 3.48-3.46 (m,4H), 1.25-1.05 (m, 2H), 0.87-0.45 (m, 4H), 0.10-0.06 (m, 4H); (ES-MS): *m*/*z* 314 (M-34, 100%); IR (neat, cm⁻¹): 3075, 1693, 1021.

### Step (vi): Synthesis of 3-((3,5-bis(trifluoromethyl)benzylamino)methyl)-N,N-bis(cyclopropylmethyl)-8-trifluoromethylquinolin-2-amine

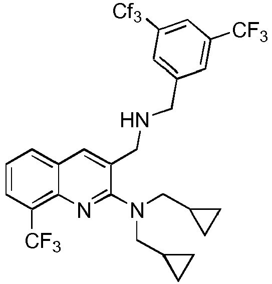

This compound (yield: 98%) was prepared by following same procedure as in step (iii) of Example 1 by using 2-(bis(cyclopropylmethyl)amino)-8-trifluoromethylquinoline-3-carbaldehyde instead of 2-(bis(cyclopropylmethyl)amino)-8-methylquinoline-3-carbaldehyde.
¹H NMR (CDCl₃, 400 MHz): δ 8.20-8.18 (m, 1H), 7.97-7.94 (m, 1H), 7.91-7.88 (m, 3H), 7.73-7.69 (m,1H), 7.52-7.48 (m, 1H), 4.98 (s, 2H), 4.77 (s,2H), 3.17-3.15 (m, 4H), 0.79-0.73 (m, 2H), 0.28-0.26 (m, 4H), 0.003-.001 (m, 4H); (ES-MS): *m*/*z* 542 (M-33, 40%), 299 (M-276, 100%); IR (neat, cm⁻¹): 3005, 1278, 1134, 1018.

### Step (vii): Synthesis of N-(3,5-bis(trifluoromethyl)benzyl)-N-((2-(bis(cyclopropylmethyl)amino)-8-trifluoromethylquinolin-3-yl)methyl)cyanamide

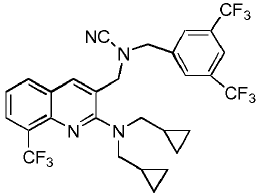

This compound (1.5 g, yield: 85%) was prepared following the same procedure as mentioned in step (iv) of Example 1 by using 3-((3,5-bis(trifluoromethyl)benzylamino)methyl)-*N*,*N*-bis(cyclopropylmethyl)-8-trifluoromethylquinolin-2-amine instead of 3-((3,5-bis(trifluoromethyl)benzylamino)methyl)-N,N bis(cyclopropylmethyl)-8-methylquinolin-2-amine.
¹H NMR (CDCl₃, 400 MHz): δ 8.03-8.00 (m,2H), 7.91-7.90 (m, 3H), 7.73-7.69 (m,1H), 7.54-7.50 (m, 1H), 4.60 (s,2H) 4.53 (s,2H), 3.28-3.26 (m, 4H), 0.94-0.87 (m, 2H), 0.043-0.032 (m, 4H). 0.019-0.004 (m, 4H); (ES-MS): *m*/*z* 567 (M-33, 100%); IR (Neat, cm⁻¹): 3006, 2210, 1276,1012.

### Step (viii): Synthesis of (3-{[(3,5-bistrifluoromethyl-benzyl)-(2H-tetrazol-5-yl)-amino]-methyl}-8-trifluoromethyl-quinolin-2-yl)-bis-cyclopropylmethyl-amine

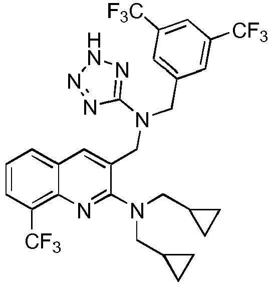

This compound (0.8 g, yield: 56 %) was prepared by following the same procedure as mentioned in step (v) of Example 1 by using *N*-(3,5-bis(trifluoromethyl)benzyl)-*N-*((2-(bis(cyclopropylmethyl)amino)-8-trifluoromethylquinolin-3-yl)methyl)cyanamide instead of *N-*(3,5-bis(trifluoromethyl)benzyl)-*N-*((2-(bis(cyclopropylmethyl)amino)-8-methylquinolin-3-yl)methyl)cyanamide.
¹H NMR (CDCl₃, 400 MHz): δ 8.69 (d, J=8.3 Hz, 1H), 8.18-8.13 (m,1H), 8.00-7.98 (m, 1H), 7.88-7.84 (m, 1H), 7.72-7.64 (m, 3H), 7.52-7.48 (m, 1H), 5.28 (s, 2H), 5.12 (s, 2H), 3.27-3.25 (m, 4H), 0.90-0.73 (m, 2H), 0.43-0.26 (m, 4H), 0.03-0.002 (m, 4H); (ES-MS): *m*/*z* 617 (M-26, 80%), 574 (M-69, 100%); IR (KBr, cm⁻¹): 3680, 2922 , 1660 , 1616.

### Step (ix): Synthesis of (3-{[3,5-bistrifluoromethyl-benzyl)-(2-methyl-2H-tetrazole-5-yl)-amino]-methyl-}-8-trifluoromethyl-quinoline-2-yl)-bis-cyclopropylmethyl-amine

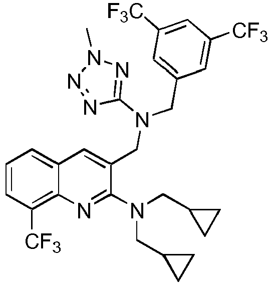

To a suspension (3-{[(3,5-bistrifluoromethyl-benzyl)-(2H-tetrazol-5-yl)-amino]-methyl}-8-trifluoromethyl-quinolin-2-yl)-bis-cyclopropylmethyl-amine (0.5 g, 0.77 mmol) in water (4 mL), sodium hydroxide (0.062 g, 1.55 mmol) was added and stirred for 15 min at RT followed by the addition of dichloromethane (4 mL). To this reaction mixture, dimethyl sulphate (0.117 g, 0.933 mmol) was added followed by the addition of tetra butyl ammonium bromide (0.012 g, 0.038 mmol). The reaction was stirred for 1 h. The organic layer was separated from aqueous layer and the aqueous layer was extracted with DCM (3 x 10 mL), the combined organic layer was washed with brine, dried over sodium sulphate and then concentrated under vacuum to afford the crude residue which was purified by column chromatography over 100-200 mesh silica gel using 8% ethyl acetate and petroleum ether to afford Synthesis of (3-{[3,5-bis trifluoromethyl-benzyl )-(2-methyl -2H-tetrazole -5-yl)-amino]-methyl-}-8-trifluoromethyl-quinoline-2-yl)- bis-cyclopropylmethyl-amine (0.1 g, 20%)..
¹H NMR (CDCl₃, 400 MHz): δ 8.67-8.65 (m, 1H), 8.19-8.17 (m, 1H), 7.83-7.79 (m, 1H), 7.66-7.64 (m, 4H). 5.36 (s, 2H), 5.05 (s, 2H), 4.15 (s, 3H), 3.28 (s, 4H), 0.99-0.93 (m, 2H), 0.40-0.31(m, 4H), 0.03-0.01(m, 4H); (ES-MS): *m*/*z* 631 (M-26, 100%); IR (KBr, cm⁻¹): 3428, 3082, 2925; 1618.

### Example 9

### Synthesis of (3-{[3,5-bis trifluoromethyl-benzyl)-(2-methyl -2H-tetrazole-5-yl)-amino]-methyl-}-8-Chloro-quinoline-2-yl)-bis-cyclopropylmethyl-amine

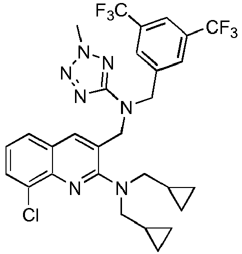

This compound was prepared by following same procedure as in step (ix) of Example 8 by using (3-{[(3,5-bistrifluoromethyl-benzyl)-(2H-tetrazol-5-yl)-amino]-methyl}-8-chloro-quinolin-2-yl)-bis-cyclopropylmethyl-amine instead of (3-{[(3,5-bistrifluoromethyl-benzyl)-(2H-tetrazol-5-yl)-amino]-trifluoromethyl}-8-methyl-quinolin-2-yl)-bis-cyclopropylmethyl-amine.
¹H NMR (CDCl₃, 400 MHz): δ 7.83 (s, 1H), 7.71-7.67 (m, 4H), 7.47-7.45 (m,1H), 7.25-7.18 (m,1H), 4.88 (s, 2H), 4.64 (s, 2H), 4.22 (s, 3H), 3.27-3.25 (m, 4H), 0.89-0.83 (m, 2H), 0.43-0.38 (m, 4H), 0.15-0.07 (m, 4H); (ES-MS): *m*/*z* 624 (M+1, 100%); IR (KBr, cm⁻¹): 2926, 1578, 1280.

### Example 10

### Synthesis of 3-{[3,5-bis trifluoromethyl-benzyl)-(2-methyl -2H-tetrazole-5-yl)-amino]-methyl-}-8-methoxy-quinoline-2-yl)-bis-cyclopropylmethyl-amine

### Step (i): Synthesis of (3-{[(3,5-bistrifluoromethyl-benzyl)-(2H-tetrazol-5-yl)-amino]-methyl}-8-methoxy-quinolin-2-yl)-bis-cyclopropylmethyl-amine

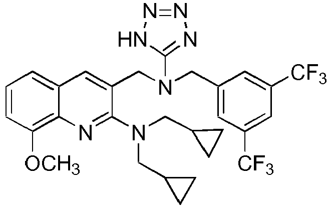

This compound was prepared by following the same procedure except using *N-*(2-methoxy phenyl) acetamide instead of *N*-o-tolyl acetamide in step (i) of Example 1. **Step** (**ii**)**:** Synthesis of 3-{[3,5-bis trifluoromethyl-benzyl)-(2-methyl -2H-tetrazole - 5-yl)-amino]-methyl-}-8-methoxy-quinoline-2-yl)- bis-cyclopropylmethyl-amine

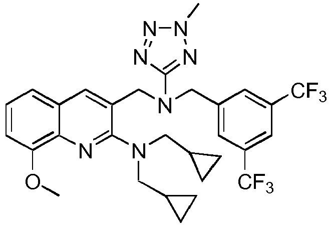

This compound was prepared by following the same procedure by using 3-{[(3,5-bistrifluoromethyl-benzyl)-(2H-tetrazol-5-yl)-amino]-methyl}-8-methoxyquinolin-2-yl)-bis-cyclopropylmethyl-amine in step (ix) of Example 8
Purity 98.43% (HPLC: Symmetry Shield RP8 (150x4.6) 20:80 [0.01M KH₂PO₄: CH₃CN], 217 nm, Rₜ 11.095 min); ¹H NMR (CDCl₃, 400 MHz): δ 7.82 (s, 1H), 7.71 (s, 1H), 7.65 (s, 2H), 7.24-7.22 (m, 1H), 7.17-7.15 (m, 1H), 6.98-6.96 (m, 1H), 4.91 (s, 2H), 4.65 (s, 2H), 4.21 (s, 3H), 4.03 (s, 3H), 3.22 -3.20 (m, 4H), 1.27-1.25 (m, 2H), 0.40-0.35 (m, 4H), 0.12-0.09 (m, 4H); (ES-MS): *m*/*z* 618 (M⁺-1, 100%); IR (neat, cm⁻¹) 3422, 1581, 1279.

### Example 11

### Synthesis of 3-{[3,5-bistrifluoromethyl-benzyl)-(2-methyl-2H-tetrazole-5-yl)-amino]-methyl-}-8-isopropyl-quinoline-2-yl)- bis-cyclopropylmethyl-amine

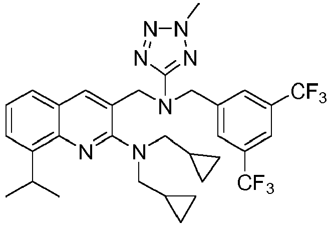

This compound was prepared as pale yellow oil (yield: 53%) by following the same procedure except using *N*-(2-isopropyl phenyl) acetamide instead of *N*-(2-methoxy phenyl) acetamide in step (i) of Example 10.
Purity 98.01% (HPLC: Symmetry Shield RP8 (150x4.6) 20:80 [0.01M KH₂PO₄: CH₃CN], 217 nm, Rₜ 11.608 min); ¹H NMR (CDCl₃, 400 MHz): δ 7.84 (s, 1H), 7.71-7.69 (m, 3H), 7.48-7.47 (m, 1H), 7.44-7.41 (m, 1H), 7.32-7.28 (m, 1H), 4.91 (s, 2H), 4.68 (s, 2H), 4.19-4.14 (m, 1H), 3.96 (s, 3H), 3.18 (d, *J=* 5.4 Hz, 4H), 1.37 (d, *J =* 6.7 Hz, 6H), 0.95-0.93 (m, 2H), 0.38-0.34 (m, 4H), 0.07-0.04 (m, 4H); (ES-MS): *m*/*z* 632 (M⁺+1, 100%); IR (neat, cm⁻¹) 3079, 2960, 1279.

### Example 12

### Synthesis of 2- {5-[[2-(bis-cyclopropylmethyl-amino)-8-methyl-quinoline-3-ylmethyl]- (3,5-bistrifluoromethyl-benzyl)-amino]-tetrazol-2-yl}-ethanol

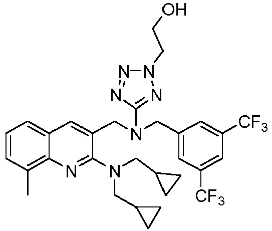

This compound (yield: 56%) was prepared following the same procedure as in Example 1 except using 2-bromoethanol instead of cyclopropanemethyl bromide. ¹H NMR (CDCl₃, 400 MHz): δ 7.84 (s,1H), 7.69 (d, *J =* 9.4 Hz, 3H), 7.44-7.41 (m, 2H), 7.24-7.20 (m, 1H), 4.67 (s, 2H), 4.63 (s, 2H), 4.62-4.60 (m, 2H), 4.16-4.09 (m, 2H), 3.19-3.18 (m, 4H), 2.71 (s, 3H), 1.02-1.00 (m, 2H), 0.40-0.35 (m, 4H), 0.09-0.07 (m, 4H).
(ES-MS): *m*/*z* 634 (M⁺+1, 100%); IR (neat, cm⁻¹): 3415, 1583.

### Example 13

### Synthesis of [3-({(3,5-bis-trifluoromethyl-benzyl)-[2-(2-methoxy-ethyl)-2H-tetrazol-5-yl]-amino}-methyl)-8-methyl-quinolin-2-yl]-bis-cyclopropylmethyl-amine

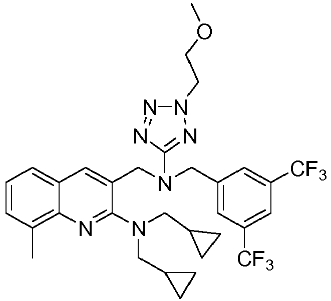

To the 2- {5-[[2-(bis-cyclopropylmethyl-amino)-8-methyl-quinoline-3-ylmethyl]- (3,5-bistrifluoromethyl-benzyl)-amino]-tetrazol-2-yl}-ethanol (0.08 g, 0.126 mmol), (example 12), was added NaOH (0.010 g, 0.25 mmol) and water (1 mL). The reaction was stirred at room temperature for 15 min and then DCM (1 mL) was added followed by the addition of DMS (0.019 g, 0.15 mmol) and TBAB (0.002 g, 0.006 mmol). The reaction was continued for overnight. The solvent was evaporated and the aqueous layer was back extracted with DCM (2 x 10 mL). The combined organic layer was washed with brine and dried over sodium sulfate. The sovent was evaporated on rotavapor. The residue obtained was purified by preparative thin layer chromatography and eluted with 15% ethyl acetate and petroleum ether to get the desired product (0.30 g, 56%).
¹H NMR (CDCl₃, 400 MHz): δ 7.83 (s,1H), 7.69-7.67 (m, 4H), 7.44 -7.42 (m, 1H), 7.23-7.21 (m, 1H), 4.91 (s, 2H), 4.76-4.74 (m, 2H), 4.66-4.63 (m, 4H), 3.75 (s, 3H), 3.18 (d, *J =* 6.7 Hz, 4H), 2.71 (s, 3H), 0.89-0.83 (m, 2H), 0.39-0.35 (m, 4H), 0.09-0.07 (m, 4H).
(ES-MS): *m*/*z* 648 (M⁺, 100%), 692 (M⁺+ 45, 100%).
IR (neat, cm⁻¹): 2925, 1279.

### Example 14

### Synthesis of 3-{[3,5-bis trifluoromethyl-benzyl)-(2-methyl -2H-tetrazole-5-yl)-amino]-methyl-}-7,8-dimethyl-quinoline-2-yl)-bis-cyclopropylmethyl-amine

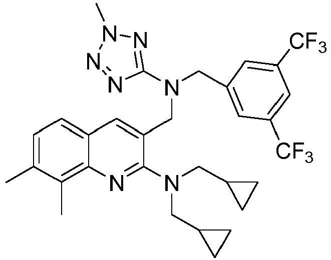

This compound was prepared as a light green oil (yield: 32%) by following the same procedure except using *N*-(2,3-dimethyl-phenyl)-acetamide instead of *N*-(2-methoxy phenyl) acetamide in step (i) of Example 10
Purity 99.31% (HPLC: Symmetry Shield RP8 (150x4.6) 20:80 [0.01M KH₂PO₄: CH₃CN], 220 nm, Rₜ 11.88 min); ¹H NMR (CDCl₃, 400 MHz): δ 7.78 (s, 1H), 7.69-7.67 (m, 2H), 7.34-7.32 (m, 1H), 7.16-7.14 (m, 1H), 4.90 (s, 2H), 4.64 (s, 2H), 4.21 (s, 3H), 3.17 (d, *J=* 7.0 Hz, 4H), 2.67 (s, 3H), 2.45 (s, 3H), 1.02-0.98 (m, 2H), 0.37-0.36 (m, 4H), 0.35-0.34 (m, 4H); (ES-MS): *m*/*z* 618 (M⁺+1, 100%); IR (neat, cm⁻¹) 2926, 1582, 1185.

### Comparitive Example 15

### Synthesis of 3-1[3,5-bistrifluoromethyl-benzyl)-(2-methyl-2H-tetrazole-5-yl)-amino]-methyl-}-benzo[h]quinolin-2-yl)-bis-cyclopropylmethyl-amine

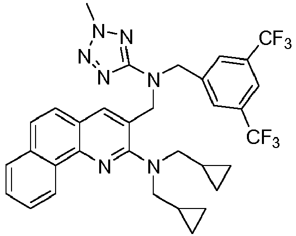

This compound was prepared as oil (0.2 g, 25%) by following the same procedure except using N-naphthalen-1-yl-acetamide instead of *N*-(2-methoxy phenyl) acetamide in step (i) of Example 10.
Purity 97.96% (HPLC: Symmetry Shield RP8 (150x4.6) 20:80 [0.01M KH₂PO₄: CH₃CN], 210 nm, Rₜ 11.69 min); ¹H NMR (CDCl₃, 400 MHz): δ 9.21-9.18 (m, 1H), 7.91 (s, 2H), 7.86-7.64 (m, 6H), 7.62-7.49 (m, 1H), 4.96 (s, 2H), 4.60 (s, 2H), 4.20 (s, 3H), 3.27 (d, *J =* 7.0 Hz, 4H), 1.06-1.01 (m, 2H), 0.99-0.85 (m, 4H), 0.86-0.35 (m, 4H)
(ES-MS): *m*/*z* 640 (M⁺+1, 100%); IR (neat, cm⁻¹) 2927, 1582, 1134.

### Example 16

### Synthesis of (S)-(+)-(3-{[(3,5-bis-trifluoromethyl-benzyl)-(2-methyl-2H-tetrazol-5-yl)-amino]methyl}-8-methyl-quinolin-2-yl)-ethyl-(tetrahydro-furan-2-ylmethyl)-amine

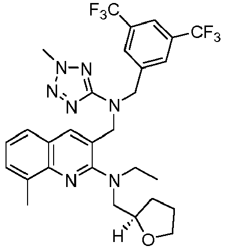

The title compound was obtained as a colorless thick liquid (yield: 40%) from 2-[ethyl-(tetrahydro-furan-2-ylmethyl)-amino]-8-methyl-quinoline-3-carbaldehyde by following analogues procedure as described in Example 1.
Purity 98.58 % (HPLC: YMC C8, 20:80 [KH₂PO₄ (0.01 M, pH 3.2):CH₃CN], Rₜ 8.66 min); ¹H NMR (CDCl₃, 300 MHz): δ 7.83 (s, 1H), 7.70-7.67 (m, 3H), 7.45-7.39 (m, 2H), 7.22-7.19 (m, 1H), 4.89 (s, 2H), 4.70 (d, *J =* 8.5 Hz, 2H), 4.21 (s, 3H), 4.18-4.08 (m, 1H), 3.86-3.76 (m, 1H), 3.69-3.60 (m, 1H), 3.56-3.43 (m, 2H), 3.36-3.22 (m, 2H), 2.69 (s, 3H), 1.86-1.78 (m, 3H), 1.55-1.46 (m, 1H), 1.11 (t, *J =* 6.81 Hz, 3H); [□]_{D} +15.2°, c = 0.25% in MeOH; MS (ESI) *m*/*z* 608 (M+1)⁺.

### Example 17

### Synthesis of (R)-(-)-(3-{[(3,5-bis-trifluoromethyl-benzyl)-(2-methyl-2H-tetrazol-5-yl)-amino]methyl}-8-methyl-quinolin-2-yl)-ethyl-(tetrahydro-furan-2-ylmethyl)-amine

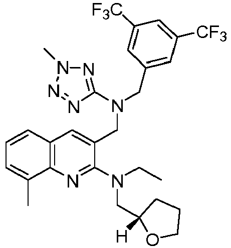

The title compound was obtained as a colorless thick liquid (10%) in a similar manner described in Example 16.
Purity: 92.36 % (HPLC: YMC C8, 20:80 [KH₂PO₄ (0.01 M, pH 3.2):CH₃CN], Rₜ 40.98 min); ¹H NMR (CDCl₃, 300 MHz): δ 7.83 (s, 1H), 7.70-7.67 (m, 3H), 7.45-7.39 (m, 2H), 7.22-7.19 (m, 1H), 4.89 (s, 2H), 4.70 (d, *J=* 8.5 Hz, 2H), 4.21 (s, 3H), 4.18-4.08 (m, 1H), 3.86-3.76 (m, 1H), 3.69-3.60 (m, 1H), 3.56-3.43 (m, 2H), 3.36-3.22 (m, 2H), 2.69 (s, 3H), 1.86-1.78 (m, 3H), 1.52-1.46 (m, 1H), 1.10 (t, *J =* 6.81 Hz, 3H); [□]_{D} -10.0°, c = 0.25% in MeOH; MS (ESI) *m*/*z* 608 (M+1)⁺.

### Example 18

### Synthesis of (5-{[(3,5-bis-trifluoromethyl-benzyl)-(2-methyl-2H-tetrazol-5-yl)-amino]-methyl}-1-phenyl-1H-pyrazolo[3,4-b]pyridin-6-yl)-bis-cyclopropylmethyl-amine

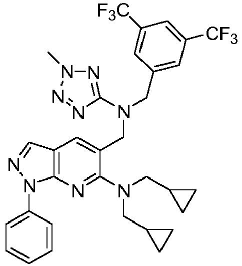

The title compound was obtained as a colorless thick liquid (63%) in a similar manner described in Example 8, by using 6-chloro-1-phenyl-1H-pyrazole[3,4-b]pyridine-5-carbaldehyde (starting material synthesized according to the procedure described in US 2858309).
Purity: 99.03 % (HPLC: YMC C8, 20:80 [KH₂PO₄ (0.01 M, pH 3.2): CH₃CN], Rₜ 10.78 min); ¹H NMR (CDCl₃, 300 MHz): δ 8.40 (d, *J=* 7.72 Hz, 2H), 7.95 (s, 1H), 7.85 (s, 1H), 7.72-7.68 (m, 3H), 7.53-7.47 (m, 2H), 7.29-7.24 (m, 1H), 4.87 (s, 2H), 4.65 (s, 2H), 4.22 (s, 3H), 3.19 (d, *J=* 6.60 Hz, 2H), 1.0-0.95 (m, 2H), 0.40-0.37 (m, 4H), 0.081-0.047 (m, 4H); MS (ESI) *m*/*z* 656 (M+1)⁺.

### Example 19

### Synthesis of (5-{[(3,5-bis-trifluoromethyl-benzyl)-(5-bromo-pyrimidin-2-yl)-amino]-methyl}-1,3-dimethyl-1H-pyrazolo[3,4-b]pyridin-6-yl)-bis-cyclopropylmethyl-amine

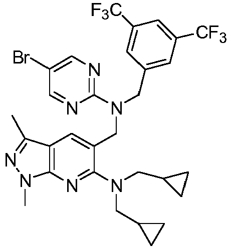

The title compound was obtained as a colorless thick liquid (yield: 44%), heating a solution of {5-[(3,5-bis-trifluoromethyl-benzylamino)-methyl-1,3-dimethyl-1H-pyrazolo[3,4-b]pyridin-6-yl}-bis-cyclopropylmethyl-amine (0.3 g, 0.6 mmol), 5-bromo-2-chloropyrimidine (0.09 g, 0.5 mmol), diisopropylethylamine (1 mmol) in isopronaol at 80-100 °C for 4-6 h followed by evaporation of volatiles and purification of the crude by chromatography using silica gel.
Purity: 97.06 % (HPLC: YMC C8, 20:80 [KH₂PO₄ (0.01 M, pH 3.2):CH₃CN], Rₜ 22.71 min); ¹H NMR (CDCl₃, 300 MHz): δ 8.4 (s, 2H), 7.72-7.58 (m, 4H), 5.03 (s, 2H), 4.77 (s, 2H), 3.95 (s, 3H), 3.13 (d, *J=* 6.0 Hz, 4H), 2.39 (s, 3H), 0.98-0.82 (m, 2H), 0.46-0.35 (m, 4H), 0.18-0.08 (m, 4H); MS (ESI) *m*/*z* 683 (M+1)⁺, 684 (M+2)⁺.

### Example 20

### Synthesis of (5-{[(3,5-bis-trifluoromethyl-benzyl)-(2-methyl-2H-tetrazol-5-yl)-amino]methyl}-1-cyclopropylmethyl-1H-pyrazolo[3,4-b]pyridin-6-yl)-cyclobutylmethyl-ethyl-amine

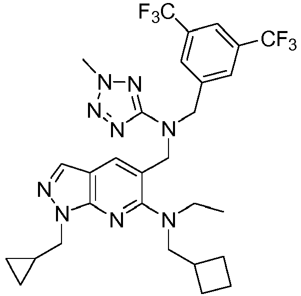

The title compound was obtained as a colorless thick liquid (yield: 27%) in a similar method described in Example 18, except using 6-chloro-1-cyclopropylmethyl-1H-pyrazolo[3,4-b]pyridine-5-carbaldehyde (starting material prepared in a similar method described in US 2965643).
Purity: 97.84 % (HPLC: YMC C8, 20:80 [KH₂PO₄ (0-01 M, pH 3.2):CH₃CN], Rₜ 3.30 min); ¹H NMR (CDCl₃, 300 MHz): δ 7.77-7.63 (m, 5H), 4.77 (s, 2H), 4.62 (s, 2H), 4.27 (d, *J* = 7.04 Hz, 2H), 4.22 (s, 3H), 3.22-3.52 (m, 4H), 1.88-1.69 (m, 4H), 1.54-1.30 (m, 4H), 1.03 (t, *J =* 7.04 Hz, 3H), 0.55-0.46 (m, 4H); MS (ESI) *m*/*z* 622 (M+1)⁺.

### Example 21

### Synthesis of (5-{[(3,5-bis-trifluoromethyl-benzyl)-(5-bromo-pyrimidin-2-yl)-amino]-methyl}-1-ethyl-1H-pyrazolo[3,4-b]pyridin-6-yl)-bis-cyclopropylmethyl-amine:

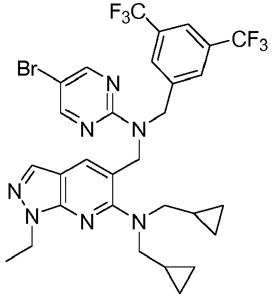

The title compound was obtained as a colorless thick liquid (0.25 g, yield: 66%) in a similar manner described in Example 19.
Purity: 93.48 % (HPLC: YMC C8, 20:80 [KH₂PO₄ (0.01 M, pH 3.2):CH₃CN], Rₜ 11.97 min); ¹H NMR (CDCl₃, 300 MHz): δ 8.39 (s, 2H), 7.74-7.67 (m, 5H), 5.01 (s, 2H), 4.77 (s, 2H), 4.48 (q, *J =* 7.26 Hz, 2H), 3.13 (d, *J =* 6.58 Hz, 4H), 1.53 (t, *J =* 7.26 Hz, 3H), 0.82-0.96 (m, 2H), 0.40-0.36 (m, 4H), 0.07-0.04 (m, 4H); MS (ESI) *m*/*z* 683 (M+1)⁺, 684 (M+2)⁺.

### Example 22

### Synthesis of (5-{[(3,5-bis-trifluoromethyl-benzyl)-(5-(4-morpholin-4-yl-phenyl)-pyrimidin-2-yl]-amino}-methyl)-1-ethyl-1H-pyrazolo[3,4-b]pyridin-6-yl)-bis-cyclopropylmethyl-amine

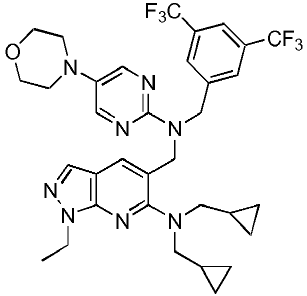

A mixture of (5-[(3,5-bis-trifluoromethyl-benzyl)-(5-bromo-pyrimidin-2-yl)-amino]-methyl}-1-ethyl-1H-pyrazolo[3,4-b]pyridin-6-yl)-bis-cyclopropylmethyl-amine 0.21 g, 0.3 mmol), tris(dibenzylideneacetone)dipalladium (0.05 g, 0.05 mmol), (2-biphenyl)-di-*tert*-butylphosphine (0.01 g, 0.03 mmol), sodium tert-butoxide (0.06 g, 0.6 mmol), morpholine (0.05 ml, 0.6 mmol) in toluene was stireed at ambient temperature for 24 h. Thereafter, the reaction mixture was quenched with water and extracted with ethyl acetate (2 x 30 mL). The organic extracts were combined, washed with brine, dried over sodium sulfate and concentrated in *vacuo*. The crude was purified by chromatography using silica gel to afford the title compound as a pale yellow liquid (0.085 g, 40%).
Purity: 96.84 % (HPLC: YMC C8, 20:80 [KH₂PO₄ (0.01 M, pH 3.2):CH₃CN], PDA 264 nm, flow rate 1.2 mL/min, Rₜ 11.57 min); ¹H NMR (CDCl₃, 300 MHz): δ 8.18 (s, 2H), 7.76-7.66 (m, 5H), 5.03 (s, 2H), 4.80 (s, 2H), 4.48 (q, *J* = 7.27 Hz, 2H), 3.89 (t, *J =* 4.77 Hz, 2H), 3.28-3.05 (m, 8H), 1.52 (t, *J =* 7.27 Hz, 3H), 0.82-0.96 (m, 2H), 0.42-0.33 (m, 4H), 0.09-0.05 (m, 4H); MS (ESI) *m*/*z* 689 (M+1)⁺.

### Example 23

### Synthesis of (3-{[(3,5-bis-trifluoromethyl-benzyl)-(5-bromo-pyrimidin-2-yl)-amino]-methyl}-8-methyl-quinolin-2-yl)-cyclobutylmethyl-ethyl-amine

### Step (i): Preparation of 2-(cyclobutylmethyl-ethyl-amino)-8-methyl-quinoline-3-carbaldehyde:

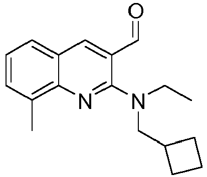

Potassium carbonate (0.62 g, 4.5 mmol) was added to a solution of 2-chloro-8-methyl-quinoline-3-carbaldehyde (0.45 g, 2.2 mmol) and cyclobutylmethyl-ethyl-amine (0.47 g, 4.1 mmol) in anhydrous dimethyl formamide (5 mL) under nitrogen. After stirring for 0.5 h at RT, the reaction mixture was heated for 20 h at 80-95 °C. Thereafter, the reaction was cooloed to RT, water (30 mL) and ethyl acetate (30 mL) were added, and the organic layer was separated from the aqeous mixture. The organic extract was washed with brine, dried over sodium sulfate and the solvent was evaporated in *vacuo*. The residue was purified by chromatography using silica gel to afford the title compound as a pale yellow liquid (0.46 g, 75%); ¹H NMR (CDCl₃, 300 MHz): δ 10.13 (s, 1H), 8.41 (s, 1H), 7.61 (d, *J=* 8.17, 1H), 7.53 (d, *J=* 7.72, 1H), 7.61 (t, *J=* 7.72, 1H), 3.55-3.46 (m, 4H), 2.80-2.74 (m, 1H), 2.65 (s, 3H), 2.01-1.67 (m, 6H), 1.23 (t, *J=* 7.04 Hz, 3H).

### Step (ii): Preparation of {3-[(3,5-bis-trifluoromethyl-benzylamino)-methyl]-8-methyl-quinolin-2-yl}-cyclobutylmethyl-ethyl-amine

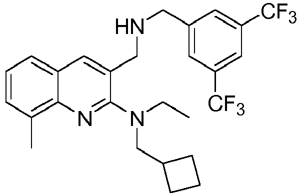

To a solution of 2-(cyclobutylmethyl-ethyl-amino)-8-methyl-quinoline-3-carbaldehyde (0.45 g, 1.6 mmol) and bis-trifluoromethyl-benzylamine (0.39 g, 1.6 mmol) in anhydrous dichloroethane (10.0 mL) was added glacial acetic acid (0.4 mL) and stirred for 20 min at RT. Sodium triacetoxyborohydride (0.68 g, 3.2 mmol) was added slowly and the reaction was stirred overnight at RT. After evaporation of volatiles in *vacuo*, water (30 mL) and ethyl acetate (30 mL) were added to the residue and the organic layer was allowed to separate. The organic layer was collected and washed with brine, dried over sodium sulfate, and the solvent was evaporated in *vacuo*. The residue was purified by chromatography using silica gel to afford the desired compound as a colorless liquid (0.485 g, yield: 60%).
¹HNMR (CDCl₃, 300 MHz): δ 7.89-7.75 (m, 4H), 7.53-7.44 (m, 2H), 7.28-7.23 (m, 1H), 3.94 (s, 2H), 3.79 (s, 2H), 3.37-3.24 (m, 4H), 2.70 (s, 3H), 2.69-2.58 (m, 1H), 1.88-1.57 (m, 6H), 1.16 (t, *J=* 7.04 Hz, 3H).

### Step (iii): Preparation of (3-{[(3,5-bis-trifluoromethyl-benzyl)-(5-bromo-pyrimidin-2-yl)-amino]-methyl}-8-methyl-quinolin-2-yl)-cyclobutylmethyl-ethyl-amine

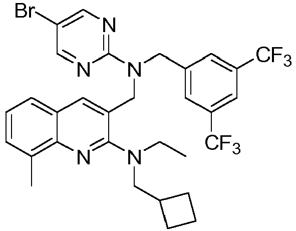

A mixture of {3-[(3,5-bis-trifluoromethyl-benzylamino)-methyl]-8-methyl-quinolin-2-yl}-cyclobutylmethyl-ethyl-amine (0.23 g, 0.46 mmol), 5-bromo-2-chloropyrimidine (0.09 g, 0.46 mmol), and potassium fluoride (0.11 g, 1.8 mmol) in dimethyl formamide (10 ml) was heated at 80 °C overnight. After cooling the reaction to ambient temperature water (10 mL) and EtOAc (20 mL) were added and stirred for 0.5 h. Thereafter, the organic layer was separated, washed with brine, dried over sodium sulfate, filtered and solvent evaporated in *vacuo*. The crude was purified by chromatography using silica gel to afford the title compound as a colorless liquid (0.14 g, 47%).
Purity: 96.74 % (HPLC: YMC C8, 20:80 [KH₂PO₄ (0.01 M, pH 3.2):CH₃CN], PDA 240 nm, flow rate 1.2 mL/min, Rₜ 65.59 min); ¹HNMR (CDCl₃, 300 MHz): δ 8.40 (s, 2H), 7.71-7.66 (m, 4H), 7.43-7.38 (m, 2H), 7.22-7.17 (m, 1H), 4.96 (s, 2H), 4.75 (s, 2H), 3.30 (d, *J =* 7.04 Hz, 2H), 3.2 (q, *J =* 7.04 Hz, 2H), 2.69 (s, 3H), 2.67-2.60 (m, 1H), 1.87-1.54 (m, 6H), 1.10 (t, *J =* 7.04 Hz, 3H). MS (ESI) *m*/*z* 667 (M+1)⁺; 668 (M+2)⁺.

### Example 24

### Synthesis of (3-{[(3,5-bis-trifluoromethyl-benzyl)-(5-bromo-pyrimidin-2-yl)-amino]-methyl}-8-methyl-quinolin-2-yl)-bis-cyclopropylmethyl-amine:

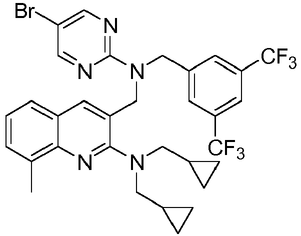

The title compound was obtained as a colorless liquid in a similar manner described in Example 23 (0.32 g, 82%).
Purity: 98.60 % (HPLC: YMC C8, 20:80 [KH₂PO₄ (0.01 M, pH 3.2): CH₃CN], PDA 254 min, flow rate 1.2 mL/min, Rₜ 51.49 min); ¹H NMR (CDCl₃, 300 MHz): δ 8.40 (s, 2H), 7.24-7.68 (m, 4H), 7.44-7.38 (m, 2H), 7.22-7.18 (m, 1H), 5.06 (s, 2H), 4.78 (s, 2H), 3.21 (d, *J* = 6.4 Hz, 4H), 2.71 (s, 3H), 1.04-0.97 (m, 2H), 0.39-0.34 (m, 4H), 0.09-0.06 (m, 4H); MS (ESI) *m*/*z* 679 (M+1)⁺; 680 (M+2)⁺.

### Example 25

### Synthesis of (3-{[(3,5-bis-trifluoromethyl-benzyl)-(5-morpholin-4-yl-pyrimidin-2-yl)-amino]-methyl}-8-methyl-quinolin-2-yl)-bis-cyclopropylmethyl-amine

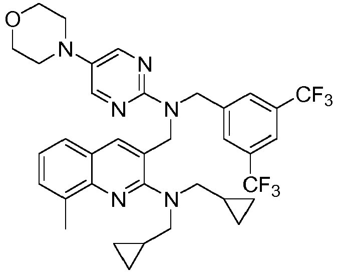

A mixture of (3-{[(3,5-bis-trifluoromethyl-benzyl)-(5-bromo-pyrimidin-2-yl)-amino]-methyl}-8-methyl-quinolin-2-yl)-bis-cyclopropylmethyl-amine (0.32 g, 0.47 mmol), tris(dibenzylideneacetone)dipalladium (0.07 g, 0.08 mmol), (2-biphenyl)-di-*tert*-butylphosphine (0.02 g, 0.06 mmol), sodium tert-butoxide (0.09 g, 1 mmol), morpholine (0.08 ml, 0.9 mmol) in toluene was stirred at ambient temperature for 24 h. Therafter, the reaction mixture is quenched with water and extracted with ethyl acetate (2 x 30 mL). The combined organic extracts was washed with brine, dried over sodium sulfate and concentrated in *vacuo.* The crude mixture was purified by chromatography using silica gel to afford the title compound as a colorless solid (0.11 g, 35%).
Mp: 123-124 °C; Purity: 99.46 % (HPLC: YMC Pro C8, 20:80 [KH₂PO₄ (0-01 M, pH 3.2):CH₃CN], PDA 264 nm, flow rate 1.2 mL/min, Rₜ 31.71 min)]; ¹H NMR (CDCl₃, 300 MHz): δ 8.18 (s, 2H), 7.75-7.67 (m, 4H), 7.43-7.38 (m, 2H), 7.22-7.18 (m, 1H), 5.05 (s, 2H), 4.79 (s, 2H), 3.91-3.85 (m, 4H), 3.21 (d, *J=* 6.0 Hz, 4H), 3.09-3.06 (m, 4H), 2.71 (s, 3H), 1.03-.099 (m, 2H), 0.39-0.34 (m, 4H), 0.09-0.07 (m, 4H); MS (ESI) *m*/*z* 685 (M+1)⁺.

### Example 26

### Synthesis of (3-{[(3,5-bis-trifluoromethyl-benzyl)-(5-ethyl-pyrimidin-2-yl)-amino]-methyl}-8-methyl-quinolin-2-yl)-bis-cyclopropylmethyl-amine:

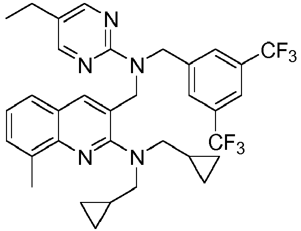

A mixture of (3-{[(3,5-bis-trifluoromethyl-benzylamino)-methyl]-8-methyl-quinolin-2-yl}-bis-cyclopropylmethyl-amine (0.32 g, 0.61 mmol) and 2-chloro-5-ethylpyrimidine (0.4 mL, 3 mmol) in 1,3-dimethyl-2-imidazolidinone (5-10 mL) was stirred overnight at 100 °C. After evaporation of volatiles the crude was purified by chromatography using silica gel to afford the title compound as a colorless liquid (0.06 g, 16%).
Purity: 91.67 % (HPLC: YMC Pro C8), 20:80 [KH₂PO₄ (0.01 M, pH 3.2):CH₃CN], PDA 240 nm, flow rate 1.2 mL/min, PDA 240 nm; Rₜ 44.76 min)]; ¹H NMR (CDCl₃, 300 MHz): δ 8.28 (s, 2H), 7.76-7.69 (m, 4H), 7.43-7.38 (m, 2H), 7.22-7.17 (m, 1H), 5.08 (s, 2H), 4.81 (s, 2H), 3.22 (d, *J* = 6.36 Hz, 4H), 2.71 (s, 3H), 2.56 (q, *J* = 7.5 Hz, 2H), 1.29 (t, *J* = 7.5 Hz, 3H), 1.08-0.98 (m, 2H), 0.39-0.34 (m, 4H), 0.12-0.06 (m, 4H); MS (ESI) *m*/*z* 628 (M+1)⁺.

### Example 27

### Synthesis of N-[2-(bis-cyclopropylmethyl-amino)-8-methyl-quinolin-3-ylmethyl]-N-(3,5-bis-trifluoromethyl-benzyl)-N,N'-dimethyl-pyrimidine-2,5-diamine

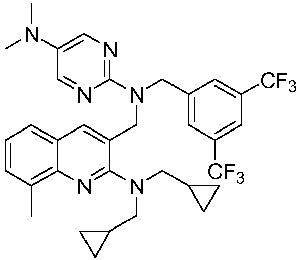

The title compound was obtained in a similar manner described in Example 25 as a colorless liquid (0.10 g, 37%).
Purity: 99.43 % (HPLC: YMC Pro C8, 20:80 [KH₂PO₄ (0.01 M, pH 3.2):CH₃CN], PDA 264 nm, flow rate 1.2 mL/min, Rₜ 31.35 min); ¹H NMR (CDCl₃, 300 MHz): δ 8.11 (s, 2H), 7.75-7.66 (m, 4H), 7.42-7.37 (m, 2H), 7.22-7.18 (m, 1H), 5.04 (s, 2H), 4.78 (s, 2H), 3.22 (d, *J* = 6.3 Hz, 4H), 2.88 (s, 6H), 2.71 (s, 3H), 1.20-.098 (m, 2H), 0.39-0.34 (m, 4H), 0.10-0.06 (m, 4H); MS (ESI) *m*/*z* 643 (M+1)⁺.

### Example 28

### Synthesis of [3-({((3,5-bis-trifluoromethyl-benzyl)-[5-(4-methyl-piperazin-1-yl)-pyrimidin-2-yl]-amino}-methyl)-8-methyl-quinolin-2-yl]-bis-cyclopropylmethyl-amine

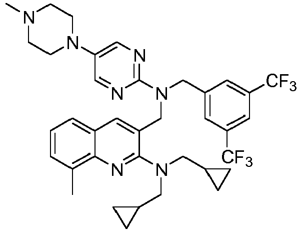

The title compound was obtained in a similar manner described in Example 25 as a colorless liquid (0.07 g, 32%).
Purity: 93.53 % (HPLC: YMC Pro C8, 20:80 [KH₂PO₄ (0.01 M, pH 3.2):CH₃CN], flow rate 1.2 mL/min, Rₜ 9.8 min); ¹H NMR (CDCl₃, 300 MHz): δ 8.19 (s, 2H), 7.74-7.67 (m, 4H), 7.41-7.39 (m, 2H), 7.21-7.17 (m, 1H), 5.04 (s, 2H), 4.78 (s, 2H), 3.21 (d, *J* = 6.6 Hz, 4H), 3.16-3.13 (m, 4H), 2.71 (s, 3H), 2.67-2.61 (m, 4H), 2.04 (s, 3H), 1.03-.099 (m, 2H), 0.38-0.33 (m, 4H), 0.10-0.06 (m, 4H). MS (ESI) *m*/*z* 698 (M+1)⁺.

### Example 29

### Synthesis of (3-{[(3,5-bis-trifluoromethyl-benzyl)-pyrimidin-2-yl-amino]-methyl}-8-methyl-quinolin-2-yl)-bis-cyclopropylmethyl-amine

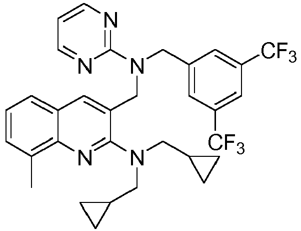

The title compound was prepared in a similar manner described in Example 26 as a colorless liquid (0.14 g, 36%).
Purity: 97.20% (HPLC: YMC Pro C8), 20:80 [KH₂PO₄ (0.01 M, pH 3.2):CH₃CN], PDA 240 nm, flow rate 1.2 mL/min, Rₜ 28.96 min)]; ¹H NMR (CDCl₃, 300 MHz): δ 8.43 (d, *J* = 4.5 Hz, 2H), 7.58-7.70 (m, 4H), 7.43-7.39 (m, 2H), 7.22-7.17 (m, 1H), 6.86 (t, *J* = 4.5 Hz, 1H), 5.10 (s, 2H), 4.82 (s, 2H), 3.22 (d, *J* = 6.3 Hz, 4H), 2.71 (s, 3H), 1.08-0.99 (m, 2H), 0.41-0.34 (m, 4H), 0.11-0.06 (m, 4H). MS (ESI) *m*/*z* 600 (M+1)⁺.

### Example 30

### Synthesis of (3-{[(3,5-bis-trifluoromethyl-benzyl)-(5-bromo-pyridin-2-yl)-amino]-methyl}-8-methyl-quinolin-2-yl)-bis-cyclopropylmethyl-amine

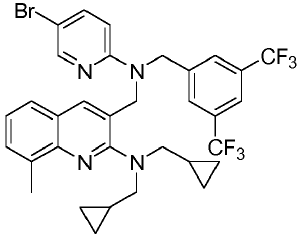

A mixture of (3-{[(3,5-bis-trifluoromethyl-benzylamino)-methyl]-8-methyl-quinolin-2-yl)-bis-cyclopropylmethyl-amine (0.3 g, 0.57 mmol), tris(dibenzylideneacetone)dipalladium (0.015 g, 0.016 mmol), 4,5-bis-diphenylphosphino-9,9-dimethylxanthine (0.03 g, 0.05 mmol), sodium tert-butoxide (0.08 g, 0.86 mmol), 2,5-dibromopyridine (0.17 gl, 0.74 mmol) in toluene was stirred at 80-100° C for 4 h. Therafter, the reaction was cooled to ambient temperature, diluted with water and extracted with ethyl acetate (2 x 30 mL). The combined organic extracts was washed with brine, dried over sodium sulfate and concentrated in *vacuo*. The crude was purified by chromatography using silica gel to afford the title compound as a colorless liquid (0.055 g, 14%).
Purity: 99.56 % (HPLC: YMC Pro C8, 20:80 [KH₂PO₄ (0.01 M, pH 3.2):CH₃CN], PDA 240 nm, flow rate 1.2 mL/min, Rₜ 52.70 min)]; ¹H NMR (CDCl₃, 300 MHz): δ 8.22 (d, *J* = 2.4 Hz, 1H), 7.77-7.32 (m, 4H), 7.46-7.40 (m, 3H), 7.26-7.23 (m, 1H), 6.34 (d, *J =* 6.9 Hz, 1H), 5.01 (s, 2H), 4.79 (s, 2H), 3.18 (d, *J* = 6.0 Hz 4H), 2.71 (s, 3H), 1.05-.099 (m, 2H), 0.41-0.36 (m, 4H), 0.10-0.07 (m, 4H). MS (ESI) *m*/*z* 678 (M+1)⁺, 679 (M+2)⁺.

### Example 31

### Synthesis of (3-{[(3,5-bis-trifluoromethyl-benzyl)-(2-methyl-2H-tetrazol-5-yl)-amino]-methyl}-6-isopropyl-5-methyl-pyridin-2-yl)-bis-cyclopropylmethyl-amine

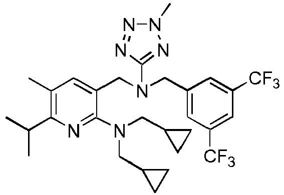

The title compound was prepared from 2-(bis-cyclopropylmethyl-amino)-6-isopropyl-5-methyl-pyridine-3-carbaldehyde by following the similar experimental procedure mentioned in the Example 8, (0.2 g, 38.0%).
Purity 96.43% (HPLC: Symmetry Shield RP8, [0.01M KH₂PO₄ (pH 6.5 with KOH): CH₃CN], 210 nm, Rₜ 9.156 min); ¹H NMR (CDCl₃, 400 MHz): δ 7.72 (s, 1H), 7.68 (s, 2H), 7.17 (s, 1H), 4.79 (s, 2H), 4.64 (s, 2H), 4.18 (s, 3H), 3.14-3.09 (m, 1H), 2.95 (d, *J* = 7.0 Hz, 4H), 2.16 (s, 3H), 1.19 (d, *J* = 6.7 Hz, 6H), 0.84 - 0.77 (m, 2H), 0.30 - 0.07 (m, 4H), -0.02-0.01 (m, 4H); CI MS: *m*/*z* 595 (M⁺, 100%); IR (neat, cm⁻¹) 2963, 1583

### Example 32

### Synthesis of (3-{[(3,5-bis-trifluoromethyl-benzyl)-(2-methyl-2H-tetrazol-5-yl)-amino]-methyl}-6-isopropyl-pyridin-2-yl)-bis-cyclopropylmethyl-amine

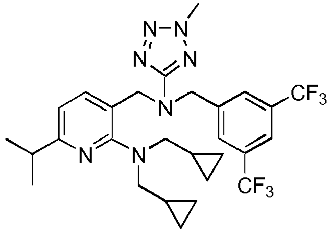

The title compound was prepared by following similar experimental procedure mentioned in the Example 31, (0.2 g, 25.0%).
Purity 98.49% (HPLC: Symmetry Shield RP8, [0.01M KH₂PO₄ (pH 6.5 with KOH): CH₃CN], 210 nm, Rₜ 8.281 min); ¹H NMR (CDCl₃, 400 MHz): δ 7.77 (s, 1H), 7.73 (s, 2H), 7.43 (d, *J* = 7.0 Hz, 1H), 6.78 (d, *J =* 7.0 Hz, 1H), 4.83 (s, 2H), 4.68 (s, 2H), 4.22 (s, 3H), 3.03 (d, *J =* 6.0 Hz, 4H), 3.00-2.94 (m, 1H), 1.28 (d, *J =* 6.8 Hz, 6H), 0.91 - 0.84 (m, 2H), 0.37 - 0.32 (m, 4H), 0.10-0.01 (m, 4H); CI MS: *m*/*z* 581 (M⁺, 100%); IR (neat, cm⁻¹) 2964, 1583.

### Example 33

### Synthesis of (3-{[(3,5-bis-trifluoromethyl-benzyl)-(2-methyl-2H-tetrazol-5-yl)-amino]-methyl}-6-ethyl-5-methyl-pyridin-2-yl)-bis-cyclopropylmethyl-amine

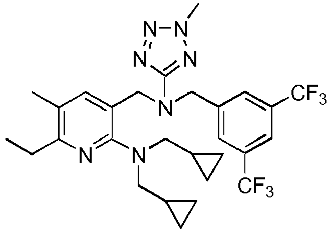

The title compound was prepared by following the similar experimental procedure mentioned in the Example 31,(0.2 g, 24.0%).
Purity 95.41% (HPLC: Symmetry Shield RP8, [0.01M KH₂PO₄ (pH 6.5 with KOH): CH₃CN], 210 nm, Rₜ 7.534 min); ¹H NMR (CDCl₃, 400 MHz): δ 7.75 (s, 1H), 7.71(s, 2H), 7.21 (S, 1H), 4.85 (s, 2H), 4.68 (s, 2H), 4.20 (s, 3H), 2.98 (d, *J* = 7.0 Hz, 4H), 2.74-2.69 (m, 2H), 2.08 (s, 3H), 1.25 (d, *J* = 7.5 Hz, 3H), 0.92 - 0.82 (m, 2H), 0.36 - 0.31 (m, 4H), 0.10-0.01 (m, 4H); CI MS: *m*/*z* 581 (M⁺, 100%); IR (neat, cm⁻¹) 2964, 1583.

### Example 34

### Synthesis of (3-{[3,5-bis-trifluoromethyl-benzyl)-(2H-tetrazol-5-yl)-amino]-methyl}-5-methyl-6-phenyl-pyridin-2-yl)-bis-cyclopropylmethyl-amine

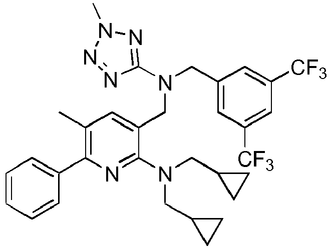

The title compound (0.24 g, 34%) was prepared by following the similar experimental procedure mentioned in the Example 31.
Purity: 97.41% (HPLC Symmetry Shield RP8 (150X4.6) {0.01M KH₂PO₄:ACN} 210 nm, RT:11.73 min; ¹H NMR (CDCl₃, 400 MHz): δ 7.73 (s,3H), 7.5 (d, *J* = 1.6Hz, 2H), 7.53-7.33 (m,4H),4.88 (s,2H), 4.71 (s,2H), 4.20 (s,3H), 2.99 (d *J* = 6.7Hz, H), 2.23 (s,3H), 0.89-0.82 (m,4H), 0.35-0.32 (m,4H) ,0.01-0.001 (m,4H); ES-MS: *m*/*z*: 630 (100%, M+1).

### Example 35

### Synthesis of {5-[[2-(bis-cyclopropylmethyl-amino)-8-methyl-quinolin-3-ylmethyl]-(3,5-bis-trifluoromethyl-benzyl)-amino]-tetrazol-2-yl}-acetic acid ethyl ester

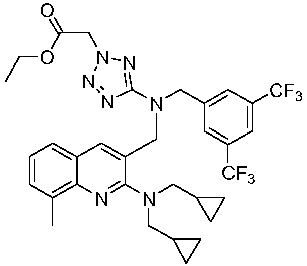

This compound (yield: 66%) was prepared following the same procedure as in Example 1 by using ethylbromoacetate instead of cyclopropanemethyl bromide.
¹H NMR (CDCl₃, 400 MHz): δ 7.84 (s, 1H), 7.69 (s, 1H), 7.67 (s, 2H), 7.44-7.41 (m, 2H), 7.23-7.21 (m, 1H), 5.25 (s, 2H), 4.93 (s, 2H), 4.66 (s, 2H), 4.30-4.25 (m, 2H), 3.19-3.17 (m, 4H), 2.70 (s, 3H), 1.30-1.21 (m, 3H), 0.88-0.85 (m, 2H), 0.39-0.34 (m, 4H), 0.09-0.05 (m, 4H); (ES-MS): *m*/*z* 676 (M⁺+1, 100%); IR (neat, cm⁻¹): 3003, 2925,1760.

### Example 36

### Synthesis of (3-{[(3,5-bis-trifluoromethyl-benzyl)-(4-chloro-[1,3,5]triazin-2-yl)-amino]-methyl}-8-methyl-quinolin-2-yl)-bis-cyclopropylmethyl-amine

### Step (i): Synthesis of N-cyanochloroformamidine

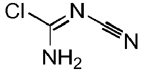

Concentrated hydrochloric acid (125 mL) was cooled to -18 °C. Sodium cyanamide (15 g, 160 mmol) was dissolved in 20 mL of water and added quickly to the hydrochloric acid. The temperature of the flask was maintained at -20 ° - 35 °C and the contents were stirred for 30 min. The flask was warmed to 40 °C and again cooled to -10 °C. The precipitate obtained (*N*-cyanochloroformamidine) was filtered, washed with water (5 mL), and dried under vacuum to give 10 g of material that was used directly for the next step without any purification (yield: 57.8 %).

### Step (ii): Synthesis of 2,4-dichloro-[1,3,5]triazine

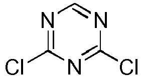

*N*-Cyanochloroformamidine (6 g, 50 mmol) was taken in dichloromethane (50 mL) and cooled to 0 °C. To this, POCl₃ (4.1 mL, 50 mmol) was added followed by DMF (50 mmol). Contents of the flask were stirred overnight from 0 °C at room temperature. Water (100 mL) was added to the flask and the compound was extracted with dichloromethane (100 mL X 3). The organic layer was dried over sodium sulfate and concentrated under vacuum to afford 4.2 g of colorless solid (yield: 48.8%).
¹H NMR (CDCl₃, 400 MHz): δ 8.2 (s, 1H).

### Step (iii): Synthesis of (3-{[(3,5-bis-trifluoromethyl-benzyl)-(4-chloro-[1,3,5]triazin-2-yl)-amino]methyl}-8-methyl-quinolin-2-yl)-bis-cyclopropylmethyl-amine

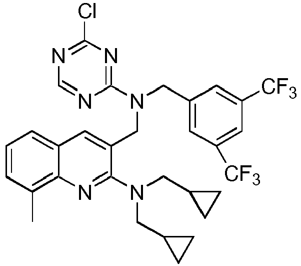

A mixture of 3-((3,5-bis(trifluoromethyl)benzylamino)methyl)-*N,N-*bis(cyclopropylmethyl)-8-methylquinolin-2-amine (Example 1 step-iii) (0.2 g, 0.38 mmol), diisopropyl ethylamine (54 µL, 0.42 mmol) was taken in DMF (20 mL) and stirred at 0 °C for 5 min. To this 2,4-dichloro-[1,3,5]triazine (84 mg, 0.57 mmol) was added to the flask at 0 °C and the contents were stirred for 30 min. The reaction mixture was taken in ethyl acetate (100 mL) and washed with water (100 mL X 3). The organic layer was separated, dried over sodium sulfate and concentrated under vacuum. The crude product was purified on column chromatography (silica gel 230-400 mesh). Elution with 2 % ethyl acetate in hexane afforded as a colorless gummy mass (yield: 67.9%).
Purity: 98.66%; ¹H NMR (CDCl₃): δ 8.53- 8.51 (m,1H), δ 7.77- 7.70 (m, 4H), 7.47-7.43 (m, 2H), 7.24 (s,1H), 5.14- 5.11 (m, 2H), 4.82- 4.77 (m, 2H), 3.20-3.19 (m, 4H), 2.71 (s, 3H), 1.03- 0.86 (m, 2H), 0.41-0.39 (m,4H), 0.11- 0.0.06 (m, 4H); ES-MS: *m*/*z* 635(M^{+.}, 100 %) IR (neat) cm⁻¹: 3386, 2925, 1566, 1498, 1421, 1383, 1354, 1278, 1174, 1135, 977, 900, 806, 763, 706, 682, 617.

### Example 37

### Synthesis of N-[2-(Bis-cyclopropylmethyl-amino)-8-methyl-quinolin-3-ylmethyl]-N-(3,5-bis-trifluoromethyl-benzyl)-N',N'-dimethyl-[1,3,5]triazine-2,4-diamine

### Step (i): Synthesis of (4-chloro-[1,3,5]triazin-2-yl)-dimethyl-amine

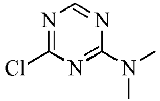

*N*-Cyanochloroformamidine (2.4 g, 20 mmol) was taken in dichloromethane (50 mL) and cooled to 0 °C, POCl₃ (3.2 mL, 40 mmol) was added followed by DMF (40 mmol). Contents of the flask were stirred overnight starting at 0 °C and then slowly warming up to room temperature. Water (100 mL) was added to the flask and the compound was extracted with dichloromethane (100 mL X 3). The organic layer was dried over sodium sulfate and concentrated under vacuum to afford 0.8 g of colorless solid (yield: 22.2%).
¹H NMR (CDCl₃, 400 MHz): δ 8.34 (s,1H), 3.22-3.90 (2s, 6H); (EI-MS) *m*/*z*: 159 (M⁺+1, 100 %).

### Step (ii): Synthesis of N-[2-(Bis-cyclopropylmethyl-amino)-8-methyl-quinolin-3-ylmethyl]-N-(3,5-bis-trifluoromethyl-benzyl)-N',N'-dimethyl-[1,3,5]triazine-2,4-diamine

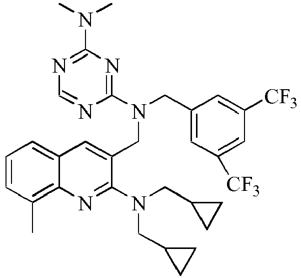

A mixture of 3-((3,5-bis(trifluoromethyl)benzylamino)methyl)-N,N-bis(cyclopropylmethyl)-8-methylquinolin-2-amine (Example 1, step-iii) (0.3 g, 0.57 mmol), diisopropyl ethylamine (81µL, 0.69 mmol) was taken in DMF (20 mL) and stirred at 0 °C for 5 min. To this (4-chloro-[1,3,5]triazin-2-yl)-dimethyl-amine (100 mg, 0.69 mmol) was then added and the contents were stirred for 30 min at 60 °C . The reaction mixture was taken in ethyl acetate (100 mL) and washed with water (100 mL X 3). The organic layer was separated, dried over sodium sulfate and concentrated under vacuum. The crude product was purified on column chromatography (silica gel 230- 400 mesh). Elution with 2 % ethyl acetate in hexane afforded 265 mg of colorless solid (yield: 71.6 %).
Purity: 99.24 %; ¹H NMR (CDCl₃): δ 8.32-8.31 (m, 1H), 7.77-7.71 (m, 4H), 7.43-7.42 (m, 2H), 7.24 (s,1H), 5.08-4.98 (m, 2H), 4.84- 4.6 7(m, 2H), 3.21- 3.09 (m, 10H), 2.71- 2.62 (m, 4H), 2.17 (s, 3H), 1.14- 0.84,(m, 2H), 0.38- 0.36 (m, 4H), 0.07-0.04 (m, 4H).
ES-MS: *m*/*z* 644 (M^{+.}+1, 100%); IR (neat) cm⁻¹: 3385, 2926,1618, 1572, 1510, 1482, 1408, 1381, 1353, 1278, 1234, 1176, 1135, 1006, 971, 898, 813, 762, 706, 682, 590.

### Example 38

### Synthesis of (3-{[(3,5-bis-trifluoromethyl-benzyl)-(4-morpholin-4-yl-[1,3,5]triazin-2-yl)-amino]-methyl}-8-methyl-quinolin-2-yl)-bis-cyclopropylmethyl-amine

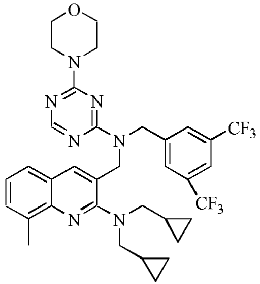

A mixture of (3-{[(3,5-bis-trifluoromethyl-benzyl)-(4-chloro-[1,3,5]triazin-2-yl)-amino]methyl}-8-methyl-quinolin-2-yl)-bis-cyclopropylmethyl-amine (Example 36) (60 mg, 0.09 mmol), diisopropyl ethylamine (13µL, 0.1 mmol) was taken in DMF (10 mL). The flask was cooled to 0 °C and the contents were stirred for 5 min. To this, morpholine (12 µL, 0.13 mmol) was added and the stirring was continued for 30 min at 0 °C and then warming up to room temperature. Contents of the flask were taken in 100 mL EtOAc and washed with water (100 mL X 3). The organic layer was separated, dried over sodium sulfate and concentrated under vacuum. The crude product was purified by column chromatography; (silica gel 230-400 mesh). Elution with 2% EtOAc in hexane afforded 58 mg of colorless mass (yield: 78%).
Purity: 97.34%; ¹H NMR (CDCl₃): δ 8.33- 8.30 (m, 1H), 7.78- 7.72 (m, 4H), 7.44-.42 (m, 2H), 7.24 (s, 1H), 5.09- 4.65 (m, 4H), 3.715 (m, 8H), 3.20-3.19 (m, 4H), 2.7 (s,3H), 1.14-0.83 (m, 2H), 0.40-0.38 (m, 4H), 0.07- 0.0 6 (m, 4H); ES-MS: *m*/*z* 686 (M^{+.}+1 ,100%); IR (neat) cm⁻¹: 3395, 2925, 2855, 1618, 1509, 1515, 1493, 1445, 1383, 1351, 1278, 1225, 1176, 1136, 1136, 1019, 991, 972, 897, 858, 812, 761, 706, 682, 582.

### Example 39

### Synthesis of (5-{[(3,5-bBis-trifluoromethyl-benzyl)-(4-N,N dimethylamino-[1,3,5]triazin-2-yl)-amino]-methyl}-1,3-dimethyl-1H-pyrazolo[3,4-b]pyridin-6-yl)-bis-cyclopropylmethyl-amine

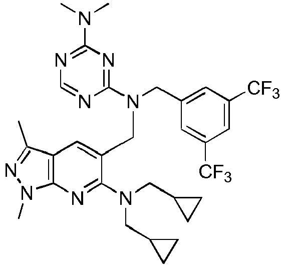

A mixture of {5-[(3,5-bis-trifluoromethyl-benzylamino)-methyl]-1,3-dimethyl-1H-pyrazolo[3,4-b]pyridin-6-yl}-bis-cyclopropylmethyl-amine (400 mg, 0.76 mmol) and diisopropylethylamine(197 mg, 1.5 mmol) in DMF (25 mL) was stirred for 5 min at 0 °C. Then (4-chloro-[1,3,5]triazin-2-yl)-dimethyl-amine (144 mg, 0.91 mmol) was added and the flask was heated for 1 h at 60 °C. The reaction mixture was taken in EtOAc (200 mL) and washed with water (200 mL X 4) and finally with brine (100 mL). The crude product was purified by column chromatography (silica gel 230- 400 mesh). Elution with 1.5% EtOAc in hexane afforded 160 mg of gummy compound (yield: 32.45%).
Purity: 93%; ¹H NMR (CDCl₃): δ 8.32- 8.29 (m,1H), 7.70- 7.62 (m,4H), 7.24 (s,1H), 5.05- 4.95 (m, 2H), 4.82-4.67 (m,2H), 43.97 (s,3H), 3.21-3.09(m, 10H), 2.4 (s,3H), 0.94-0.85 (m, 2H), 0.39-0.36 (m, 4H), 0.07-0.04 (m,4H); ES-MS: *m*/*z* 648 (M^{+.}+1, 100%).
IR (neat) cm⁻¹: 3416, 3080, 3004, 2931, 1610, 1573, 1510, 1318, 1278, 1175, 1135, 1005,813,682.

### Example 40

### Synthesis of (3-{[(3,5-bis-trifluoromethyl-benzyl)-(2-methyl-2H-tetrazol-5-yl)-amino]-methyl}-6-methyl-quinolin-4-yl)-bis-cyclopropylmethyl-amine

### Step (i): Synthesis of 2-(p-tolylamino-methylene)-malonic acid diethyl ester

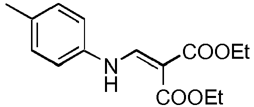

*p*-Toludine (10 g, 0.093 mol) was taken in 2-ethoxymethylene-malonic acid diethyl ester (24.2 g, 0.11 mol) and heated at 110 °C for 2 h. The reaction mixture was concentrated under reduced pressure to remove the liberated ethanol from the reaction. The residue (25.8 g, 100 %) was carried out for the next reaction without any purification.

### Step(ii): Synthesis of 4-chloro-6-methyl-quinoline-3-carboxylic acid ethyl ester

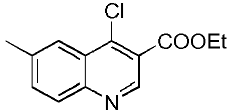

Phosphorous oxychloride (83.6 mL, 900 mmol) was added slowly to 2-(p-tolylamino-methylene)-malonic acid diethyl ester (28 g, 90 mmol) at 0 °C for 20 min and then heated at 115 °C for 5h. The excess POCl₃ was distilled off under reduced pressure the residue was neutralized with saturated sodium carbonate and extracted with ethyl acetate (500 mL X 3) and washed with water (500 mL) and sodium chloride (500 mL). The organic layer was concentrated and purified through column chromatography using 100-200 mesh silica gel; elution was with 5% ethyl acetate in petroleum ether as light yellow solid (12.0 g, yield: 51 %).
¹H NMR (CDCl₃, 200 MHz): δ 9.1 (s, 1H), 8.1(s, 1H), 8.02-7.98 (d, *J*= 8.5Hz 1H), 7.66-7.62 (d, *J*= 8.5Hz 1H), 4.54-4.44 (q,*J*= 7.2Hz 2H), 2.58 (s, 3H), 1.50- 1.42 (t, *J* = 7.1Hz, 3H); (CI-MS) *m*/*z* : 250 (M+1^{+,} 100%).

### Step(iii): Synthesis of (4-chloro-6-methyl-quinolin-3-yl)-methanol

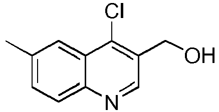

4-Chloro-6-methyl-quinoline-3-carboxylic acid ethyl ester (4 g, 16 mmol) was taken in dry THF (150 mL) under nitrogen atmosphere and DIBAL-H (20% solution in toluene) (12.5 mL, 100 mmol) was added at -78 °C over a 30 min period. The flask was stirred from -78 °C to room temperature for 48 h and then cooled back down to 0 °C. Excess DIBAL-H was quenched with saturated ammonium chloride (150 mL) solution. The precipitate was filtered and washed with ethyl acetate. The filtrate was washed with water (500 mL X 3). The organic layer was separated, dried over sodium sulfate and concentrated under vacuum. The solid obtained was washed with 1% EtOAc in hexane (150 mL X 3) to remove the unreacted ester. The resultant white solid was filtered and dried under vacuum to give 1.7 g of material (yield: 51.5%).
¹H NMR (CDCl₃, 200 MHz): δ 8.88(s, 1H), 7.99- 7.95 (m,2H), 7. 58 -7.54 (d, *J*= 8.5Hz 1H), 5.00 (s,2H), 2.57 (s, 3H); (CI-MS): *m*/*z* 207 (M^{+,} 100%).

### Step (iv): Synthesis of 4-chloro-6-methyl-quinoline-3-carbaldehyde

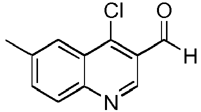

(4-Chloro-6-methyl-quinolin-3-yl)-methanol (500 mg, 2.42 mmol) was taken in dichloromethane and activated manganese (IV) oxide (6.6 g, 70 mmol) was added to it. The contents were stirred for 1 h. Manganese oxide was filtered and washed with dichloromethane (300 mL). The filtrate was concentrated under vacuum to afford 422 mg of colorless solid (yield: 85.2%).
¹H NMR (CDCl₃, 200 MHz): δ 10.70 (s, 1H), 9.20 (s, 1H) 8.16- 8.04 (m, 2H), 7.77-7.72 (d, *J*= 8.5Hz, 1H), 2.63 (s, 3H); (CI-MS): *m*/*z* 205 (M^{+,} 100%).

### Step (v) Synthesis of 4-(bis-cyclopropylmethyl-amino)-6-methyl-quinoline-3-carbaldehyde

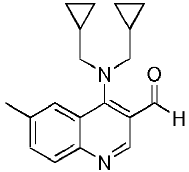

To a mixture of N bis-cyclopropyl methylamine (263 mg, 2 mmol) and potassium carbonate (484 mg, 3 mmol) was added in DMSO (25 mL), 4-Chloro-6-methyl-quinoline-3-carbaldehyde (360 mg, 1.7 mmol) was added and the contents of the flask were stirred at 130 °C for 3 h. The reaction mixture was diluted with EtOAc (200 mL) and washed with water (200 mL X 4) and finally with brine (100 mL). The organic layer was separated, dried over sodium sulfate and concentrated under vacuum. The crude product was purified by column chromatography (silica gel 230-400 mesh). Elution with 10% EtOAc in hexane afforded the compound as a yellow oil (0.325 gm, yield: 62%).
¹H NMR (CDCl₃, 400 MHz): δ 10.53 (s, 1H), 9.09 (s, 1H), 8.00- 7.98 (d, *J*= 8.5Hz, 1H), 7.86 (s,1H),7.60-7.58 (d, *J*= 8.5Hz, 1H) 3.54- 3.52(d,*J*= 6.7Hz, 4H), 2.58 (s, 3H), 1.40-1.25 (m, 2H), 0.49- 0.47 (m, 4H), 0.08- 0.06 (m, 4H); (CI-MS): *m*/*z* 295 (M+1^{+,}100%).

### Step (vi) Synthesis of {3-[(3,5-bis-trifluoromethyl-benzylamino)-methyl]-6-methyl-quinolin-4-yl}-bis-cyclopropylmethyl-amine

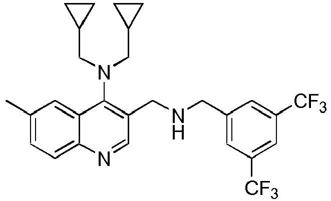

A mixture of 4-(bis-cyclopropylmethyl-amino)-6-methyl-quinoline-3-carbaldehyde (310 mg, 1 mmol), 3,5-bis-trifluoromethyl-benzylamine (334 mg, 1.3 mmol) and acetic acid (63 µL, 1 mmol) was taken in methanol (20 mL) and stirred at 0 °C for 30 min. Sodium borohydride (130 mg, 2 mmol) was added to it at room temperature portion wise over a period of 5 min. The contents were stirred at room temperature for 1 h and then taken in EtOAc (250 mL) and washed with water (200 mL X 3). Organic layer separated, dried over sodium sulfate and concentrated under vacuum. The crude product was purified by column chromatography (Silica gel 100-200 mesh). Elution with 2 % EtOAc in hexane afforded 0.490 gm (yield: 89.2 %) of gummy oil.
¹H NMR (CDCl₃, 400 MHz): δ 8.86 (s, 1H), 8.00- 7.75 (m, 6H), 7.48-7.45 (d, *J*= 8.3Hz, 1H) 4.08- 4.01 (m, 4H),3.24- 3.20 (m, 4H), 2.53 (s, 3H), 0.96-0.93 (m, 2H), 0.37- 0.35 (m, 4H), 0.07- 0.01 (m, 4H); (CI-MS): *m*/*z* 522 (M+1^{+,}100%).

### Step(vii) Synthesis of [4-(bis-cyclopropylmethyl-amino)-6-methyl-quinolin-3-ylmethyl]-(3,5-bis-trifluoromethyl-benzyl)-cyanamide

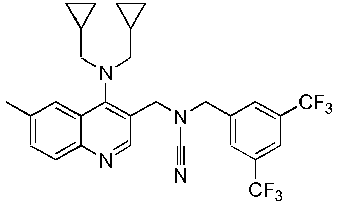

A mixture of {3-[(3,5-bis-trifluoromethyl-benzylamino)-methyl]-6-methyl-quinolin-4-yl}-bis-cyclopropylmethyl-amine (490 mg, 0.94 mmol) and sodium hydrogen carbonate (159 mg, 1.87 mmol) in methanol (10 mL) was stirred at room temperature for 5 min. To this, cyanogen bromide (149 mg, 1.40 mmol) was added and the contents were stirred at room temperature for 30 min. Reaction mixture was diluted with EtOAc (200 mL) and washed with water (200 mL X 3). Organic layer separated, dried over sodium sulfate and concentrated under vacuum. The crude product was purified with column chromatography (silica gel 230-400 mesh). Elution with 0.5% methanol in dichloromethane afforded 0.460 gm of oily compound (yield: 89.6%).
¹H NMR (CDCl₃, 400 MHz): δ 8.86 (s, 1H), 8.00- 7.75 (m, 6H), 7.53-7.52 (d,*J*= 6.7Hz 1H) 4.08- 4.01 (m, 4H), 3.21-3.19 (m,*J*= 6.7Hz, 4H), 2.53 (s, 3H), 0.96-0.93 (m, 2H), 0.37- 0.35 (m, 4H), 0.07- 0.01 (m, 4H); (CI-MS): *m*/*z* 547 (M+1^{+.},100%).

### Step (viii): Synthesis of (3-{[(3,5-bis-trifluoromethyl-benzyl)-(2H-tetrazol-5-yl)-amino]-methyl}-6-methyl-quinolin-4-yl)-bis-cyclopropylmethyl-amine

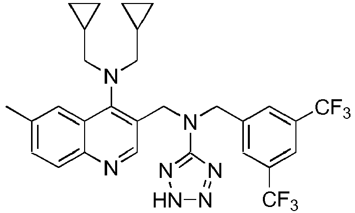

A mixture of [4-(bis-cyclopropylmethyl-amino)-6-methyl-quinolin-3-ylmethyl]-(3,5-bis-trifluoromethyl-benzyl)-cyanamide (450 mg, 0.824 mmol), zinc bromide (222 mg, 0.986 mmol) and sodium azide (74 mg, 1.138 mmol) in water (50 mL) was heated under reflux for 5 h. The flask was cooled to room temperature and 5 % hydrochloric acid was added to it. The reaction mixture was extracted with EtOAc (100 mL X 3). The organic layer was separated, dried over sodium sulfate and concentrated under vacuum to afford 700 mg of the crude compound and it was directly used for the next step without any purification.

### Scheme (ix) Synthesis of (3-{[(3,5-bis-trifluoromethyl-benzyl)-(2-methyl-2H-tetrazol-5-yl)-amino]-methyl}-6-methyl-quinolin-4-yl)-bis-cyclopropylmethyl-amine

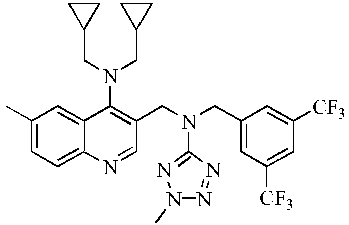

A mixture of (3-{[(3,5-bBis-trifluoromethyl-benzyl)-(2H-tetrazol-5-yl)-amino]-methyl}-6-methyl-quinolin-4-yl)-bis-cyclopropylmethyl-amine (700 mg, 1.18 mmol) and sodium hydride (60 %) (61 mg, 1.5 mmol) in DMF (25 mL) was stirred at 0 °C for 5 min. To this, methyl iodide (337 g, 2 mmol) was then added and stirring was continued for another 30 min. The reaction mixture was diluted with EtOAc (200 mL) and washed with water (200 mL X 4) and finally with brine (100 mL). The organic layer was separated, dried over sodium sulphate and concentrated under vacuum. The crude product was purified by column chromatography (silica gel 230-400 mesh). Elution with 1.5 % EtOAc in hexane afforded 0.205 gm of gummy mass (yield: 28.63%).
Purity: 95%; ¹H NMR (CDCl₃): δ 8.61 (s, 1H), 7.97-7.95 (d, *J*= 8.3Hz 1H), 7.78-767 (m, 4H), 7.48-7.46 (d, *J*= 6.7Hz, 1H), 5.10 (s, 2H), 4.79 (s, 2H), 4.18 s,3H), 3.18-3.16 (d, *J*= 6.7Hz,4H) 2.53 (s, 3H), 0.81- 0.77 (m, 2H), 0.31-0.29 (m, 4H), -0.008- - 0.02 (m, 4H).
ES-MS: *m*/*z* 604 (M^{+.}+1, 100%); IR (neat) cm⁻¹: 3004, 1575, 1505, 1508, 1426, 1379, 1279, 1173, 1134, 1019, 903, 827, 754, 706, 682.

### Example 41

### Synthesis of (3-{[(3,5-bis-trifluoromethyl-benzyl)-(2-methyl-2H-tetrazol-5-yl)-amino]-methyl}-quinolin-4-yl)-bis-cyclopropylmethyl-amine

### Step (i): Synthesis of 2-phenylaminomethylene-malonic acid diethyl ester

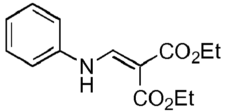

A mixture of aniline (10 g, 107 mmol), 2-ethoxymethylene-malonic acid diethyl ester (27.8 g, 128 mmol) was heated at 110 °C for 2 h. The reaction mixture was concentrated under reduced pressure to remove the liberated ethanol from the reaction. The residue (28.2 g) was carried out for the next reaction without any purification.

### Step (ii): Synthesis of 4-chloro-quinoline-3-carboxylic acid ethyl ester

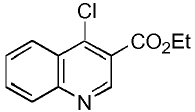

Phosphorous oxychloride (98.6 mL, 1.06 mol) was added slowly to 2-phenylaminomethylene-malonic acid diethyl ester (28 g, 106 mmol) at 0 °C for 20 min and then heated at 115 °C for 5h. The excess POCl₃ was distilled off under reduced pressure and the residue was neutralized with saturated sodium carbonate and extracted with ethyl acetate (500 mL X 3) and washed with water (500 mL) and sodium chloride (500 mL). The organic layer was concentrated and purified through column chromatography using 100-200 mesh silica gel, elution with 5% ethyl acetate in petroleum ether afforded 12.5 g of product (yield: 50%) as a light yellow solid.
ES-MS: *m*/*z* 236 (M^{+.}+1,100%).

### Step (iii): Synthesis of 4-(Bis-cyclopropylmethyl-amino)-quinoline-3-carboxylic acid ethyl ester

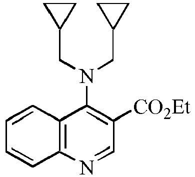

A mixture of *4c*-quinoline-3-carboxylic acid ethyl ester (5 g, 20 mmol) and bis-cyclopropylmethyl-amine (3.98 g, 30 mmol) were taken in isopropanol (50 mL) and the reaction mixture was refluxed for 10 h. Isopropanol was removed under reduced pressure then the crude was washed with petroleum ether and dried to afford 5.4 g (yield: 79%) of yellow colored gummy mass.
ES-MS: *m*/*z* 325 (M⁺+1, 100 %).

### Step (iv): Synthesis of [4-(bis-cyclopropylmethyl-amino)-quinolin-3-yl]-methanol

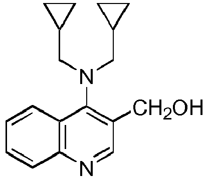

4-(Bis-cyclopropylmethyl-amino)-quinoline-3-carboxylic acid ethyl ester (1 g, 3.0 mmol) was taken in DCM at -78 °C under nitrogen atmosphere and DIBAL-H (8.76 mL (20 % solution in toluene), 12 mmol) was added at the same temperature over a period of 20 min. The reaction mixture was continued to stir at room temperature for 48 h. It was again cooled to -78 °C and saturated potassium sodium tartrate solution (15 mL) was added slowly for 20 mins and extracted with ethyl acetate (100 mL X 3) and washed with water (100 mL). The organic layer was dried over sodium sulfate, concentrated under reduced pressure and purified through column chromatography using 100-200 mesh silica gel. Elution with 1 % methanol in dichloromethane afforded 0.450 gm (yield: 45%) of yellow colored gummy mass.
¹H NMR (CDCl₃) δ: 8.89 (s, 1H), 8.13-8.10 (d, *J* = 9.134, 1H), 8.07-8.04 (d, , *J* = 9.40, 1H) 7.67 (dd, *J*=1.48, 1.34 Hz, 1H), 7.49 (dd, *J*=1.6, 1.4 Hz, 1H), 5.00 (s, 2H), 3.32-3.31 (d, *J*= 6.99, 4H), 1.03-0.96 (m, 2H), 0.45-0.40 (m, 4H), 0.06-0.02 (m, 4H); ES-MS *m*/*z*: 283 (M⁺+1, 100%).

### Step (v): Synthesis of 4-(bis-cyclopropylmethyl-amino)-quinoline-3-carbaldehyde

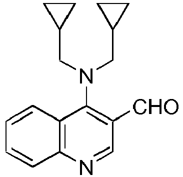

A mixture of [4-(bis-cyclopropylmethyl-amino)-quinolin-3-yl]-methanol (450 mg, 1.5 mmol) and MnO₂ (1.3 g, 15 mmol) in methylene chloride (15 mL) was stirred at room temperature for 30 min. The reaction mixture was filtered through celite with methylene chloride. The filtrate was concentrated under reduced pressure to afford 400 mg (yield: 90%) of yellow colored gummy mass.
(CI-MS): *m*/*z*: 281 (M⁺+1, 100 %).

### Step (vi): Synthesis of {3-[(3,5-bis-trifluoromethyl-benzylamino)-methyl]-quinolin-4-yl} -bis-cyclopropylmethyl-amine

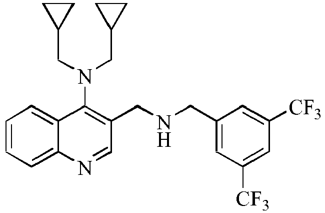

A mixture of 4-(bis-cyclopropylmethyl-amino)-quinoline-3-carbaldehyde (400 mg, 1.4 mmol), 3,5-bis-trifluoromethyl-benzylamine (380 mg, 1.5 mmol) in methanol (20 mL) was added acetic acid (2 drops). The contents were stirred at room temperature for 30 min and sodium cyano borohydride (176 mg, 2.8 mmol) was added to it at 0 °C over a period of 5 min. The flask was warmed to room temperature and continued to stir for 1 h. Methanol was removed under vacuum and water (15 mL) added to the residue. The crude product was extracted with ethyl acetate (100 mL X 3); the organic layer was washed with water (100 mL X 3) and dried over sodium sulfate. Concentration under vacuum afforded 0.468 gm (yield: 65%) of yellow colored gummy mass.
¹H NMR (CDCl₃) δ: 8.93 (s, 1H), 8.11-8.06 (m, 2H), 7.87-7.75 (m, 3H), 7.63 (t, *J*=4.2 Hz, 1H), 7.48 (t, *J*=4.2 Hz), 1H), 4.09 (s, 2H), 4.02 (s, 2H), 3.26-3.24 (m, 4H), 0.95-0.86 (m, 2H), 0.41-0.34 (m, 4H), 0.06-0.02 (m, 4H); ES-MS: m/z 508 (M⁺+1, 100 %).

### Step (vii): Synthesis of [4-(bis-cyclopropylmethyl-amino)-quinolin-3-ylmethyl]-(3,5-bis-trifluoromethyl-benzyl)-cyanamide

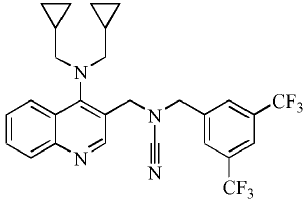

A mixture of {3-[(3,5-bis-trifluoromethyl-benzylamino)-methyl]-quinolin-4-yl}-bis-cyclopropylmethyl-amine (460 mg, 0.9 mmol) and sodium hydrogen carbonate (151 mg, 1.8 mmol) in methanol (20 mL) was stirred at 0 °C for 30 min then cyanogen bromide (190 mg, 1.8 mmol) was added and the resulting reaction mixture was stirred at room temperature for 1 h. Methanol was removed under vacuum and water (50 mL) was added to the reaction mixture; the crude product was extracted with ethyl acetate (100 mL X 3) and washed with water (100 mL X 3) then the combined organic layer was dried over sodium sulfate and concentrated to afford 0.360 gm (yield: 7 %) of light yellow solid.
¹H NMR (CDCl₃) δ: 8.91 (s, 1H), 8.13-8.11 (d, *J*= 8.33, 1H), 8.05-8.03 (d, 1H, *J* = 9.14), 7.90 (s, 1H), 7.81 (s, 2H), 7.70 (t, 1H), 7.52 (t, 12H), 4.62 (s, 2H), 4.39 (s, 2H), 3.23-3.21 (d, *J*=6.71 Hz, 4H), 0.88-0.81 (m, 2H), 0.39-0.37 (m, 4H), 0.08-0.04 (m, 4H); ES-MS: *m*/*z* 533 (M⁺+1, 100%).

### Step (viii): Synthesis of (3-{[(3,5-bis-trifluoromethyl-benzyl)-(2H-tetrazol-5-yl)-amino]-methyl}-quinolin-4-yl)-bis-cyclopropylmethyl-amine

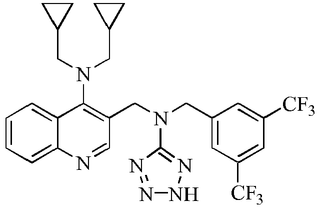

A mixture of [4-(bis-cyclopropylmethyl-amino)-quinolin-3-ylmethyl]-(3,5-bis-trifluoromethyl-benzyl)-cyanamide (350 mg, 0.65 mmol), sodium azide (51 mg, 0.78 mmol) and zinc bromide (175 mg, 0.78 mmol) were taken in water (15 mL) and refluxed for 1 h. 5% Hydrochloric acid was added and the crude product was extracted with ethyl acetate (100 mL X 3) and washed with water (75 mL); the organic layer was dried over sodium sulfate and concentrated to afford 0.300 gm (yield: 80 %) of yellow colored gummy mass. This was directly carried for the next reaction.

### Step (ix): Synthesis of (3-{[(3,5-bis-trifluoromethyl-benzyl)-(2-methyl-2H-tetrazol-5-yl)-amino]-methyl}-quinolin-4-yl)-bis-cyclopropylmethyl-amine

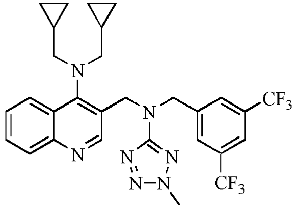

A mixture of (3-{[(3,5-bis-trifluoromethyl-benzyl)-(2H-tetrazol-5-yl)-amino]-methyl}-quinolin-4-yl)-bis-cyclopropylmethyl-amine (300 mg, 0.52 mmol) and sodium hydride (31 mg, 0.78 mmol) were taken in DMF (10 mL) at 0 °C and stirred for 20 min, then methyl iodide (0.067 mL, 1.04 mmol) was added at that temperature and stirred for 1 h. Water (20 mL) was added to the reaction mixture and extracted with ethyl acetate (100 mL X 3) and the organic layer was washed with water (75 mL). Drying over sodium sulfate and concentration under vacuum yielded the crude product that was purified by column chromatography using 230-400 mesh silica gel eluting with 5% ethyl acetate in petroleum ether to afford 0.107 gm (yield: 35%) of yellow colored gummy mass.
Purity: 94%; ¹H NMR (CDCl₃): δ 8.68 (s,1H), 8.09-8.04 (m,2H), 7.75-7.47 (m, 5H), 5.09 (s, 2H) 4.80 (s,2H), 4.18 (s,3H), 3.20-3.18 (d, *J*=6.7 Hz, 4H), 0.91- 0.76 (m,2H), 0.31-0.30 (m,4H), -0.008- -0.05 (m, 4H); ES-MS: *m*/*z* 590 (M^{+.}+1,100%); IR (neat) cm⁻¹: 3385, 2926, 1567, 1500, 1380, 1279, 1174, 1134, 1020, 903, 843, 766, 706, 682.

### Comparative Example 42

### Synthesis of (6-{[(3,5-bis-trifluoromethyl-benzyl)-(2-methyl-2H-tetrazol-5-yl)-amino]-methyl}-2,3-dimethyl-3H-imidazo[4,5-b]pyridin-5-yl)-bis-cyclopropylmethyl-amine

### Step (i): Synthesis of N-(2,3-dimethyl-3H-imidazol-4-yl)-acetamide

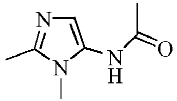

5% Palladium on carbon (7.5 g) was taken in to 500 mL round bottom flask and to that 1,4-dioxane (450 mL) was added. 1,2-dimethyl-5-nitro-1H-imidazole (15.0 g, 106.2 mmol.) was added to it and stirred gently at room temperature under hydrogen atmosphere for 12 h. Acetic anhydride (30.1 mL, 318.6 mmol.) was added very slowly and continued to stir at room temperature for 5 h; then the reaction mixture was then passed through celite and washed with dichloromethane (500 mL) and the filtrate was concentrated under vacuum to yield 15.0 g (yield: 92%) of dark yellow oily liquid.
¹H NMR (CDCl₃): δ 8.57 (bs, 1H), 6.65 (s,1H), 3.39-3.34 (s, 3H), 2.37-2.31 (s, 3H), 2.28-2.24 (s, 3H); (CI-MS): *m*/*z* 153 (M+100%), 110.

### Step (ii): Synthesis of 5-chloro-2,3-dimethyl-3H-imidazo[4,5-b]pyridine-6-carbaldehyde

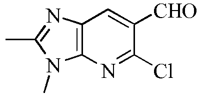

*N*-(2,3-Dimethyl-3H-imidazol-4-yl)-acetamide (15.0 g, 988.0 mmol) was taken in POCl₃ (54.8 mL, 588 mmol.) at 0 °C and stirred for 3 h at 90 °C then DMF (22.72 mL, 294 mmol.) was added to it slowly at the same temperature over a period of 30 min. the reaction was continued to stir at the same temperature for 1.5 h. The excess POCl₃ was distilled off under reduced pressure then the residue was neutralized with saturated solution of sodium carbonate and extracted with ethyl acetate (600 mL X 3) and washed with water (300 mL) and the organic layer was dried over sodium sulfate and concentrated under vacuum. The crude compound was purified through column chromatography using 100-200 mesh silica gel. Elution was 1% methanol in dichloromethane to yield 5.25 g (yield: 25.6%) of colorless solid.
¹H NMR (CDCl₃): δ 10.5 (s, 1H), 8.46 (s,1H), 3.83 (s, 3H), 2.67 (s, 3H); (CI-MS): *m*/*z* 210 (M+1^{+.}, 100%).

### Step (iii): Synthesis of 5-chloro-2,3-dimethyl-3H-imidazo[4,5-b]pyridine-6-carbaldehyde oxime

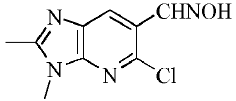

To a mixture of 5-chloro-2,3-dimethyl-3H-imidazo[4,5-b]pyridine-6-carbaldehyde (4.2 g, 20 mmol.) and hydroxylamine hydrochloride (2.8 g, 40 mmol.) in methanol was added triethylamine (5.415 mL, 40 mmol.) at 0 °C then the reaction mixture was stirred at room temperature for 12 h. Methanol was distilled off under reduced pressure and then the residue was taken in ice cold water (500 mL) and stirred for 15 min. The resulting solid was filtered out and washed with water (500 mL) and then dried under vacuum to yield 3.0 g (yield: 66.9%) of color less solid.
(CI-MS): *m*/*z* 225 (M+1^{+.}, 100 %),207,178,129.

### Step (iv): Synthesis of 5-chloro-2, 3-dimethyl-3H-imidazo[4,5-b]pyridine-6-carbonitrile

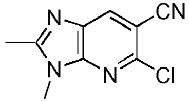

5-Chloro-2 ,3-dimethyl-3H-imidazo[4,5-b]pyridine-6-carbaldehyde oxime (2.2 g, 9.8 mmol.) was taken in acetic anhydride (40 mL, excess) and refluxed for 14 h in nitrogen atmosphere. The reaction mixture was then taken in water (250 mL) and extracted with ethyl acetate (100 mL X 3), washed with saturated sodium bicarbonate solution (100 mL) followed by water (250 mL) and concentrated under vacuum. Upon purification by column chromatography using 230-400 mesh silica gel using 2 % acetone in dichloromethane yielded 1.1 g (yield: 54.4 %) of pure colorless solid. ¹H NMR (CDCl₃, 200 MHz): δ 8.67 (s, 1H), 3.75 (s, 3H), 2.66 (s, 3H); (CI-MS): *m*/*z* 207 (M^{+.}1, 100%).

### Step (v): Synthesis of 5-(bis-cyclopropylmethyl-amino)-2,3-dimethyl-3H-imidazo[4,5-b]pyridine-6-carbonitrile

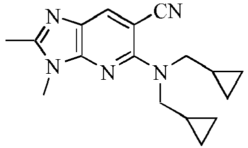

A mixture of 5-chloro-2,3-dimethyl-3H-imidazo[4,5-b]pyridine-6-carbonitrile (1.0 g, 4 mmol.), bis-cyclopropylmethyl-amine (1.213 g, 9 mmol.) and potassium carbonate (1.339 g, 9 mmol.) were taken in DMF (20 mL) in a 80 mL microwave vessel and subjected to microwave irradiation at 250 W, 120 °C for 60 min. Then the reaction mixture was taken in to ethyl acetate (250 mL) and washed with water (250 mL X 3), the organic layer dried over anhydrous sodium sulfate and concentrated under vacuum. The crude compound was purified by column chromatography using 230-400 mesh silica gel. Elution with 1% isopropanol in dichloromethane yielded 0.350 gm (yield: 24.4%) of colorless solid.
¹H NMR (CDCl₃, 200 MHz): δ 7.97 -7.96 (s, 1H), 3.68-3.67 (s, 1H), 3.62-3.61 (d, *J*=6.7 Hz 4H) 2.56-2.54 (s, 3H), 0.87-0.81 (m, 2H) 0.57-0.47 (m, 4H), 0.29-0.22 (m, 4H); (CI-MS): *m*/*z*295 (M^{+.}1, 100 %).

### Step (vi): Synthesis of 5-(bis-cyclopropylmethyl-amino)-2,3-dimethyl-3H-imidazo[4,5-b]pyridine-6-carbaldehyde

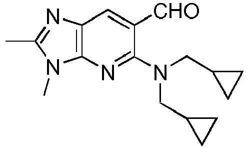

5-(Bis-cyclopropylmethyl-amino)-2,3-dimethyl-3H-imidazo[4,5-b]pyridine-6-carbonitrile (0.220 g, 0.7 mmol.) was taken in dichloromethane at -75 °C and DIBAL-H (20% solution in toluene) (0.630 mL, 0.8 mmol.) was added dropwise over a period of 20 min and then stirred at room temperature for 12 h. The reaction mixture was quenched with saturated solution of potassium sodium tartrate at 0 °C and the crude product was extracted with ethyl acetate (250 mL X 3), washed with water (250 mL X 3) and the combined organic layer was dried over anhydrous sodium sulfate and concentrated under reduced pressure to yield 0.220 gm of yellow oily liquid.
(CI-MS) *m*/*z*: 299 (M^{+.}1, 100 %).

### Step (vii): Synthesis of {6-[(3,5-bis-trifluoromethyl-benzylamino)-methyl]-2,3-dimethyl-3H-imidazo[4,5-b]pyridin-5-yl}-bis-cyclopropylmethyl-amine

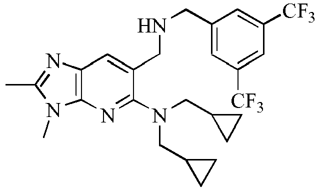

To a mixture of 5-(bis-cyclopropylmethyl-amino)-2,3-dimethyl-3H-imidazo[4,5-b]pyridine-6-carbaldehyde (0.220 g, 0.73 mmol.) and 3, 5-bis-trifluoromethyl-benzylamine (215 mg, 0.87 mmol) in methanol (8 mL) was added acetic acid (0.042 mL, 0.073 mol) at 0 °C and the resulting reaction mixture was stirred for 15 min at the same temperature. NaCNBH₃ (0.069 g, 0.011 mmol.) was added and the mixture was stirred at room temperature for 45 min. Methanol was removed under reduced pressure and the crude was extracted with ethyl acetate (250 mL) and washed with water (250 mL X 2). The organic layer was dried over anhydrous sodium sulfate and concentrated under reduced pressure to yield 0.220 g of colorless gummy compound.

### Step (viii): Synthesis of [5-(Bis-cyclopropylmethyl-amino)-2,3-dimethyl-3H-imidazo[4,5-b]pyridin-6-ylmethyl]-(3,5-bis-trifluoromethyl-benzyl)-cyanamide

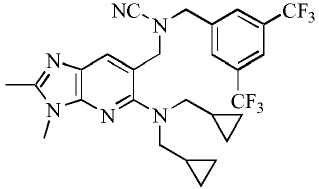

A mixture of {6-[(3,5-bis-trifluoromethyl-benzylamino)-methyl]-2,3-dimethyl-3H-imidazo[4,5-b]pyridin-5-yl}-bis-cyclopropylmethyl-amine (0.220 g, 0.41 mol) and sodium bicarbonate (0.071 mg, 0.83 mol.) in methanol (8 mL) was stirred at 0 °C for 15 min. Cyanogen bromide (0.053 g, 0.05 mol) was added and continued to stir at room temperature for 1.5 h. Methanol was removed under reduced pressure, extracted with ethyl acetate (250 mL), washed with water (250 mL X 2) and the organic layer was dried over anhydrous sodium sulfate. Concentration under reduced pressure afforded 0.220 g of colorless gummy compound.

### Step (ix): Synthesis of (6-{[(3,5-bis-trifluoromethyl-benzyl)-(2H-tetrazol-5-yl)-amino]-methyl}-2,3-dimethyl-3H-imidazo[4,5-b]pyridin-5-yl)-bis-cyclopropylmethyl-amine

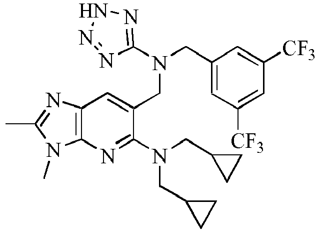

A mixture of [5-(bis-cyclopropylmethyl-amino)-2,3-dimethyl-3H-imidazo[4,5-b]pyridin-6-ylmethyl]-(3,5-bis-trifluoromethyl-benzyl)-cyanamide (0.220 g, 0.04 mmol), sodium azide (0.130 g, 2 mmol.) and ammonium chloride (0.106 g, 2 mmol) were taken in DMF (15 mL) and heated at 90 °C for 2 h. The reaction mixture was taken in ethyl acetate (250 mL) and washed with water (250 mL X 4) and the organic layer was dried over anhydrous sodium sulfate and concentrated under reduced pressure to yield 0.220 g of colorless gummy liquid.

### Step (x): Synthesis of (6-{[(3,5-bis-trifluoromethyl-benzyl)-(2-methyl-2H-tetrazol-5-yl)-amino] -methyl} - 2,3 -dimethyl-3H-imidazo[4,5-b]pyridin-5-yl)-bis-cyclopropylmethyl-amine

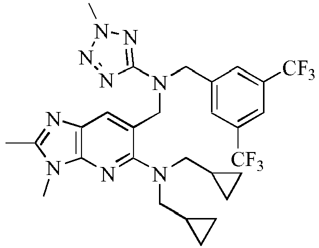

To a solution of (6-{[(3,5-bis-trifluoromethyl-benzyl)-(2H-tetrazol-5-yl)-amino]-methyl}-2,3-dimethyl-3H-imidazo[4,5-b]pyridin-5-yl)-bis-cyclopropylmethyl-amine (0.220 g, 3.7 mmol.) in DMF (8 mL) was added 60 % sodium hydride (0.017 g, 0.741 mmol.) at 0 °C and the resulting reaction mixture was stirred at the same temperature for 15 min. and then methyl iodide (0.046 mL, 0.0741 mmol.) was added and continued to stir for 30 min. The crude product was extracted with ethyl acetate (250 mL), washed with water (250 mL X 2) and the organic layer was dried over anhydrous sodium sulfate and concentrated under reduced pressure. Purification by column chromatography using 230-400 mesh silica gel eluting with 1:1 hexane: ethyl acetate yielded 0.010 gm (yield: 4.4%) of pure colorless gummy compound.
Purity: 90.28%; ¹H NMR (CDCl₃): δ 7.76-7.65 (m, 4H), 4.94 (s,2H), 4.64 (s, 2H), 4.8 (s, 3H), 3.73 (s, 3H), 3.01(d, *J*=6 Hz, 4H), 2.57 (s, 3H), 0.92-0.81 (m, 2H), 0.33-0.29 (m, 4H), 0.00- -0.09 (m, 4H); ES-MS *m*/*z*: 608 (M+1^{+.}, 100%); IR (neat) cm⁻¹: 3358, 2925, 1688, 1578, 1484, 1382, 1279, 1174, 1135, 1049, 902, 752, 706, 682.

### Comparative Example 43

### Synthesis of 3-{[(3,5-Bis-trifluormethyl-benzyl)-(2-methyl-2H-tetrazol-5-yl)-amino]-methyl}-1H-indole-2-carboxylic acid ethyl ester

### Step (i): Synthesis of 1H-indole-2-carboxylic acid ethyl ester:

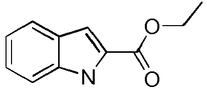

1H-indole-2-carboxylic acid (1 g, 6.2 mmol) was dissolved in 20 mL of ethanol. To this thionyl chloride (5.86 g, 49.68 mmol) was added drop wise with stirring at 0° C and stirring was continued for 30 h at room temperature. Excess of SOCl₂ was evaporated, water was added to the residue and the suspension was filtered to afford the title compound (1.0 g, yield: 85.5%).
¹H NMR (CDCl₃) □ 8.85 (br s, 1H), 7.71-7.67 (m, 1H), 7.44-7.28 (m, 2H), 7.23-7.12 (m, 2H), 4.41 (q, *J*= 7.2 Hz, 2H), 1.42 (t, *J* = 7.0 Hz, 3H); *m*/*z* (CI-MS): 190 (M⁺+1, 100%).

### Step (ii): Synthesis of 3-formyl-1H-indole-2-carboxylic acid ethyl ester:

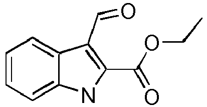

A solution of *N*-methyl formanilide (1.18 g, 8.73 mmol) and POCl₃ (1.26 g, 8.2 mmol) was stirred at room temperature for 0.5 h under nitrogen atmosphere. Dry 1,2-dichloroethane (10 mL) and 1H-indole-2-carboxylic acid ethyl ester (1 g, 5.29 mmol) were added, and the suspension was stirred at 80°C for 6 h. The reaction mixture was poured into a 50% aqueous solution of NaOAc (50 mL) and filtered the suspension to afford the title compound (0.890 g, yield: 77.4%).
¹H NMR (CDCl₃)□□ 10.76 (s, 1H), 9.31 (br s, 1H), 8.50-8.47 (m, 1H), 7.45-7.32 (m, 3H), 4.53 (q, *J =* 7.2 Hz, 2H), 1.48 (t, *J =* 7.0 Hz, 3H); (CI-MS): *m*/*z* 218 (M⁺+1, 100%).

### Step (iii): Synthesis of 3-[(3,5-bis-trifluoromethyl-benzylamino)-methyl]-1H-indole-2-carboxylic acid ethyl ester

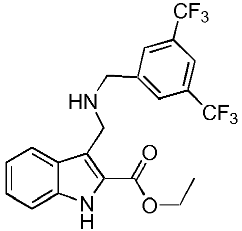

3-Formyl-1H-indole-2-carboxylic acid ethyl ester (2 g, 9.21 mmol), obtained in step (ii), 3,5-bis-trifluoromethylbenzylamine (2.23 g, 9.21 mmol) and acetic acid (1.66 g, 18.43 mmol) were put in a 50 mL RB flask. To this was added 10 mL of methanol and stirred at RT for 15 min. Sodium cyanoborohydride (2.61 g, 27.64 mmol) was added portionwise and stirring was continued at RT for another 1 h. Methanol was removed from the reaction mixture under vacuum, water was added to this crude and was extracted with ethyl acetate (3 x 50 mL). The organic layer was washed with saturated NaHCO₃ solution, brine and dried over sodium sulphate and then evaporated the solvent to get the required compound (3.8 g, yield: 93%).
(CI-MS): *m*/*z* 445 (M⁺+1, 90%).

### Step (iv): Synthesis of 3-{[(3,5-bis-trifluoromethyl-benzyl)-cyano-amino]-methyl}-1H-indole-2-carboxylic acid ethyl ester

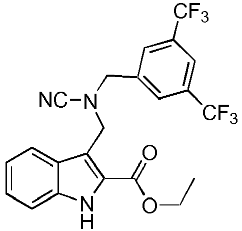

To a solution of 3-[(3,5-bis-trifluoromethyl-benzylamino)-methyl]-1H-indole-2-carboxylic acid ethyl ester (0.1 g, 0.22 mmol), obtained in step (iii), in MeOH (10 mL) under N₂ atmosphere was added sodium bicarbonate (0.38 g, 0.45 mmol) followed by the addition of cyanogen bromide (0.028 g, 0.26 mmol). The reaction mixture was stirred at RT for 4 h. The solvent was removed under vacuum to get the crude material. The residue was dissolved in water and was extracted with ethyl acetate and dried over sodium sulphate. The solvent was evaporated and concentrated in *vacuo* which was purified by column chromatography over 100-200 mesh silica gel using 20% ethyl acetate and petroleum ether to afford 3-{[(3,5-bis-trifluoromethyl-benzyl)-cyano-amino]-methyl}-1H-indole-2-carboxylic acid ethyl ester (0.08 g, yield: 80%).
¹H NMR (CDCl₃, 400 MHz): δ 8.89 (br s, 1H), 7.76-7.73 (m, 2H), 7.60 (s, 2H), 7.39-7.37 (m, 2H) ,7.25-7.22 (m, 1H), 4.88 (s, 2H), 4.43-4.37 (m, 2H), 4.30 (s, 2H), 1.43-1.39 (m, 3H); *m*/*z* (CI-MS) 202 (M⁺-268, 90%), 269(M⁺-202, 40%), 469 (M⁺,10%).

### Step (v): Synthesis of 3-{[(3,5-bis-trifluoromethyl-benzyl)-(1H-tetrazol-5-yl)-amino]-methyl}-1H-indole-2-carboxylic acid ethyl ester

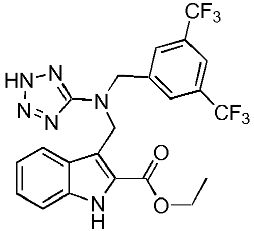

3-{[(3,5-Bis-trifluoromethyl-benzyl)-cyano-amino]-methyl}-1H-indole-2-carboxylic acid ethyl ester (0.5 g, 1.06mmol), obtained in step (iv), sodium azide (0.381 g, 5.33mmol) and ammonium chloride (0.316 g, 5.33 mmol) were taken in a 25 mL RB flask with dry DMF (5 mL). The reaction was refluxed under nitrogen atmosphere for 1 h. The reaction mixture was cooled to room temperature and the aqueous solution was extracted with ethyl acetate (3 x 10 mL) and the combined organic layer was washed with brine. The solvent was dried over sodium sulphate and concentrated under vacuum to afford the title compound (0.3 g, yield: 56%).
¹H NMR (CDCl₃, 400 MHz):δ 8.89 (br s, 1H), 7.70 (s, 1H), 7.61 (s, 2H), 7.51-7.49 (m, 1H), 7.42-7.35 (m, 2H), 7.17-7.13 (m, 1H), 5.08 (s, 2H), 5.05 (s, 2H), 4.32 (q, *J* = 7.2 Hz, 2H), 4.23 (s, 3H), 1.29 (t, *J* = 7.0 Hz, 3H); (CI-MS): *m*/*z* 526 (M⁺, 100%). **Step (vi):** Synthesis of 3-{[(3,5-bis-trifluoromethyl-benzyl)-(2-methyl-1H-tetrazol-5-yl)-amino]-methyl}-1H-indole-2-carboxylic acid ethyl ester

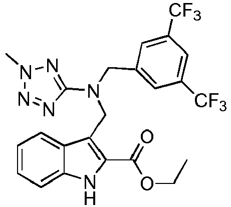

To a suspension of 3-{[(3,5-bis-trifluoromethyl-benzyl)-(1H-tetrazol-5-yl)-amino]-methyl}-1H-indole-2-carboxylic acid ethyl (0.19 g, 0.37 mmol) in water (4 mL) was added sodium hydroxide (0.03 g, 0.742 mmol) and stirred for 15 min at RT followed by the addition of dichloromethane (4 mL). To this reaction mixture was added dimethyl sulphate (0.051 g, 0.241 mmol) and tetra-butylammonium bromide (0.006 g, 0.018 mmol). The reaction was stirred for 15 min. The organic layer was separated from aqueous layer, aqueous layer was extracted with dichloromethane (3 x 10 mL). The combined organic layers were washed with brine, dried over sodium sulphate and then concentrated under vacuum to afford the crude residue which was purified by column chromatography over 100-200 mesh silica gel using 15% ethyl acetate and pet ether to afford 3-{[(3,5-bis-trifluoromethyl-benzyl)-(2-methyl-2H-tetrazol-5-yl)-amino]-methyl}-1H-indole-2-carboxylic acid ethyl ester (0.18 g, 94%). Mp: 130 °C; Purity: 97.37% (Symmetry shield RP 8 [0.01M KH₂PO₄, ACN], 210.nm, Rₜ=5.81); ¹H NMR (CDCl₃, 400 MHz):δ 8.69 (br s, 1H), 7.70-7.68 (d, *J* = 7.8 Hz, 1H), 7.54 (s, 1H), 7.39 (s, 2H) ,7.32-7.20 (m, 2H), 7.11-7.09 (m, 1H), 5.35 (s, 2H), 4.67 (s, 2H), 4.31 (q, *J* = 7.2 Hz, 2H), 1.46 (t, *J* = 7.0 Hz, 3H); (CI-MS): *m*/*z* 202 (M⁺-311, 100%), 512 (M⁺, 50%); IR (neat, cm⁻¹): 1708, 1583, 1278.

### Comparative Example 44

### Synthesis of 3-{[(3,5-bis-trifluoromethyl-benzyl)-(2-methyl-2H-tetrazol-5-yl)-amino]-methyl}-1-methyl-1H-indole-2-carboxylic acid ethyl ester

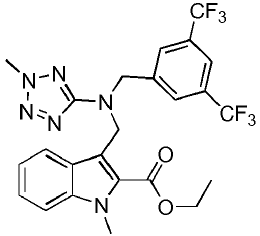

To a suspension of NaH (0.003 g, 0.114 mmol) in DMF (2 mL) was added 3-{[(3,5-bis-trifluoromethyl-benzyl)-(2-methyl-1H-tetrazol-5-yl)-amino]-methyl}-1H-indole-2-carboxylic acid ethyl ester (0.03 g, 0.05 mmol), obtained in step (vi) of Example 43, at 0°C, and stirred for 15 min. Methyl iodide (0.016 g, 0.114 mmol) was added to this at the same temperature and the reaction was stirred for another 1 h. The aqueous layer was extracted with ethyl acetate (3 x 10 mL). The combined organic layers were washed with brine, dried over sodium sulphate and then concentrated under vacuum to afford the crude residue which was purified by column chromatography over 100-200 mesh silica gel using 15% ethyl acetate and petroleum ether to afford 3-{[(3,5-bis-trifluoromethyl-benzyl)-(2-methyl-2H-tetrazol-5-yl)-amino]-methyl}-1H-indole-2-carboxylic acid ethyl ester (0.35 g, 70%).
Mp: 83 °C; Purity: 94.57% (Inertsil ODS [0.01M KH₂PO₄, ACN], 210.nm, Rₜ=8.06). ¹H NMR (CDCl₃, 400 MHz):δ 7.65 (m, 1H), 7.53-7.52 (m, 1H), 7.33-7.26 (m, 4H),7.11-7.07 (m, 1H), 5.29 (s, 2H), 4.64 (s, 2H), 4.78 (q, *J* = 7.2 Hz, 2H), 4.23 (s, 3H), 1.27-1.23 (m, 3H); (CI-MS): *m*/*z* 541 (M⁺+1, 100%); IR (neat, cm⁻¹): 1708, 1583, 1278.

### Comparative Example 45

### Synthesis of 3-{[(3,5-bis-trifluoromethyl-benzyl)-(2-methyl-2H-tetrazol-5-yl)-amino]-methyl}-1-propyl-1H-indole-2-carboxylic acid ethyl ester

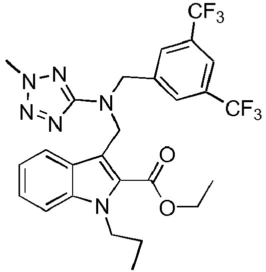

To a suspension of NaH (0.23 g, 1.9 mmol) in DMF (5 mL) was added 3-{[(3,5-bis-trifluoromethyl-benzyl)-(2-methyl-2H-tetrazol-5-yl)-amino]-methyl}-1H-indole-2-carboxylic acid ethyl ester (0.03 g, 0.05 mmol), obtained in step (vi) of Example 43, at 0°C, and stirred for 15 min. Bromopropane (0.23 g, 1.9 mmol) was added to this at the same temperature and the reaction was stirred for another 1 h. The aqueous layer was extracted with ethyl acetate (3 x 20 mL). The combined organic layers were washed with brine, dried over sodium sulphate and then concentrated under vacuum to afford the crude residue which was purified by column chromatography over 100-200 mesh silica gel using 10% ethyl acetate and pet ether to afford 3-{[(3,5-bis-trifluoromethyl-benzyl)-(2-methyl-2H-tetrazol-5-yl)-amino]-methyl}-1-propyl-1H-indole-2-carboxylic acid ethyl ester (0.2 g, yield: 38%).
Purity: 98.49% (Symmetry shield RP8 [0.01M KH₂PO₄, ACN], 227.nm, Rₜ=8.96); ¹H NMR (CDCl₃, 400 MHz):δ 7.67-7.65 (m, 1H), 7.53-7.52 (m, 1H), 7.34-7.30 (m, 4H,7.11-7.08 (m, 1H), 5.29 (s, 2H), 4.64 (s, 2H), 4.37-4.30 (m, 2H), 4.28-4.24 (m, 2H), 4.22 (s, 2H), 1.72-1.64 (m, 2H), 1.28-1.23 (m, 3H), 0.87-0.83 (m, 3H); (CI-MS): *m*/*z* 541 (M⁺+1, 100%); IR (neat, cm⁻¹): 1708, 1583, 1278.

### Comparative Example 46

### Synthesis of 3-{[(3,5-bis-trifluoromethyl-benzyl)-(2-methyl-2H-tetrazol-5-yl)-amino]-methyl}-1-cyclopentanecarbonyl-1H-indole-2-carboxylic acid ethyl ester

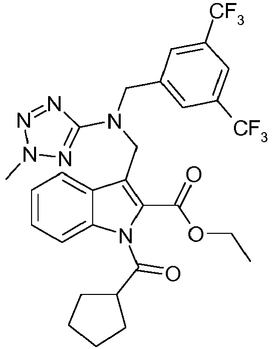

To a suspension of NaH (0.01 g, 0.45 mmol) in DMF (5 mL) was added 3-{[(3,5-bis-trifluoromethyl-benzyl)-(2-methyl-2H-tetrazol-5-yl)-amino]-methyl}-1H-indole-2-carboxylic acid ethyl ester (0.2 g, 0.38mmol), obtained in step (vi) of Example 43, at 0°C, and stirred for 15 min. Cyclopropane carbonyl chloride (0.05 g, 0.38 mmol) was added to this at the same temperature. The reaction was stirred for 1 h. The aqueous layer was extracted with ethyl acetate (3 x 20 mL). The combined organic layers were washed with brine, dried over sodium sulphate and then concentrated under vacuum to afford the crude residue which was purified by column chromatography over 100-200 mesh silica gel using 12% ethyl acetate and petroleum ether to afford 3-{[(3,5-bis-trifluoromethyl-benzyl)-(2-methyl-2H-tetrazol-5-yl)-amino]-methyl}-1-cyclopentanecarbonyl-1H-indole-2-carboxylic acid ethyl ester (0.1 g, yield: 42.3%).
Purity: 93.78% (Symmetry shield RP8 [0.01M KH₂PO₄, ACN], 280 nm, Rₜ=7.77). ¹H NMR (CDCl₃, 400 MHz):δ 7.73-7.71 (m, 1H), 7.67-7.65 (m, 1H), 7.55 (s, 1H), 7.48 (s, 2H), 7.40-7.36 (m, 1H), 7.21-7.17 (m, 1H), 5.14 (s, 2H), 4.73 (s, 2H), 4.30 (q, *J =* 7.2 Hz, 2H), 4.22 (s, 3H), 3.30-3.26 (m, 1H), 1.91-1.77 (m, 6H), 1.64-1.62 (m, 2H), 1.28 (m, 3H); IR (neat, cm⁻¹): 1713, 1578, 1279.

### Comparative Example 47

### Synthesis of 3-{[(3,5-bis-trifluoromethyl-benzyl)-(2-methyl-2H-tetrazol-5-yl)-amino]-methyl}-1-ethyl-1H-indole-2-carboxylic acid ethyl ester

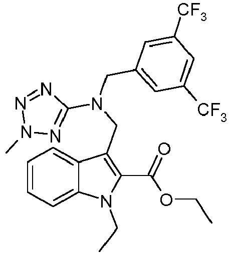

To a suspension of NaH (0.01 g, 0.38 mmol) in DMF (5 mL) was added 3-{[(3,5-bis-trifluoromethyl-benzyl)-(2-methyl-2H-tetrazol-5-yl)-amino]-methyl}-1H-indole-2-carboxylic acid ethyl ester (0.1 g, 0.19 mmol), obtained in step (vi) of Example 43, at 0° C, and stirred for 15 min. Ethyl iodide (0.09 g, 1.08 mmol) was added to this at the same temperature and this reaction was stirred for 1 h. The aqueous layer was extracted with ethyl acetate (3 x 20 mL). The combined organic layers were washed with brine, dried over sodium sulphate and then concentrated under vacuum to afford the crude residue which was purified by column chromatography over 100-200 mesh silica gel using 15% ethyl acetate and pet ether to afford 3-{[(3,5-bis-trifluoromethyl-benzyl)-(2-methyl-2H-tetrazol-5-yl)-amino]-methyl}-1-ethyl-1H-indole-2-carboxylic acid ethyl ester (0.08 g, 80%);
Purity: 98.49% (Inertsil ODS 3V [0.01M KH₂PO₄, ACN], 210nm, Rₜ=8.44); ¹H NMR (CDCl₃, 400 MHz):δ 7.68-7.65 (m, 1H), 7.52 (m, 1H), 7.33-7.30 (m, 4H), 7.11-7.07 (m, 1H), 5.29 (s, 2H), 4.65 (s, 2H), 4.43 (q, *J* = 6.9 Hz, 2H), 4.31-4.25 (m, 2H), 4.22 (s, 3H), 1.30-1.22 (m, 6H); (CI-MS): *m*/*z* 554 (M⁺-2, 10%); IR (neat, cm⁻¹): 1708, 1583, 1278.

### Comparative Example 48

### Synthesis of 3-{[(3,5-bis-trifluoromethyl-benzyl)-(2-methyl-2H-tetrazol-5-yl)-amino]-methyl}-indole-1,2-carboxylic acid ethyl ester

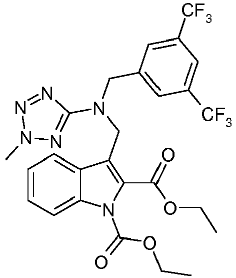

To a suspension of NaH (0.01 g, 0.38 mmol) in DMF (5 mL) was added 3-{[(3,5-Bis-trifluoromethyl-benzyl)-(2-methyl-2H-tetrazol-5-yl)-amino]-methyl}-1H-indole-2-carboxylic acid ethyl ester (0.1 g, 0.19 mmol), obtained in step (vi) of Example 43, at 0°C, and stirred for 15 min. Ethyl chloroformate (0.02 g, 0.22 mmol) was added to this at the same temperature. The reaction was stirred for 4 h. The aqueous layer was extracted with ethyl acetate (3 x 20 mL). The combined organic layers were washed with brine, dried over sodium sulphate and then concentrated under vacuum to afford the crude residue which was purified by column chromatography over 100-200 mesh silica gel using 20% ethyl acetate and petroleum ether to afford 3-{[(3,5-bis-trifluoromethyl-benzyl)-(2-methyl-2H-tetrazol-5-yl)-amino]-methyl}-indole-1,2-carboxylic acid ethyl ester (0.05 g, 45%).
Purity: 90.58% (Inertsil ODS [0.01M KH₂PO₄, ACN], 210nm, Rₜ =13.19); ¹H NMR (CDCl₃, 400 MHz):δ 7.96-7.94 (m, 1H), 7.52-7.50 (m, 4H), 7.36-7.26 (m, 1H) ,7.17-7.13(m, 1H), 4.99 (s, 2H), 4.72 (s, 2H), 4.46 (q, *J* = 7.2 Hz, 2H), 4.33 (q, *J* = 7.2 Hz, 2H), 4.23 (s, 3H), 1.31-1.29 (m, 3H); *m*/*z* (CI-MS) 598 (M⁺+1, 100%).

### Comparative Example 49

### Synthesis of 3-{[(3,5-bis-trifluoromethyl-benzyl)-(2-methyl-2H-tetrazol-5-yl)-amino]-methyl}-1-cyclopropanecarbonyl-1H-indole-2-carboxylic acid ethyl ester

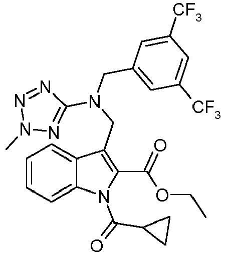

To a suspension of NaH (0.004 g, 0.18 mmol) in DMF (5 mL) was added 3-{[(3,5-bis-trifluoromethyl-benzyl)-(2-methyl-2H-tetrazol-5-yl)-amino]-methyl}-1H-indole-2-carboxylic acid ethyl ester (0.05 g, 0.095 mmol), obtained in step (vi) of Example 43, at 0°C, and stirred for 15 min. Cyclopropanecarbonyl chloride (0.012 g, 0.11 mmol) was added to this at the same temperature and the reaction was stirred for 6 h. The aqueous layer was extracted with ethyl acetate (3 x 20 mL). The combined organic layers were washed with brine, dried over sodium sulphate and then concentrated under vacuum to afford the crude residue which was purified by column chromatography over 100-200 mesh silica gel using 15% ethyl acetate and petroleum ether to afford 3-{[(3,5-bis-trifluoromethyl-benzyl)-(2-methyl-2H-tetrazol-5-yl)-amino]-methyl}-1-cyclopropanecarbonyl-1H-indole-2-carboxylic acid ethyl ester (0.04 g, yield: 71%).
Purity: 98.45% (Inertsil ODS [0.01M KH₂PO₄, ACN], 210nm, Rₜ =9.448.85); ¹H NMR (CDCl₃, 400 MHz):δ 7.89 (m, 1H), 7.67 (m, 1H), 7.53 (s, 2H), 7.38-7.31 (m, 2H) ,7.2 (m, 1H), 5.35 (s, 2H), 4.75 (s, 2H), 4.32-4.27 (m, 2H), 4.20 (s, 3H), 2.04-1.96 (m, 1H), 1.29-1.24 (m, 4H), 1.14-1.12 (m, 3H); *m*/*z* (CI-MS) 595 (M⁺+1, 100%); IR (neat, cm⁻¹): 1711, 1581, 1279.

### Comparative Example 50

### Synthesis of 3-{[(3,5-bis-trifluoromethyl-benzyl)-(2-methyl-2H-tetrazol-5-yl)-amino]-methyl}-3H-indole-2-carboxylic acid diethylamide

### Step(i): Synthesis of 3-{[(3,5-bis-trifluoromethyl-benzyl)-(2-methyl-2H-tetrazol-5-yl)-amino]-methyl}-1H-indole-2-carboxylic acid

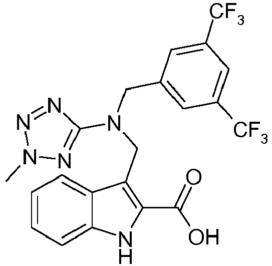

To the solution of 3-{[(3,5-bis-trifluoromethyl-benzyl)-(2-methyl-2H-tetrazol-5-yl)-amino]-methyl}-1H-indole-2-carboxylic acid ethyl ester (0.2 g, 0.38 mmol) in 5 mL ethanol, was added NaOH (0.076 g, 1.9 mmol). The reaction was refluxed under nitrogen atm for 1 h. The reaction mixture was acidified and the suspension was filtered to afford the title compound (0.1 g, 53%)
¹H NMR (CDCl₃, 400 MHz):δ 11.33 (br s, 1H), 7.73 (s, 1H), 7.59-7.57 (m, 3H), 7.35-7.33 (m, 1H) ,7.17-7.13 (m, 1H), 6.97-6.93 (m, 1H), 5.30 (s, 2H), 4.71 (s, 2H), 4.19 (s, 3H); *m*/*z* (CI-MS) 499 (M⁺+1, 100%); IR (neat, cm⁻¹): 1578, 1278, 1133 **Step (ii):** Synthesis of 3-{[(3,5-bis-trifluoromethyl-benzyl)-(2-methyl-2H-tetrazol-5-yl)-amino]-methyl}-1H-indole-2-carboxylic acid diethylamide

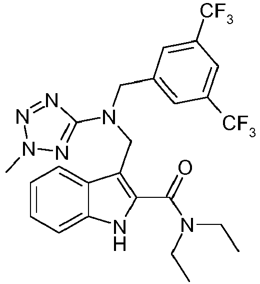

To a solution of 3-{[(3,5-bis-trifluoromethyl-benzyl)-(2-methyl-2H-tetrazol-5-yl)-amino]-methyl}-1H-indole-2-carboxylic acid (0.05 g , 0.10 mmol), obtained in step (i), in DCM (5 mL) under N₂ atmosphere was added EDC (0.36 g, 0.12 mmol) at 0°C followed by the addition of TBTU (0.035 g, 0.12 mmol). The reaction mixture was stirred for 10 min, DIPEA (0.04 g, 0.3 mmol) was added followed by diethylamine (0.008 g, 0.10 mmol) and stirring was continued at RT for 4 h. Water was added to the reaction mixture and extracted with DCM (3 x 10 mL). The combined organic layers were washed with brine, dried over sodium sulphate. The solvent was evaporated and concentrated in *vacuo* which was purified by column chromatography over 100-200 mesh silica gel using 15% ethyl acetate and petroleum ether to afford 3-{[(3,5-bis-trifluoromethyl-benzyl)-(2-methyl-2H-tetrazol-5-yl)-amino]-methyl}-1H-indole-2-carboxylic acid diethylamide (0.035 g, yield: 64%).
Mp 98 °C; Purity: 97.76% (Inertsil ODS 3V [0.01M KH₂PO₄, ACN], 215 nm, Rₜ =7.81).
¹H NMR (CDCl₃, 400 MHz): δ 8.14 (bs, 1H), 7.52 (s, 2H), 7.45-7.43 (m, 2H), 7.23-7.17 (m, 1H), 7.15-6.98 (m, 1H), 4.66 (s, 2H), 4.22 (s, 2H), 3.46-3.41 (m, 4H), 1.12 (t, *J* = 7.1Hz, 6H); *m*/*z* (CI-MS) 553 (M⁺,100%).

### Comparative Example 51

### Synthesis of 3-{[(3,5-bis-trifluoromethyl-benzyl)-(2-methyl-2H-tetrazol-5-yl)-amino]-methyl}-1-methyl-1H-indole-2-carboxylic acid diethylamide

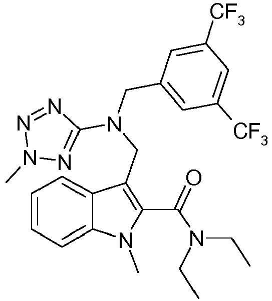

To a suspension of NaH (0.01 g, 0.36 mmol) in DMF (5ml) was added 3-{[(3,5-bis-trifluoromethyl-benzyl)-(2-methyl-2H-tetrazol-5-yl)-amino]-methyl}-1H-indole-2-carboxylic acid diethylamide (0.1 g, 0.18 mmol), obtained in Example 50, at 0°C, and stirred for 15 min. Methyl iodide (0.031 g, 0.216 mmol) was added to this at the same temperature. The reaction was stirred for 6 h and the aqueous layer was extracted with ethyl acetate (3x20 mL). The combined organic layers were washed with brine, dried over sodium sulphate and then concentrated under vacuum to afford the crude residue which was purified by column chromatography over 100-200 mesh silica gel using 15% ethyl acetate and petroleum ether to afford 3-{[(3,5-bis-trifluoromethyl-benzyl)-(2-methyl-2H-tetrazol-5-yl)-amino]-methyl}-1-methyl-1H-indole-2-carboxylic acid diethylamide (0.07 g, 60%).
Purity: 99.11% (Symmetry shield RP 8 [0.01M KH₂PO₄, ACN], 220 nm, Rₜ=6.71); ¹H NMR (CDCl₃, 400 MHz): δ 7.56 (s, 2H), 7.44-7.39 (m, 2H), 7.20-7.18 (m, 2H), 7.00-6.97 (m, 1H), 5.38-5.34 (m, 1H), 4.74-4.70 (m, 1H), 4.56-4.52 (m, 1H), 4.36-4.32 (m, 1H), 4.22 (s, 2H), 3.65 (s, 3H), 3.62-3.56 (m, 2H), 3.34-3.29 (m, 1H), 3.15-3.09 (m, 1H), 1.21-1.17 (m, 3H), 1.03 (t, *J =* 7.0 Hz, 3H); *m*/*z* (CI-MS) 567 (M⁺, 20%); IR (neat, cm⁻¹): 2933, 1278, 1132.

### Comparative Example 52

### Synthesis of 3-{[(3,5-bis-trifluoromethyl-benzyl)-(2-methyl-2H-tetrazol-5-yl)-amino]-methyl}-1-methyl-1H-indole-2-carboxylic acid bis-cyclopropylmethyl-amide

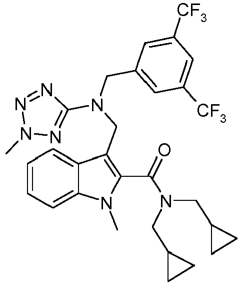

3-{[(3,5-Bis-trifluoromethyl-benzyl)-(2-methyl-2H-tetrazol-5-yl)-amino]-methyl}-1-methyl-1H-indole-2-carboxylic acid (0.08 g, 0.156 mmol) and bis cyclopropylmethylamine (0.02 g, 0.156 mmol) were used to synthesize the title compound instead of using 3-{[(3,5-bis-trifluoromethyl-benzyl)-(2-methyl-2H-tetrazol-5-yl)-amino]-methyl}-1H-indole-2-carboxylic acid and diethylamine in step (ii) of example 50 (0.05 g, yield: 62%).
Purity: 98.43% (Symmetry shield RP 8 [0.01M KH₂PO₄, ACN], 220 nm, Rₜ=7.315); ¹H NMR (CDCl₃, 400 MHz):δ 7.53-7.51 (m, 1H), 7.42-7.40 (m, 2H), 7.19-7.17 (m, 2H), 7.00-6.96 (m, 1H), 5.39-5.36 (m, 1H), 4.73-4.69 (m, 1H), 4.54-4.50 (m, 1H), 4.38-4.34 (m, 1H), 4.22 (s, 3H), 3.67 (s, 3H), 3.65-3.55 (m, 1H), 3.54-3.52 (m, 1H), 3.17-3.15 (m, 2H), 0.88-0.83 (m, 2H), 0.49-0.30 (m, 2H), 0.53-0.50 (m, 4H); (CI-MS): *m*/*z* 620 (M⁺+1, 90%).

### Comparative Example 53

### Synthesis of 3-{[(3,5-bis-trifluoromethyl-benzyl)-(2-methyl-2H-tetrazol-5-yl)-amino]-methyl}-1-propyl-1H-indole-2-carboxylic acid bis-cyclopropylmethyl-amide

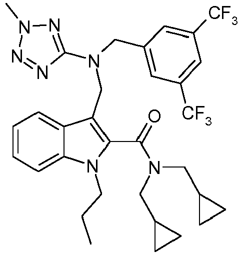

3-{[(3,5-Bis-trifluoromethyl-benzyl)-(2-methyl-2H-tetrazol-5-yl)-amino]-methyl}-1-propyl-1H-indole-2-carboxylic acid (0.16g, 0.29 mmol) and bis cyclopropylmethylamine (0.37 g, 0.29 mmol) were used to synthesize the title compound instead of using 3-{[(3,5-bis-trifluoromethyl-benzyl)-(2-methyl-2H-tetrazol-5-yl)-amino]-methyl}-1H-indole-2-carboxylic acid and diethylamine in step (ii) of example 50 (0.08 g, 42%)
Purity: 98.49% (Symmetry shield RP18 [0.01M KH₂PO₄, ACN], 220 nm, Rₜ=8.85). ¹H NMR (CDCl₃, 400 MHz): δ 7.51-7.48 (m, 3H), 7.24-7.21 (m, 2H), 7.19-7.17 (m, 1H), 7.04-7.00 (m, 1H), 4.69-4.58 (q, *J*=16.1Hz, 2H), 4.20 (s, 3H), 4.15-4.08 (m, 1H), 3.92-3.85 (m, 1H), 3.70-3.64 (m, 1H), 3.55-3.45 (m, 1H), 3.13-3.12 (m, 4H), 1.82-1.660.88-0.83 (m, 2H), 1.29-1.22 (m, 2H), 0.86 (t, *J =* 7.3Hz, 3H), 0.51-0.47 (m, 4H), 0.01 (m, 4H); (CI-MS) *m*/*z*: 648 (M⁺+1, 60%).

### Example 54

### Synthesis of (3,5-bis-trifluoromethyl-benzyl)-(8-methyl-2-propylsulfanyl-quinolin-3-ylmethyl)-carbamic acid ethyl ester

### Step (i): Synthesis of 8-methyl-2-propylsulfanyl-quinoline-3-carbaldehyde

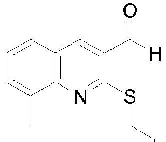

To 2-chloro-8-methylquinoline-3-carboxaldehyde (2.055 g, 10 mmol) in ethanol (25 mL) was added anhydrous potassium carbonate (2.76 g, 20 mmol) followed by the addition of *n*-propanethiol (1.12 mL, 13 mmol). The reaction mixture was heated at reflux under a nitrogen atmosphere with stirring for 1.75 h. Then water (30 mL) was added and the mixture was stirred approximately 15 h at room temperature. The mixture was extracted 3 times diethyl ether, and the combined ether phases were sequentially washed one time with 10% sodium hydroxide and brine. The organic phase was dried over potassium carbonate, filtered and concentrated by rotary evaporation. The material was purified by chromatography. (Biotage Horizon HPFC chromatography system, SiO₂, 80:20 hexanes: ethyl acetate) gave a yellow solid (1.979 g, 80%). No further purification was completed and the material was used as is for the next step.
HPLC: Inertsil ODS-3V C18, 30:70 [KH₂PO₄ (0.01M, pH 3.2): CH₃CN], 264 nm, Rₜ 27 min, 77.5% purity; ¹H NMR (300 MHz, CDCl₃, TMS): □ 10.33 (s, 1H), 8.42 (s, 1H), 7.62-7.10 (m, 2H), 7.38 (t, *J* = 7.5 Hz, 1H), 3.37 (t, *J* = 7.2 Hz, 2H), 2.75 (s, 3H), 1.87 (sextet, *J* = 7.2 Hz, 2H), 1.11 (t, *J* = 7.5 Hz, 3H).

### Step (ii): Synthesis of (3,5-bis-trifluoromethyl-benzyl)-(8-methyl-2-propylsulfanyl-quinolin-3-ylmethyl)-amine

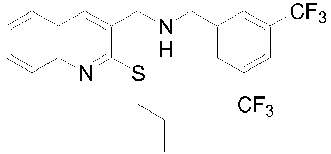

3,5-Bis-(trifluoromethyl) benzylamine (1.014 g, 4.2 mmol) and 8-methyl-2-propylsulfanyl-quinoline-3-carbaldehyde (1.006 g, 4.0 mmol) was dissolved in anhydrous methanol (12 mL). Acetic acid (0.5 mL, 8.8 mmol) was added and the mixture was stirred for approximately 1 h at RT under a nitrogen atmosphere. Sodium cyanoborohydride (0.800 g, 12.6 mmol) was added and the mixture was stirred for an additional 24 h at RT. The reaction was quenched with saturated sodium bicarbonate and then stirred for 15-20 min at RT. The mixture was extracted 3 times with diethyl ether. The combined ether layers were then washed 2 times with brine, then dried over anhydrous potassium carbonate, filtered and concentrated *in vacuo.* The material was purified by chromatography. (Biotage Horizon HPFC chromatography system, SiO₂, 90:10 hexanes: ethyl acetate) gave an oil (1.13 g, 58.6%).
HPLC: Inertsil ODS-3V C18, 30:70 [KH₂PO₄ (0.01M, pH 3.2): CH₃CN], 264 nm, Rₜ 50.7 min, 99.5% purity; ¹H NMR (300 MHz, CDCl₃, TMS): □ 7. 69-7.88 (m, 4 H), 7.48-7.57 (m, 2 H), 7.29-7.34 (m, 1H), 3.97 (s, 2H), 3.96 (s, 2H), 3.37 (t, *J=* 7.2H, 2H), 2.75 (s, 3H), 1.85 (sextet, *J* = 7.5 Hz, 3 H; D₂O exchange, overlap ofNH), 1.09 (t, *J* = 7.5Hz, 3H); Mass: LC-MSD (ES+): *m*/*z* 473 (M+H, 100).

### Step (iii): Synthesis of (3,5-bis-trifluoromethyl-benzyl)-(8-methyl-2-propylsulfanyl-quinolin-3-ylmethyl)-carbamic acid ethyl ester

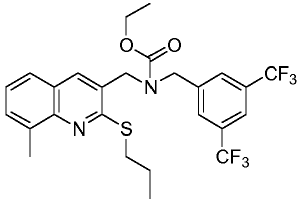

To ethyl chloroformate (0.4 ml, 4.2 mmol) and potassium carbonate (0.875 g, 6.3 mmol) were added to (3,5-bis-trifluoromethyl-benzyl)-(8-methyl-2-propylsulfanyl-quinolin-3-ylmethyl)-amine (1.058 g, 2.1 mmol) which was dissolved in anhydrous THF (10 mL). The resulting mixture was stirred at room temperature for overnight. The mixture was diluted with methylene chloride, and was washed one time with water. The organic phase was dried over potassium carbonate and concentrated by rotary evaporation. The resulting sample was dried under vacuum gave a light yellow solid (1.18 g, 97%).
M.P.: 42 °C; HPLC: Inertsil ODS-3V C18, 30:70 [KH₂PO₄ (0.01M, pH 3.2): CH₃CN], 264 nm, Rₜ 61.9 min, 99.6% purity; ¹H NMR (300 MHz, CDCl₃, TMS): □ 7.65-7.32 (brm, 3H), 7.51 (t, *J* =7.2 Hz, 2H), 7.32 (t, *J* =7.5 Hz, 1H), 4.67-4.58 (m, 4H), 4.3 (q, *J* =7.2 Hz, 2H), 3.34 (t, *J* =7.5 Hz, 2H), 2.74 (s, 3H), 1.87-1.79 (m, 2H), 1.30 (t, *J* = 7.2 Hz, 3H), 1.07 (t, *J* = 7.5 Hz, 3H); Mass: LC-MSD (ES+): *m*/*z* 545 (M+H, 100).

### Example 55

### Synthesis of (3,5-Bis-trifluoromethyl-benzyl)-[8-methyl-2-(propane-1-sulfonyl)-quinolin-3-ylmethyl]-carbamic acid ethyl ester

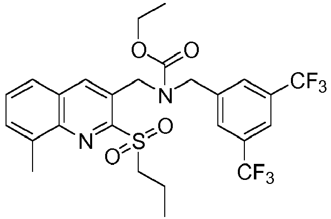

(3,5-Bis-trifluoromethyl-benzyl)-(8-methyl-2-propylsulfanyl-quinolin-3-ylmethyl)-carbamic acid ethyl ester (Example 54) (504 mg, 0.9 mmol) was dissolved in methanol (15 mL) and water (10 mL) then OXONE (5.54 g, 9 mmol) was added. The resulting mixture was stirred at room temperature overnight. The mixture was diluted with methylene chloride, and was washed one time with water and one time with brine. The organic phase was dried over sodium sulfate and concentrated by rotary evaporation. The resulting sample was dried under vacuum. (Biotage Horizon HPFC chromatography system, SiO₂, 80:20 hexanes: ethyl acetate) gave a white solid (274 mg, 51%).
M.P.: 98°C; HPLC: Inertsil ODS-3V C18, 30:70 [KH₂PO₄ (0.01M, pH 3.2): CH₃CN], 264 nm, Rₜ 40.9 min, 95.3% purity; ¹H NMR (300 MHz, CDCl₃, TMS): □ 8.2-8.0 (brm, 1H), 7.73-7.54 (m, 6H), 5.20-5.24 (m, 2H, rotomers), 4.68 (s, 2H), 4.31 (brs, 2H), 3.81-3.76 (m, 2H), 2.74 (s, 3H), 2.11-1.99 (m, 2H), 1.306-1.23 (m, 3H), 1.20 (t, *J* = 7.5 Hz, 3H); Mass: LC-MSD (ES+): *m*/*z* 599 (M+Na, 100).

### Example 56

### Synthesis of (3, 5-bis-trifluoromethyl-benzyl)-[8-methyl-2-(propane-1-sulfinyl)-quinolin-3-ylmethyl]-carbamic acid ethyl ester

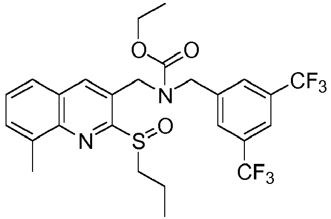

(3,5-Bis-trifluoromethyl-benzyl)-(8-methyl-2-propylsulfanyl-quinolin-3-ylmethyl)-carbamic acid ethyl ester (Example 54) (116 mg, 0.18 mmol) was dissolved in methanol (2 mL) and water (2 mL) followed by the addition of magnesium bis(mono peroxy phthalate hexahydrate) (MMPP) (267.1 mg, 0.54 mmol). The resulting mixture was stirred at room temperature overnight. The sample was diluted with methylene chloride and washed one time with water and one time with brine. The organic phase was dried, filtered and concentrated by rotary evaporation. The resulting sample was dried overnight under vacuum. (Biotage Horizon HPFC chromatography system, SiO₂, 70:80 hexanes: ethyl acetate) gave a whit solid (80 mg, 72%).
M.P.: 86°C; HPLC: Inertsil ODS-3V C18, 20:80 [KH₂PO₄ (0.01M, pH 3.2): CH₃CN], 264 nm, Rₜ 20.7 min, 99.8% purity; ¹H NMR (300 MHz, CDCl₃, TMS): □ 8.18-7.99 (m, 1H), 7.7 (brs, 3H), 7.65-7.60 (m, 2H), 7.54-7.49 (m, 1H), 5.24-5.10 (brm, 2H, rotomers), 4.72 (s, 2H), 4.28 (q, *J* = 6.9 Hz, 2H), 3.30 (t, *J* = 7.2 Hz, 2H), 2.77 (s, 3H), 1.95-1.72 (m, 2H), 1.27 (t, J = 6.6 Hz, 3H), 1.11 (t, *J* = 7.5 Hz, 3H); Mass: LC-MSD (ES+): *m*/*z 561* (M+H, 100).

### Example 57

### Synthesis of (3-{[(3,5-Bis-trifluoromethyl-benzyl)-(2-methyl-2H-tetrazol-5-yl)-amino]-methyl}-6-fluoro-pyridin-2-yl)-bis-cyclopropylmethyl-amine

### Step (i): Synthesis of 2-(bis-cyclopropylmethyl-amino)-6-fluoro-pyridine-3-carbaldehyde

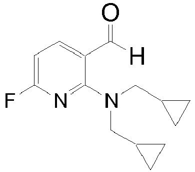

Diisopropylamine (1.4 mL, 9.6 mmol) was stirred in ether (10 mL). The solution is cooled -78°C with dry ice and acetone. n-Butyllithium (4 mL, 9.6 mmol) was added and the resulting solution was allowed to stir at -78°C for about 15 minutes. 2,6-Difluoropyridine (0.8 mL, 8.7 mmol) was added and the solution was allowed to stir at -78°C for about 2 hours. *N,N-*bis-cyclopropylmethyl formamide (1.71g, 10.9 mmol) was added and the solution was allowed to stir at -78°C for about 1 hour. The solution was quenched with 10% potassium phosphate. The solution was extracted with ether (2 x 20 mL). The combined organic layers were washed with water and brine, dried over sodium sulfate, filtered and then concentrated under vacuum to give an orange oily product. Purification by silica gel chromatography and eluting with 20% ethyl acetate in hexanes afforded the title compound as an orange oil (224 mg, yield: 10.3%).
¹HNMR (CDCl₃, 300 MHz): □ 9.96 (s, 1H), 8.08 (t, *J* = 8.4 Hz, 1H), 6.34 (dd, *J* = 5.1, 3.3 Hz, 1H), 3.48 (d, *J* = 6.6 Hz, 4H), 1.16-1.07 (m, 2H), 0.574-0.513 (m, 4H), 0.245-0.193 (m, 4H); Mass: LC-MSD (ES+): *m*/*z* 248 (M + 1, 100%).

### Step (ii): Synthesis of {3-[(3,5-bis-trifluoromethyl-benzylamino)-methyl]-6-fluoro-pyridin-2-yl}-bis-cyclopropylmethyl-amine

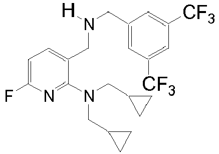

To 2-(bBis-cyclopropylmethyl-amino)-6-fluoro-pyridine-3-carbaldehyde (200 mg, 0.8 mmol) stirred in THF (10 mL) was added 3,5-bis-(trifluoromethyl) benzylamine (198.1 mg, 0.8 mmol) and acetic acid (0.05 mL, 0.8 mmol). After stirring at room temperature for about 1 hour, sodium triacetoxyborohydride (237.5 mg, 1.12 mmol) was added and the solution was allowed to stir at room temperature overnight. The solution was extracted with ether (2 x 10 mL). The combined organic layers were washed with water and brine, dried over potassium carbonate, filtered, and then concentrated under vacuum to give the title compound an orange oily product (361 mg, yield: 95%). Used as is with no purification.
¹H NMR (CDCl₃, 300 MHz): □ 7.82-7.74 (m, 3H), 7.67 (t, *J* = 8.4 Hz, 1H), 6.52 (dd, *J* = 4.5, 3.3 Hz, 1H), 4.90 (s, 1H), 3.85 (d, *J* = 13.5 Hz, 4H), 3.10 (d, *J* = 6.9 Hz, 3H), 0.948-0.851 (m, 2H), 0.419-0.358 (m, 4H), 0.069-0.011 (m, 4H); Mass: LC-MSD (ES+): *m*/*z* 476 (M + 1, 99.7%).

### Step (iii): Synthesis of [2-(bis-cyclopropylmethy-amino)-6-fluoro-pyridin-3-ylmethyl]-(3,5-bis-trifluoromethyl-benzyl)-cyanamide

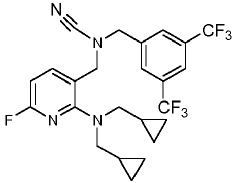

To {3-[(3,5-bis-trifluoromethyl-benzylamino)-methyl]-6-fluoro-pyridin-2-yl}-bis-cyclopropylmethyl-amine (302 mg, 0.64 mmol) stirred in methanol (10 mL) was added sodium bicarbonate (139.6 mg, 1.24 mmol). After stirring at room temperature for about 15 minutes, cyanogen bromide (237.5 mg, 1.12 mmol) was added and the solution was allowed to stir at room temperature for about 4 hours. The solution was extracted with ethyl acetate (2 x 15 mL). The combined organic layers were washed with water and brine, dried over sodium sulfate, filtered, and then concentrated under vacuum to give the title compound as an orange oily product (284 mg, yield: 87%) and was used as is without purification.
¹H NMR (CDCl₃, 300 MHz): □ 7.89 (s, 1H), 7.81-7.73 (m, 3H), 6.61 (dd, *J* = 4.5, 3.6 Hz, 1H), 4.27 (broad d, *J* = 23.4 Hz, 4H), 3.28 (d, *J* = 6.9 Hz, 1H), 3.04 (d, *J* = 6.6 Hz, 2H); Mass: LC-MSD (ES+): *m*/*z* 501 (M + 1, 81%).

### Step (iv): Synthesis of (3-{[(3,5-bis-trifluoromethyl-benzyl)-(1H-tetrazol-5-yl)-amino]-methyl}-6-fluoro-pyridine-2-yl)-bis-cyclopropylmethyl-amine

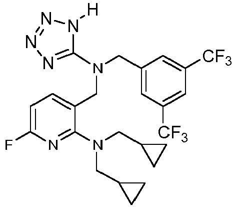

To [2-(bis-cyclopropylmethy-amino)-6-fluoro-pyridin-3-ylmethyl]-(3,5-bis-trifluoromethyl-benzyl)-cyanamide (244 mg, 0.5 mmol) stirred in isopropanol (5 mL) and water (10 mL) was added sodium azide (38.4 mg, 0.55 mmol) and zinc bromide (115.6 mg, 0.5 mmol). After stirring at reflux overnight, the solution was extracted with ethyl acetate (2 x 15 mL). The combined organic layers were washed with water and brine, dried over sodium sulfate, filtered and then concentrated under vacuum to give the title compound as an orange oily product (248 mg, yield: 91%) and was used as is without purification.
Mass: LC-MSD (ES+): *m*/*z* 544 (M + 1, 82%).

### Step (v): Synthesis of (3-{[(3,5-bis-trifluoromethyl-benzyl)-(2-methyl-2H-tetrazol-5-yl)-amino]-methyl}-6-fluoro-puridine-2-yl)-bis-cyclopropylmethyl-amine

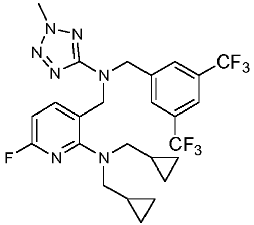

To sodium hydroxide (33.6 mg, 0.8 mmol) stirred in water (2 mL) was added (3-{[(3,5-bis-trifluoromethyl-benzyl)-(1H-tetrazol-5-yl)-amino]-methyl}-6-fluoro-pyridine-2-yl)-bis-cyclopropylmethyl-amine (222 mg, 0.4 mmol) in dichloromethane (5 mL). After stirring for 15 minutes, dimethyl sulfate (0.05 mL, 0.44 mmol) and tetrabutylammonium bromide (7.5 mg, 0.0176 mmol) was added and the solution was left to stir at room temperature for 1 hour. The solution was extracted with dichloromethane (2 x 15 mL). The combined organic layers were washed with water and brine, dried over sodium sulfate, filtered and then concentrated under vacuum to give an orange oily product. Purification by silica gel chromatography and eluting with 30% ethyl acetate in hexanes afforded the title compound as an orange oil (124.6 mg, yield: 56%).
¹H NMR (CDCl₃, 300 MHz): □ 7.75-7.67 (m, 3H), 7.56 (t, *J* = 8.1 Hz, 3H), 6.45 (dd, *J* = 4.5, 3.6 Hz, 1H), 4.67 (broad d, *J* = 39.9 Hz, 4H), 4.20 (s, 3H), 3.03 (d, *J* = 6.6 Hz, 4H), 0.900-0.809 (m, 2H), 0.399-0.338 (m, 4H), 0.063-0.025 (m, 4H); Mass: LC-MSD (ES+): *m*/558 (M + 1, 90%).

### Example 58

### Synthesis of 2-[4-(3-{[(3,5-Bis-trifluoromethyl-benzyl)-(5-bromo-pyrimidin-2-yl)-amino]-methyl}-8-methyl-quinolin-2-yl)-piperazin-1-yl]-1-pyrrolidin-1-yl-ethanone

### Step (i): Synthesis of 8-methyl-2-[4-(2-oxo-2-pyrrolidin-1-yl-ethyl)-piperazin-1-yl]-quinoline-3-carbaldehyde

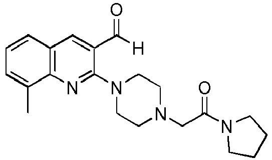

To 2-chloro-8-methylquionline-3-carboxaldehyde (0.2034 g, 1 mmol) dissolved in *N,N*-dimethylformamide (7 mL) was added 1-[2-(piperazine-1-yl)acetyl] pyrrolidine (0.2271 g, 1.15 mmol) followed by potassium carbonate (0.1395 g, 1 mmol). After stirring at reflux overnight under nitrogen, the solution was extracted with dichloromethane. The combined organic layers were washed with water and brine, dried over potassium carbonate, filtered and then concentrated under vacuum. Purification by silica gel chromatography and eluting with 90:9:1 (methylene chloride: methanol: NH₄OH) afforded the title compound as a yellow gummy liquid. (166 mg, yield: 46%).
HPLC: YMC Pro C-8, 40:30:30 [KH₂PO₄ (0.01M, pH 3.2): CH₃CN: methanol], 264 nm, Rₜ 2.5 min, 97.6% purity.
¹H NMR (300 MHz, CDCl₃, TMS): □ 10.15 (s, 1H), 8.50 (s, 1H), 7.68-7.58 (m, 2H), 7.34-7.29 (m, 1H), 3.97-3.46 (brm, 14H), 2.06-1.97 (m, 2H), 1.94-1.85 (m, 2H). Mass: LC-MSD (ES+): *m*/*z* **367** (M + 1, 100).

### Step (ii): Synthesis of 2-(4-{3-[(3,5-bis-trifluoromethyl-benzylamino)-methyl]-8-methyl-quinolin-2-yl}-piperazin-1-yl)-1-pyrrolidin-1-yl-ethanone

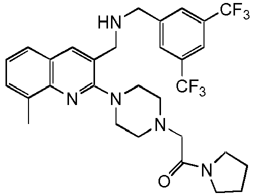

To of 8-methyl-2-[4-(2-oxo-2-pyrrolidin-1-yl-ethyl)-piperazin-1-yl]-quinoline-3-carbaldehyde (152 mg, 0.4 mmol) stirred in THF (7 mL) was added 3,5-bis-(trifluoromethyl) benzylamine (0.0995 mg, 0.4 mmol) and acetic acid (0.02 mL, 0.4 mmol). After stirring at room temperature for about 1 hour, sodium triacetoxyborohydride (0.1201 mg, 0.56 mmol) was added and the solution was allowed to stir at room temperature overnight under nitrogen. The solution was extracted with ether (2 x 10 mL). The combined organic layers were washed with water and brine, dried over potassium carbonate, filtered, and then concentrated under vacuum. Purification by silica gel chromatography and eluting with 90:9:1 (methylene chloride: methanol: NH₄OH) afforded the title compound as a brown liquid (144 mg, yield: 60%) and was used without further purification.
HPLC: YMC Pro C-8, 40:30:30 [KH₂PO₄ (0.01M, pH 3.2): CH₃CN: methanol], 254 nm, Rₜ 5.0 min, 89.8% purity; Mass: LC-MSD (ES+): *m*/*z* **594** (M + 1, 100).

### Step (iii): Synthesis of 2-[4-(3-{[(3,5-bis-trifluoromethyl-benzyl)-(5-bromo-pyrimidin-2-yl)-amino]-methyl}-8-methyl-quinolin-2-yl)-piperazin-1-yl]-1-pyrrolidin-1-yl-ethanone

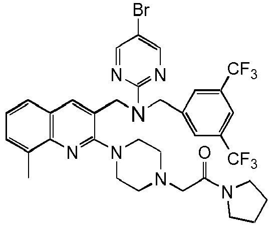

To 8-methyl-2-[4-(2-oxo-2-pyrrolidin-1-yl-ethyl)-piperazin-1-yl]-quinoline-3-carbaldehyde (121 mg, 0.2 mmol) stirred in *N,N-*dimethylformamide (5 mL) was added 5-bromo-2-chloropyrimidine (0.0587 mg, 0.3 mmol) and diisopropylethylamine (0.03 mL, 0.2 mmol). After stirring at reflux overnight under nitrogen, the solution was extracted with dichloromethane. The combined organic layers were washed with water two times and brine one time, dried over potassium carbonate, filtered and then concentrated under vacuum. Purification by silica gel chromatography and eluting with 95:5 (methylene chloride: methanol) afforded the title compound as a brown semisolid. (140 mg, yield: 92%).
HPLC: YMC Pro C-8, 30:70 [KH₂PO₄ (0.01M, pH 3.2): CH₃CN], 254 nm, Rₜ 4.1 min, 95.3% purity; ¹H NMR (300 MHz, CDCl₃, TMS): □ 8.41 (s, 2H), 7.70-7.69 (brm, 2H), 7.62 (s, 2H), 7.42 (t, *J* = 8.4 Hz, 2H), 7.26-7.20 (m, 1H), 5.00 (s, 2H), 4.78 (s, 2H), 3.50 (q, *J*₁ = 9.3 Hz, 4H), 3.33 (brt, *J* = 4.5 Hz, 4H), 3.20 (s, 2H), 2.73 (brs, 4H), 2.69 (s, 3H), 2.01-1.92 (m, 2H), 1.90-1.81 (m, 2H); Mass: LC-MSD (ES+): *m*/*z* 752 (M + 1, 100).

### Example 59

### Synthesis of [3-({((3,5-bis-trifluoromethyl-benzyl)-[(4-morpholin-4-yl-phenyl)-amino]-methyl)-8-methyl-quinolin-2-yl]-bis-cyclopropylmethyl-amine

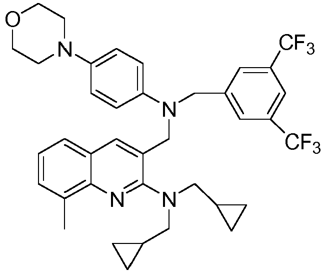

The title compound of 97.0 % purity (HPLC: YMC Pro C8, 20:80 [KH₂PO₄ (0.01 M, pH 3.2):CH₃CN], flow rate 1.2 mL/min, Rₜ 23.5 min) was obtained in a similar manner described in Example 25 as a pale yellow thick liquid (0.11g, 36%).
¹H NMR (CDCl₃, 300 MHz): δ 7.88 (s, 1H), 7.77-7.72 (m, 3H), 7.46-7.39 (m, 2H), 7.24-7.19 (m, 1H), 6.82-6.77 (m, 2H), 6.69-6.63 (m, 2H), 4.72-4.69 (m, 4H), 3.85-3.80 (m, 4H), 3.21 (d, J= 6.6 Hz, 4H), 3.08-2.98 (m, 4H), 2.72 (s, 3H), 1.09-1.0 (m, 2H), 0.43-0.35 (m, 4H), 0.13-0.06 (m, 4H); MS (ESI) *m*/*z* 683 (M+1)⁺.

### Example 60

### Synthesis of 4-{[[2-(bis-cyclopropylmethyl-amino)8-methyl-quinoline-3-ylmethyl]-(2-methyl-2H-tetrazol-5-yl)-amino]-methyl}-2,6-di-tert-butyl-phenol

### Step (i): Synthesis of [2-(bis-cyclopropylmethyl-amino)-8-methyl-quinolin-3-yl]-methanol

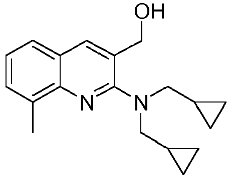

To a solution of of 2-(bis-cyclopropylmethyl-amino)-8-methyl-quinolin-3-carbaldehyde (0.276 g, 0.938 mmol) obtained in step (ii) of Example 57 in 5mL THF was added sodiumborohydride (0.042 g, 1.12 mmol). The reaction mixture was stirred at RT for 5 min, then water was added to reaction mixture and this was extracted with ethyl acetate (3 x 10 mL). The combined organic layer was washed with water and brine respectively and the solution was dried over sodium sulfate. The volatiles were removed under vacuum to get the pure compound (0.25 g, yield: 93%). ¹H NMR (CDCl₃, 400 MHz): δ 7.88 (s, 1H), 7.58 (m, 1H), 7.56 (m, 1H), 7.48-7.46 (m, 1H), 5.18 (br s, 1H), 4.89 (s, 2H), 3.22-3.20 (m, 4H), 2.73 (s, 3H), 1.08-1.01 (m, 2H), 0.14-0.10 (m ,4H), 0.14-0.11 (m, 4H); *m*/*z* (ES-MS): 297 (M⁺+1, 100%); IR (neat, cm⁻¹):3382, 1618, 1050.

### Step (ii): Synthesis of (3-azidomethyl-8-methyl-quinolin-2-yl-bis-cyclopropylmethyl-amine

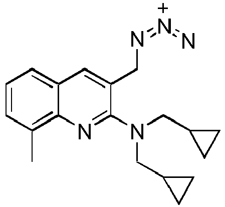

Triethylamine (1.75 mL, 12.62 mmol) was added to a solution of of [2-(bis-cyclopropylmethyl-amino)-8-methyl-quinolin-3-yl]-methanol (1.2 g, 4.05 mmol), obtained in step (i) in 15 mL DCM. This solution was cooled to 0°C, and mesyl chloride (0.47 mL, 12.62 mmol) was added over a period of 15 min. The reaction mixture was stirred at RT for overnight and the solvent was evaporated to get the required methanesulfonic acid 2-(bis-cyclopropylmethyl-amino)-8-methyl-quinolin-3-yl methyl ester (1.5 g, yield: 90%).

The methanesulfonate (1.5 g, 4.03 mmol) was dissolved in 4 mL of DMF in a 25 mL RB flask. To this solution, sodium azide (0.786 g, 12.09 mmol) was added and stirring was continued for 1 h at 40°C. The reaction mixture was cooled to RT and then water was added to this which was extracted with ethyl acetate (3 x 10 mL). The combined organic layer was washed with water and brine and the volatile was removed under vacuum to get the crude which was purified by column chromatography over silica gel (100-200 mesh) using 2% ethyl acetate and petroleum ether to give (3-azidomethyl-8-methyl-quinolin-2-yl-bis-cyclopropylmethyl-amine (0.4 g, yield: 30%).
¹H NMR (CDCl₃, 400 MHz): δ 8.0 (s, 1H), 7.60-7.56 (m, 1H), 7.49-7.45 (m, 1H), 7.28-7.24 (m, 1H), 4.61(s, 2H), 3.27-3.25 (m, 4H), 2.71 (s, 3H), 1.12-1.04 (m, 2H), 0.45-0.40 (m ,4H), 0.14-0.11 (m, 4H); *m*/*z* (ES-MS): 322 (M⁺+1, 10%), 225 (M⁺-42, 100%)

### Step (iii): Synthesis of (3-aminomethyl-8-methyl-quinolin-2-yl-bis-cyclopropylmethyl-amine

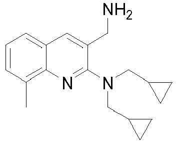

(3-Azidomethyl-8-methyl-quinolin-2-yl-bis-cyclopropylmethyl-amine (0.35 g, 1.09 mmol) was dissolved in 3 mL of THF in a 25 mL RB. Triphenyl phosphine followed by one drop of water was added to this and then stirring was continued for 1 h at 40°C. The reaction mixture was cooled to RT and then poured in 1N HCl (10 mL) solution which was diluted with ethyl acetate and basified with potassium carbonate where pH was adjusted to 9. This was extracted with ethyl acetate (3 x 10 mL). The combined organic layer was washed with water and brine and the volatile was removed under vacuum to give the required amine (0.4 g, yield: 60%)
¹H NMR (CDCl₃, 400 MHz): δ 7.95 (s, 1H), 7.56-7.54 (m, 1H), 7.45-7.44 (m, 1H), 7.29-7.26 (m, 1H), 4.15-4.11 (m, 2H), 3.24-3.17 (m, 2H), 2.72 (s, 3H), 1.07-1.03 (m, 2H), 0.46-0.41 (m ,4H), 0.14-0.10 (m, 4H); *m*/*z* (ES-MS): 296 (M⁺+1, 80%).

### Step (iv): Syntheis of 4-({[2-(bis-cyclopropylmethyl-amino)-8-methyl-quinolin-3-ylmethyl]-amino}-methyl)-2,6-di-tert-butyl-phenol

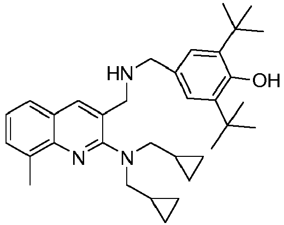

(3-Aminomethyl-8-methyl-quinolin-2-yl-bis-cyclopropylmethyl-amine (0.05 g, 0.17 mmol) obtained in step (iii), 3,5-di-*tert*-butyl-4-hydroxy-benzaldehyde (0.096 g, 0.041 mmol) and acetic acid (0.02 g, 0.338 mmol) were put in a 25 mL RB flask. To this, 2 mL of methanol was added and stirred at RT for 15 min. Sodium cyanoborohydride (0.021 g, 0.39 mmol) was added portion wise and stirring was continued at RT for another 1 h. Methanol was removed from the reaction mixture under vacuum, water was added to this crude and was extracted with ethyl acetate (3 x 50 mL). The organic layer was washed with saturated NaHCO₃ solution, brine and dried over sodium sulphate. The solvent was evaporated and the crude residue was purified by column chromatography over silica gel (100-200 mesh) eluting with 4% ethyl acetate in petroleum ether to give the title amine (0.05 g, yield: 69%);
¹H NMR (CDCl₃, 400 MHz): δ 8.09 (s, 1H), 7.72 (m, 2H), 7.68-7.66 (m, 1H), 7.58-7.57 (m, 1H), 7.45-7.43 (m, 1H), 4.35 (s, 2H), 4.03 (s, 2H), 3.08-3.06 (m, 4H), 2.73 (s, 3H), 1.44 (s, 18H), 0.75 (m, 2H), 0.39-0.35 (m ,4H), 0.07-0.00 (m, 4H); *m*/*z* (ES-MS): 514 (M⁺+1, 100%).

### Step (v): Synthesis of [2-(bis-cyclopropylmethyl-amino)-8-methyl-quinolin-3-ylmethyl]-(3,5-di-tert-butyl-4-hydroxy-benzyl)-cyanamide

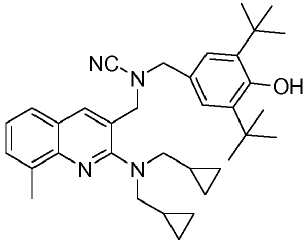

To a solution of 4-({[2-(bis-cyclopropylmethyl-amino)-8-methyl-quinolin-3-ylmethyl]-amino}-methyl)-2,6-di-*tert*-butyl-phenol (0.05 g, 0.097 mmol), obtained in step (iv), in MeOH (4 mL) under N₂ atmosphere was added sodium bicarbonate (0.016 g, 0.195 mmol) followed by the addition of cyanogen bromide (0.018 g, 0.175 mmol). The reaction mixture was stirred at RT for 2 h. The solvent was removed under vacuum to give the crude residue which was dissolved in water, extracted with ethyl acetate and dried over sodium sulphate. The solvent was evaporated and concentrated in *vacuo* to afford [2-(bis-cyclopropylmethyl-amino)-8-methyl-quinolin-3-ylmethyl]-(3,5-di-*tert*-butyl-4-hydroxy-benzyl)-cyanamide (0.035 g, yield: 66.7%).
¹H NMR (CDCl₃, 400 MHz): δ 8.05 (s, 1H), 7.57 (m, 1H), 7.48 (m, 1H), 7.30 (s, 1H), 7.03 (s, 2H), 4.40 (s, 2H), 4.06 (s, 2H), 3.18 (m, 4H), 2.71 (s, 3H), 1.44 (s, 3H), 1.02 (m, 2H), 0.40 (m, 4H), 0.09 (m, 4H); *m*/*z* (ES-MS): 539 (M⁺+1, 100%); IR (KBr, cm⁻¹): 3377, 2211, and 1434.

### Step (vi): Synthesis of 4-{[[2-(bis-cyclopropylmethyl-amino)-8-methyl-quinolin-3-ylmethyl]-(2H-tetrazol-5-yl)-amino]-methyl}-2,6-di-tert-butyl-phenol

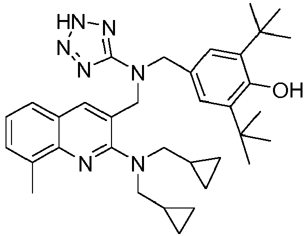

[2-(Bis-cyclopropylmethyl-amino)-8-methyl-quinolin-3-ylmethyl]-(3,5-di-*tert-*butyl-4-hydroxy-benzyl)-cyanamide (0.035 g, 0.065 mmol) obtained in step (v), sodium azide (0.021 g, 0.325 mmol) and ammonium chloride (0.175 g, 0.325 mmol) were put in a RB flask under N₂ atmosphere. To this reaction mixture, DMF (2 mL) was added and heated at 100°C for 1 h. The reaction mixture was cooled to RT and ice was added to this and was extracted with ethyl acetate (3 x 10 mL). The combined organic layer was washed with brine, dried over sodium sulphate and then concentrated under vacuum to afford of 4-{[[2-(bis-cyclopropylmethyl-amino)-8-methyl-quinolin-3-ylmethyl]-(2H-tetrazol-5-yl)-amino]-methyl}-2,6-di-*tert*-butyl-phenol (0.02 g, yield: 52.9%.).
¹H NMR (CDCl₃, 400 MHz): δ 7.48-7.46 (s, 1H), 7.35 -7.27 (m, 3H), 7.20 (s, 2H ), 4.83 (s, 2H), 4.55 (s, 2H), 3.30-3.29 (m, 4H), 2.69 (s, 3H) 1.4 (s, 18H), 1.21-1.10 (m, 2H), 0.54-0.49 (m, 4H), 0.19-0.15 (m, 4H); *m*/*z* (ES-MS): 582 (M⁺+1, 100%); IR (KBr , cm⁻¹) 3637, 2923, 1434.

### Step (vii): Synthesis of 4-{[[2-(bis-cyclopropylmethyl-amino)-8-methyl-quinolin-3-ylmethyl]-(2-methyl-2H-tetrazol-5-yl)-amino]-methyl}-2,6-di-tert-butyl-phenol

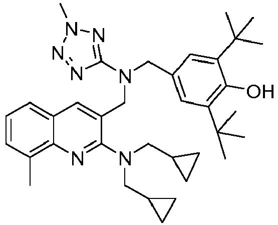

To a suspension of 4-{[[2-(bis-cyclopropylmethyl-amino)-8-methyl-quinolin-3-ylmethyl]-(2H-tetrazol-5-yl)-amino]-methyl}-2,6-di-*tert*-butyl-phenol (0.3 g, 0.51mmol) in water (5 mL), sodium hydroxide (0.041 g, 1.032 mmol) was added and was stirred for 15 min at RT followed by the addition of dichloromethane (5 mL). To this reaction mixture, dimethyl sulphate (0.12 g, 1.032 mmol) was added followed by the addition of tetra butyl ammonium bromide (0.008 g, 0.02 mmol). The reaction was stirred for 1 h. The organic layer was separated from aqueous layer and the aqueous layer was extracted with DCM (3 x 20 mL). The combined organic layer was washed with brine, dried over sodium sulphate and then concentrated under vacuum to afford the crude residue which was purified by column chromatography over 100-200 mesh silica gel using 8% ethyl acetate and petroleum ether to afford 4-{[[2-(bis-cyclopropylmethyl-amino) -8-methyl-quinolin-3-ylmethyl]-(2-methyl-2H-tetrazol-5-yl)-amino]-methyl}-2,6-di-*tert*-butyl-phenol (0.03g, yield: 13%).
Purity 94.36% (HPLC: Symmetry Shield RP8 (150x4.6) [0.01M KH₂PO₄: CH₃CN], 218nM, Rₜ 12.55 min); ¹H NMR (CDCl₃, 400 MHz): δ 7.74 (s, 1H), 7.42-7.39(m, 2H), 7.20-7.17 (m, 1H), 7.03 (s, 2H), 5.17(s, 1H), 4.82 (s, 2H), 4.53 (s, 2H), 4.17 (s, 3H), 3.21 -3.19 (m, 4H), 2.70 (s, 3H), 1.33 (s, 18H), 1.08-1.00 (m,2H), 0.38-0.34 (m, 4H), 0.10-0.06 (m, 4H); *m*/*z* (ES-MS): 596 (M⁺+1, 100%); IR (neat, cm⁻¹) 3628, 3383, 1210.

### Example 61

### Determination of in vitro activity

An *in vitro* fluorescence-based assay to identify CETP inhibitors was developed from modifications of the protocols outlined in Bisgaier et al., J Lipid Res., 34(9): 1625-34 (1993) and Epps et al., Chem Phys Lipids., 77(1): 51-63 (1995). Acceptor and donor lipid microemulsions were prepared according to Bisgaier *et al*., 1993, except that the buffer was prepared with 0.67 ug/mL human HDL (Calbiochem). Donor microemulsions contained the fluorescent cholesteryl ester analog BODIPY-CE (Molecular Probes), characterized by excitation and emission maxima at 503 nm and 518 nm, respectively. The CETP-mediated transfer of fluorescent cholesteryl ester to acceptor particles was monitored over a 2-hour time period using the FAM filter set (excitation 492 nm, emission 516 nm) in an MX3000P fluorescent plate reader (Stratagene). Recombinant CETP enzyme (Cardiovascular Targets) was used at 0.14 ng/µl, final concentration, to achieve lipid transfer. CETP inhibition by compounds was compared to DMSO controls and graphed as a percentage of the control CETP activity over 2 hours. The IC₅₀ curves for CETP inhibition were generated from the activity profiles. Active compounds were also tested for CETP inhibition as above, but in the presence of 3% human serum albumin, fraction V (Calbiochem).

Using this protocol, compounds given in Examples 2, 3, 43, 44, 45 and 49 were shown to exhibit a CETP activity with an IC₅₀ of less than or equal to 5 µM; compounds given in Examples 1, 4, 5, 6, 9 10, 11, 12, 13, 14, 15, 16, 17, 18, 19, 20, 21, 22, 23, 24, 25, 26, 27, 28, 29, 30, 31, 32, 33, 34, 36, 37, 42 and 46, have shown CETP activity with an IC₅₀ of less than or equal to 1 µM.

## Claims

1. A compound selected from 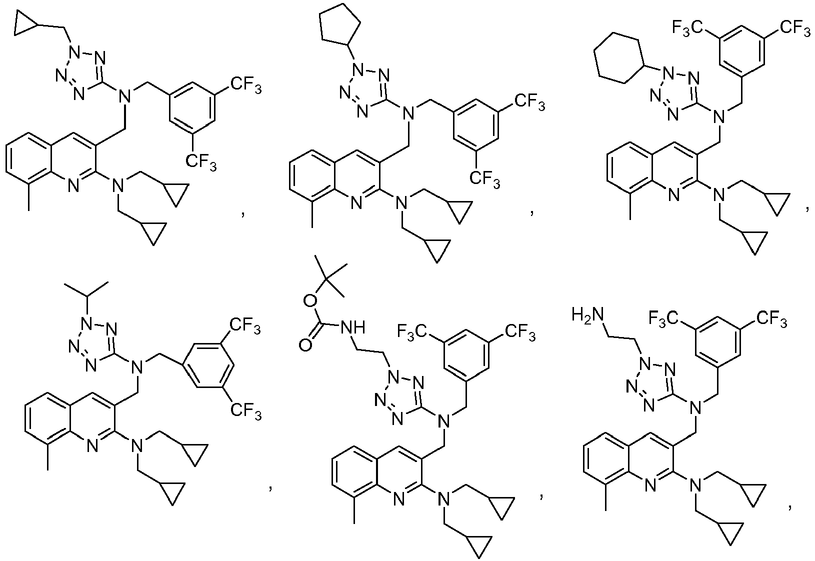 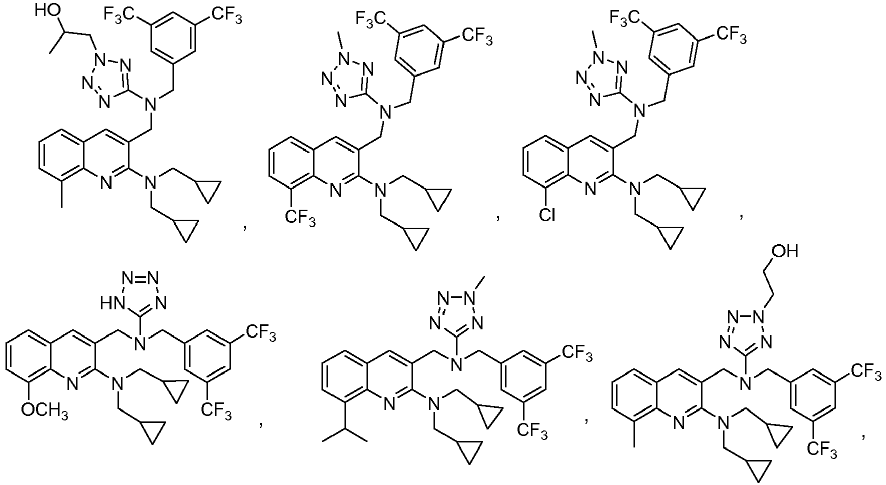 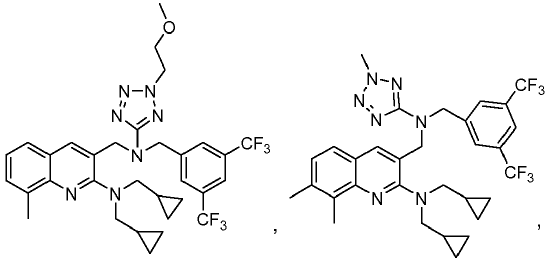 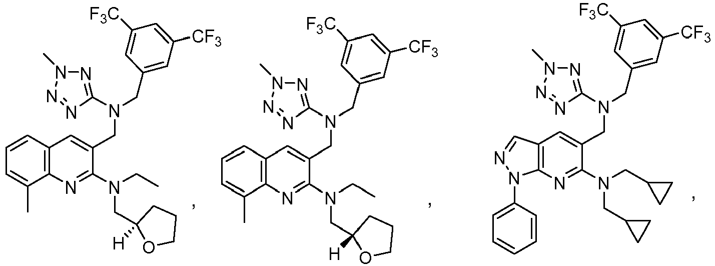 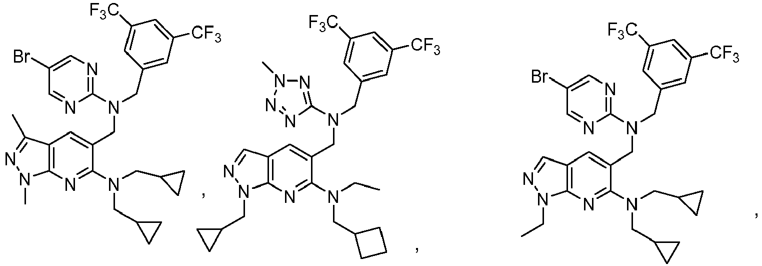 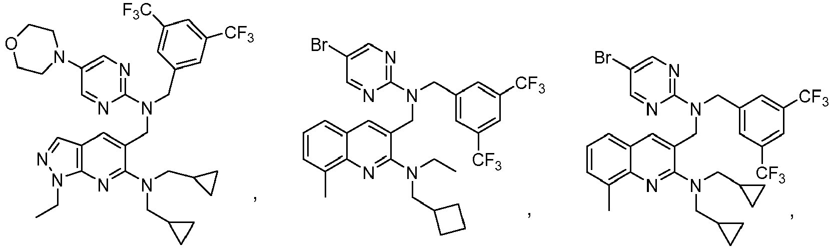 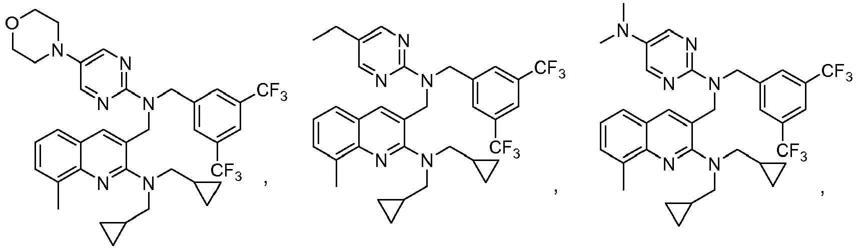 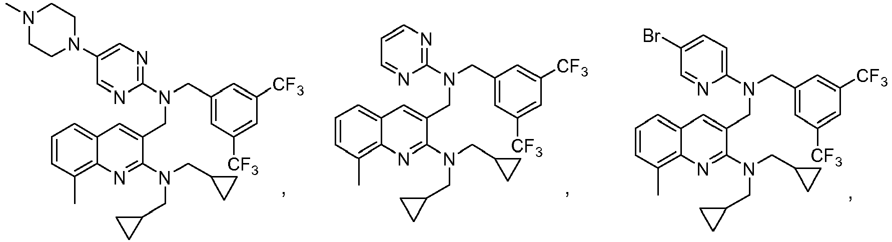 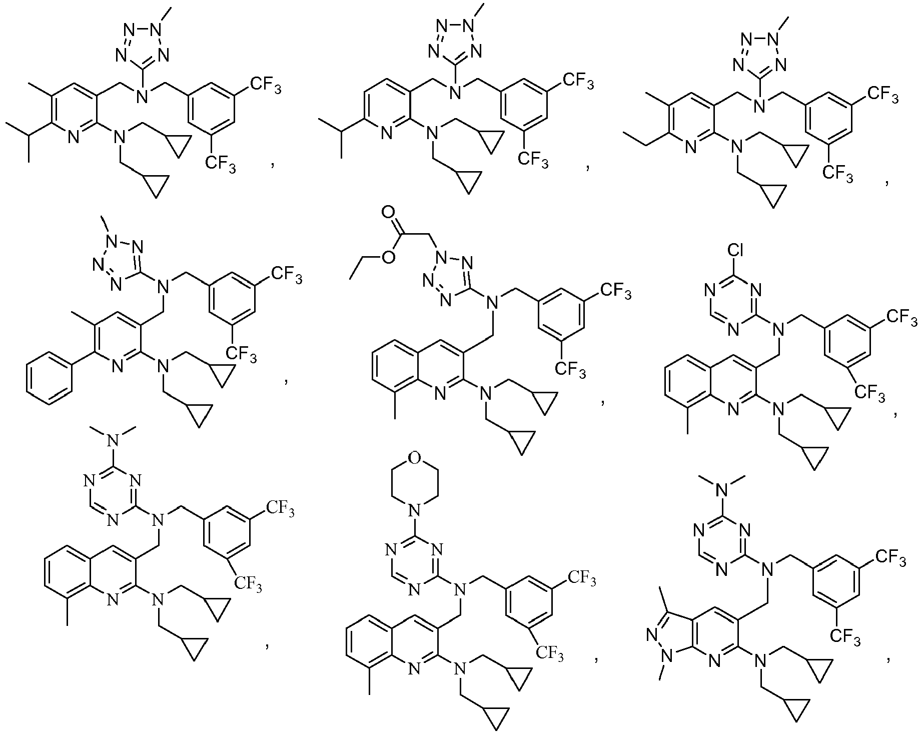 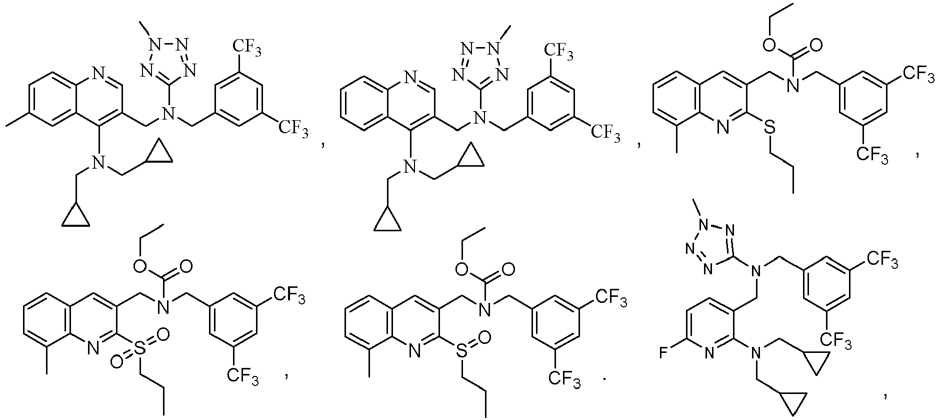 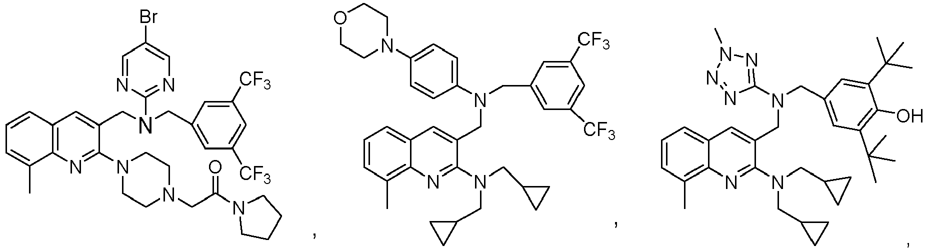 or a salt, a prodrug, a diastereomeric mixture, an enantiomer, a tautomer, or a racemic mixture thereof.

2. A pharmaceutical composition comprising a pharmaceutically acceptable carrier and at least one compound according to claim 1.

3. A pharmaceutical composition according to Claim 2, further comprising at least one of the following:
a pharmaceutically acceptable auxiliary;
a pharmaceutically acceptable preservative;
a pharmaceutically acceptable excipient;
a pharmaceutically acceptable diluent;
a pharmaceutically acceptable solvate; or
any combination thereof.

4. A pharmaceutical composition according to claim 2 or 3, in the form of a tablet, a capsule, a cachet, a powder, a granule, a solution, a suspension, an emulsion, a bolus, a lozenge, a suppository, a pessary, a tampon, a cream, a gel, a paste, a foam, a spray, an aerosol, a microcapsule, a liposome, a transdermal patch, a pastille, a paste, or a mouthwash.

5. A compound according to claim 1 which is a cholesteryl ester transfer protein (CETP) inhibitor.

## Patentansprüche

1. Verbindung, ausgewählt aus 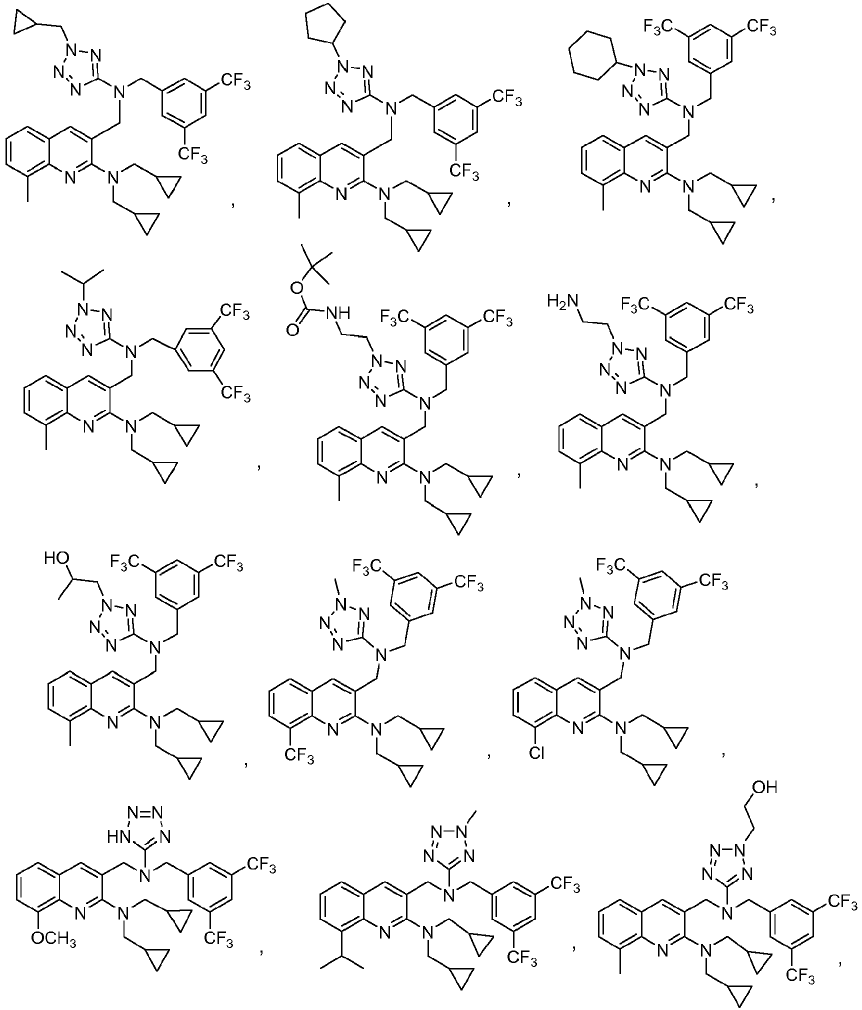 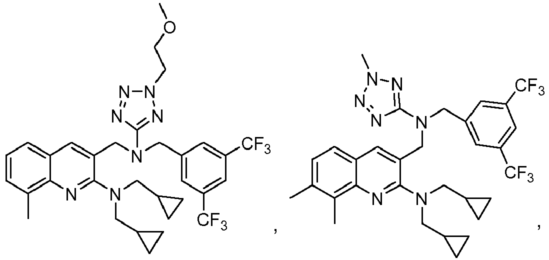 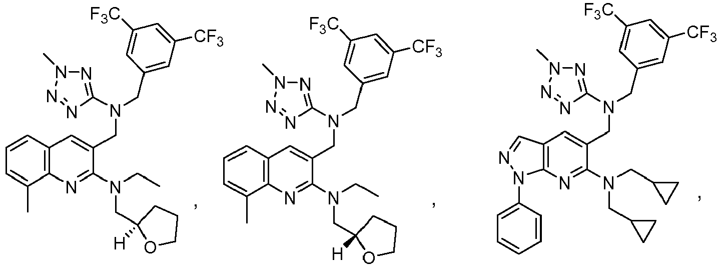 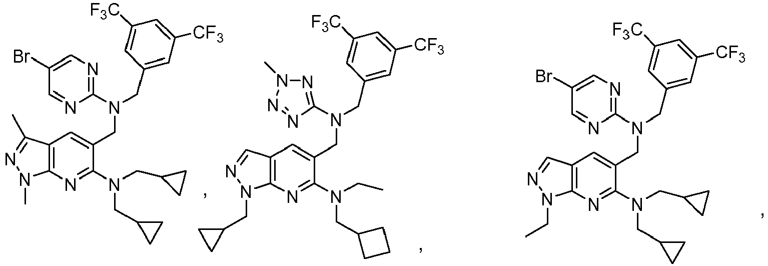 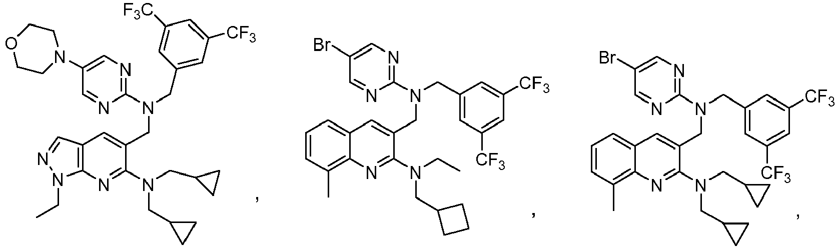 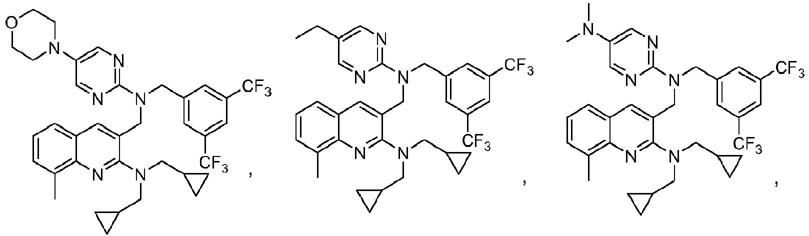 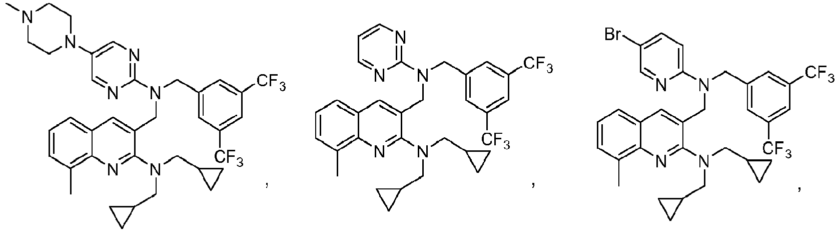 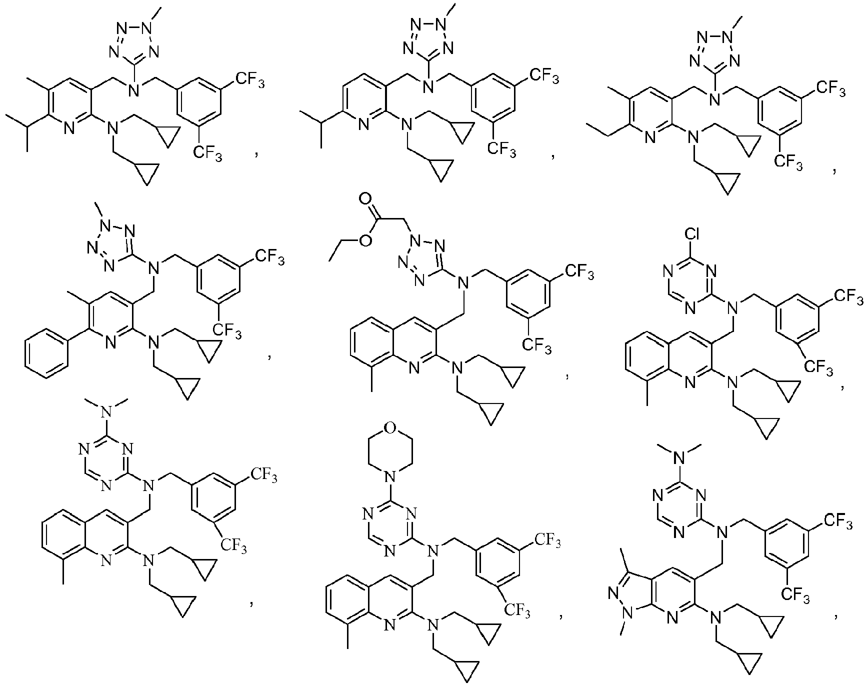 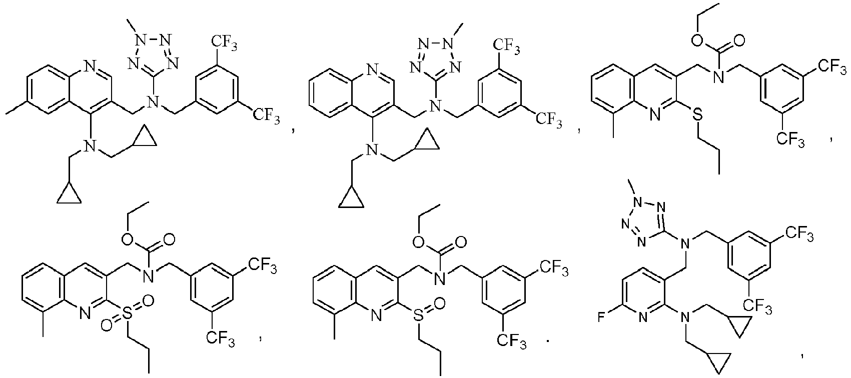 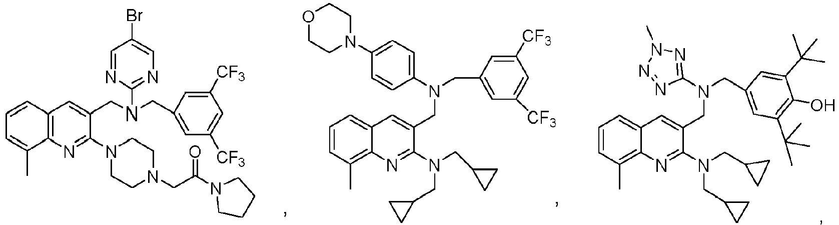 oder ein Salz, eine Prodroge, ein diastereomeres Gemisch, ein Enantiomer, ein Tautomer oder ein racemisches Gemisch davon.

2. Pharmazeutische Zusammensetzung, die einen pharmazeutisch akzeptablen Träger und wenigstens eine Verbindung nach Anspruch 1 umfasst.

3. Pharmazeutische Zusammensetzung nach Anspruch 2, die ferner wenigstens eines der Folgenden umfasst:
einen pharmazeutisch akzeptablen Hilfsstoff;
ein pharamzeutisch akzeptables Konservierungsmittel;
einen pharmazeutisch akzeptablen Exzipienten;
ein pharmazeutisch akzeptables Verdünnungsmittel;
ein pharmazeutisch akzeptables Solvat; oder
eine beliebige Kombination davon.

4. Pharmazeutische Zusammensetzung nach Anspruch 2 oder 3 in Form einer Tablette, einer Kapsel, eines Cachet, eines Pulvers, eines Granulats, einer Lösung, einer Suspension, einer Emulsion, eines Bolus, einer Lutschtablette, eines Suppositoriums, eines Pessars, eines Tampons, einer Creme, eines Gels, einer Paste, eines Schaums, eines Spray, eines Aerosols, einer Mikrokapsel, eines Liposoms, eines transdermalen Patch, einer Pastille, einer Paste oder eines Mundwassers.

5. Verbindung nach Anspruch 1, die ein CETP-(Cholesterylestertransferprotein)-Inhibitor ist.

## Revendications

1. Composé sélectionné parmi 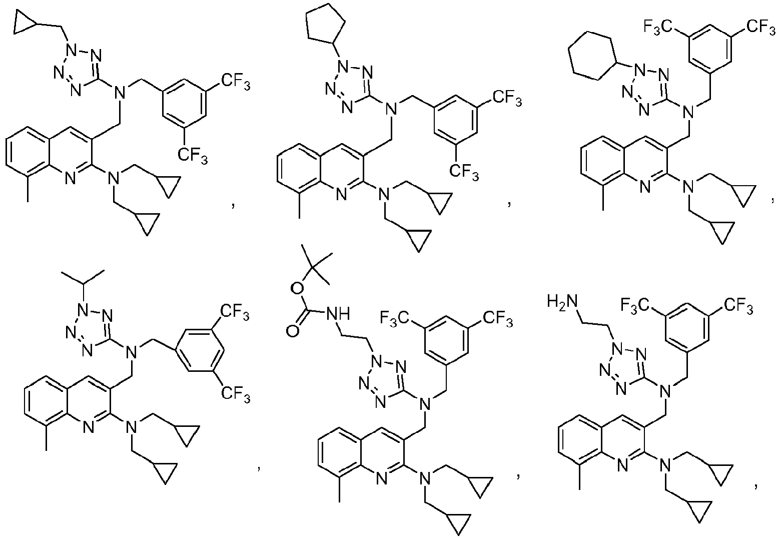 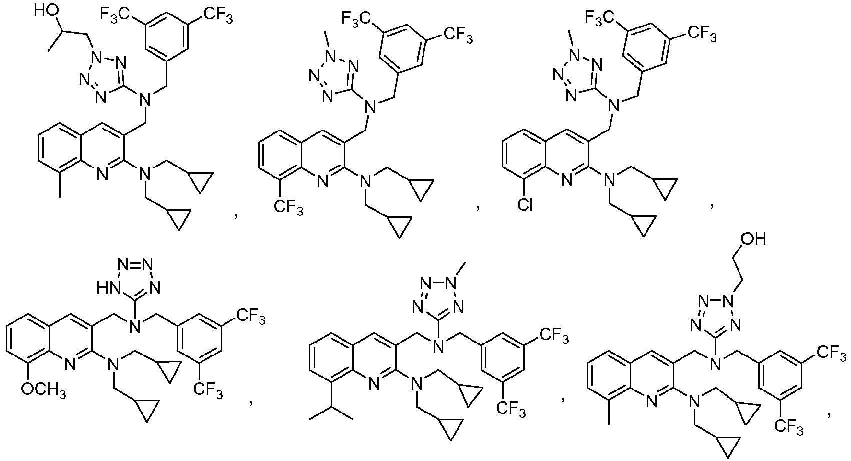 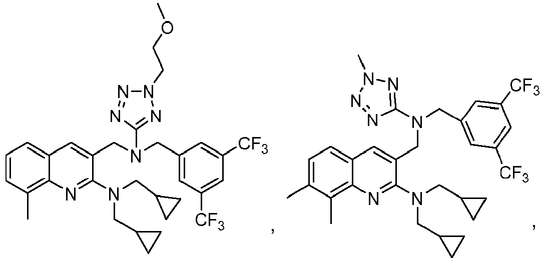 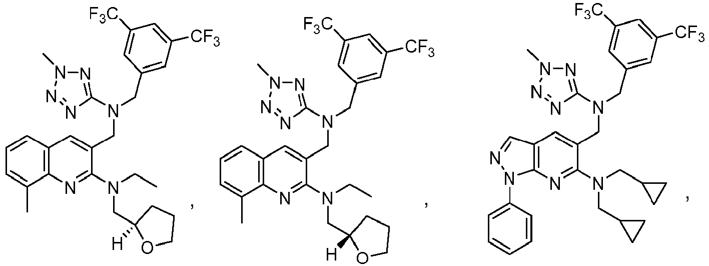 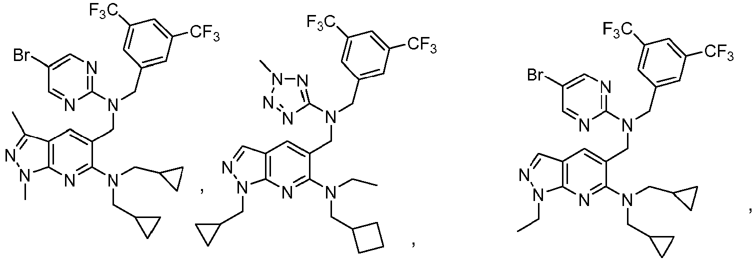 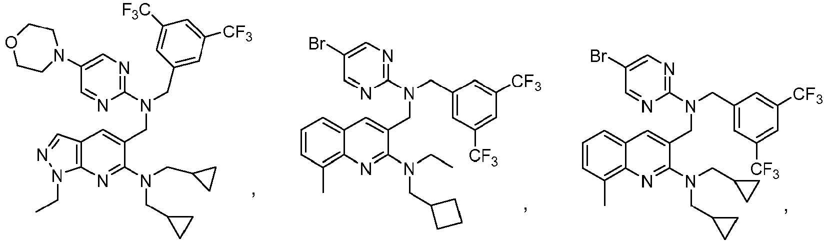 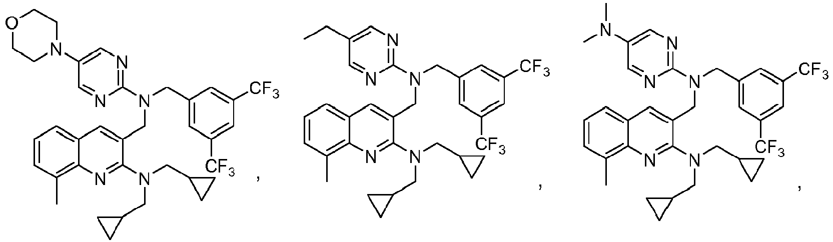 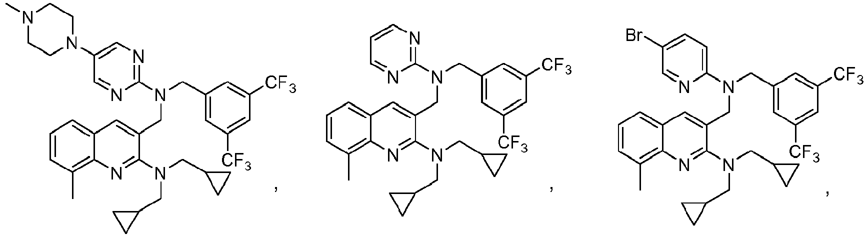 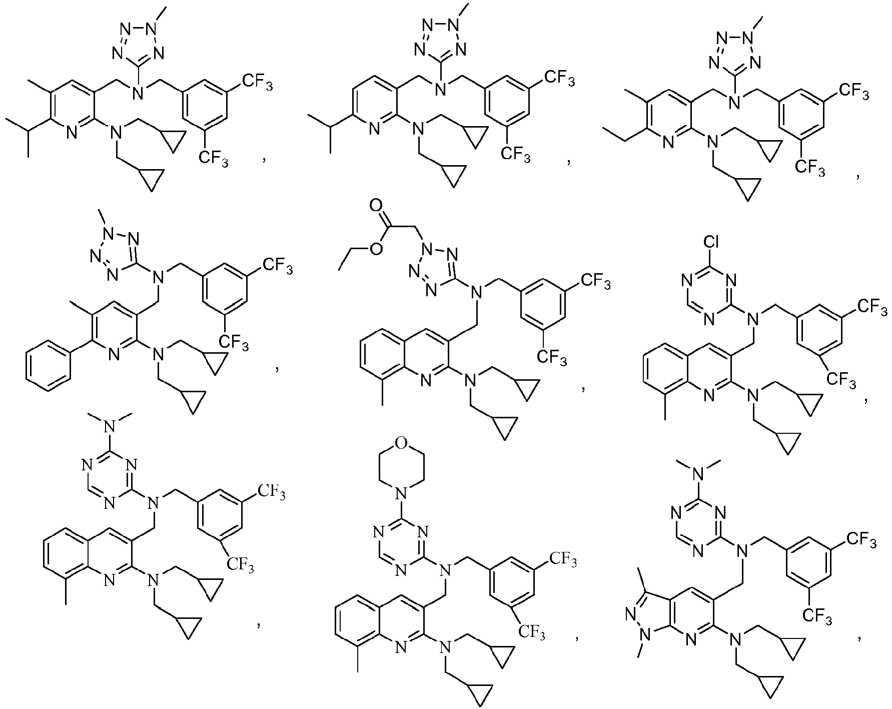 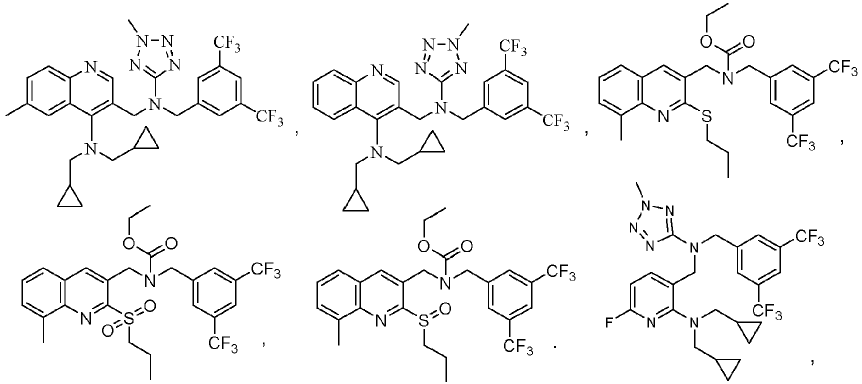 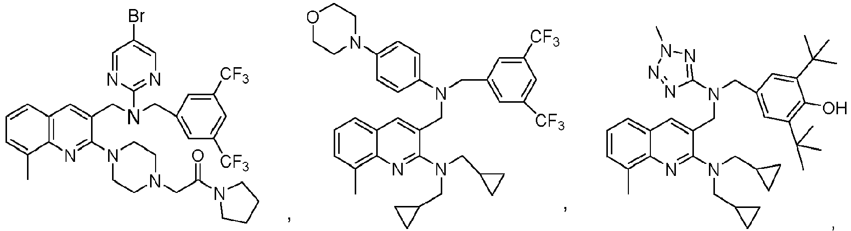 ou un sel, une prodrogue, un mélange diastéréomérique, un énantiomère, un tautomère, ou un mélange racémique de celui-ci

2. Composition pharmaceutique comprenant un véhicule pharmaceutiquement acceptable et au moins un composé selon la revendication 1.

3. Composition pharmaceutique selon la revendication 2, comprenant en outre au moins l'un des suivants :
un auxiliaire pharmaceutiquement acceptable ;
un conservateur pharmaceutiquement acceptable ;
un excipient pharmaceutiquement acceptable ;
un diluant pharmaceutiquement acceptable ;
un solvate pharmaceutiquement acceptable ; ou
un combinaison quelconque de ceux-ci.

4. Composition pharmaceutique selon la revendication 2 ou 3, sous la forme d'un comprimé, d'une capsule, d'un cachet, d'une poudre, d'un granule, d'une solution, d'une suspension, d'une émulsion, d'un bolus, d'une pastille, d'un suppositoire, d'un pessaire, d'un tampon, d'une crème, d'un gel, d'une pâte, d'une mousse, d'une pulvérisation, d'un aérosol, d'une microcapsule, d'un liposome, d'un timbre transdermique, d'une pastille, d'une pâte ou d'un bain de bouche.

5. Composé selon la revendication 1, qui est un inhibiteur de la protéine de transfert des esters de cholestérol (CETP).
